# EUROPEAN PATENT SPECIFICATION

(11) **EP 0 622 356 B1**
(45) Date of publication and mention of the grant of the patent: **01.07.1998**
(21) Application number: 94303101.3
(22) Date of filing: 28.04.1994
(51) Int. Cl.: C07D 209/42, A61K 31/40

(54) **Indoloylguanidine derivatives as inhibitors of sodium-hydrogen exchange**
Indoloylguanidinderivate als Inhibitoren des Natrium-Protonen Austauschs
Dérivés d'indoloylguanidine comme inhibiteurs de l'échange sodium-hydrogène

(30) Priority: 28.04.1993 JP 125085/93
(43) Date of publication of application: 02.11.1994
(73) Proprietor: SUMITOMO PHARMACEUTICALS COMPANY, LIMITED, Osaka 541 (JP)
(72) Inventor: Kojima, Atsuyuki, Takarazuka-shi (JP); Kitano, Masahumi, Nishinomiya-shi (JP); Miyagishi, Akira, Kobe-shi (JP); Noguchi, Tsuyoshi, Toyonaka-shi (JP); Yagi, Hideki, Studio Inn Dekijimaeki 318, Osaka-shi (JP); Nakano, Kazuhiro, Studio Inn Dekijimaeki 345, Osaka-shi (JP); Ohashi, Naohito, Takatsuki-shi (JP)
(74) Representative: Cresswell, Thomas Anthony

(56) References cited:
- EP-A- 0 116 360
- EP-A- 0 416 499
- EP-A- 0 639 573
- DE-A- 4 127 026
- MERCK INDEX, 11TH EDITION, 1989 , RAHWAY N.J. USA page 67 S. BUDAVARI

## Description

The present invention relates to novel indoloylguanidine derivatives. The present invention also relates to sodium/proton (Na⁺/H⁺) exchanger inhibitors comprising the indoloylguanidine derivatives as the active component which are useful for the treatment and prevention of diseases caused by increased sodium/proton (Na⁺/H⁺) exchanger activity.

Certain polycyclic aroylguanidine derivatives are known as those having polycondensed rings, for example, a naphthalene, 9,10-dihydroanthracene, benzofuran, 2,3-dihydrobenzofuran, benzothiophene, benzothiazole, methylenedioxybenzene, pyridothiophene, pyrimidothiophene, quinoline, 1,6-naphthylidine, 1,8-naphthylidine, 3,4-dihydrobenzopyran, 3,4-dihydroquinazolin-4-one, 1,2,3,4-tetrahydroquinazolin-2-one, quinoxaline, 5,6,7,8-tetrahydroquinoxaline, benzoazepine, benzotriazepine, benzimidazolothiazine, benzopyranopyran or benzocarbazole ring. As one of the aroylguanidine derivatives having indole rings there is known 1-guanidino-carbonyltryptophane but this compound is merely registered in Chemical Abstracts under Registered No. 18322-34-4, without any reference to its source.

Turning to some monocyclic aroylguanidine derivatives, pyrazinoylguanidine derivative represented by, e.g., Amiloride, are known to exhibit the sodium/proton (Na⁺/H⁺) exchanger inhibition activity and anti-arrhythmic activity, cf., J. Membrane Biol., Vol. 105, 1 (1988); and Circulation, Vol. 79, 1257 (1989). Recent reports also reveal that benzoylguanidine derivatives possess the sodium/proton (Na⁺/H⁺) exchanger inhibition and anti-arrhythmic activities, cf., J. Mol. Cell. Cardiol., Vol. 24, Supple. I, S. 92 (1992); ibid., Vol. 24, Suppl. I, S. 117 (1992); and Japanese Patent KOKAI Nos. 3-106858 and 5-339228.

DE-A-4,127,026 discloses a pyrazine derivative of formula: 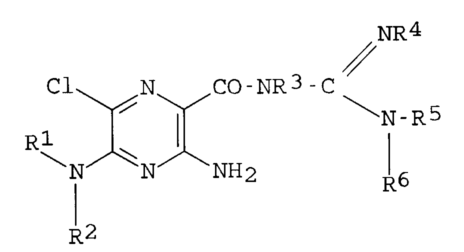 wherein
R¹ is hydrogen or a C₁-C₈ alkyl group,
R² is a number of different group, and
R³ to R⁶ are each hydrogen, a C₁-C₈ alkyl group or a benzyl group.

EP-A-116,360 discloses an indol derivative of formula: 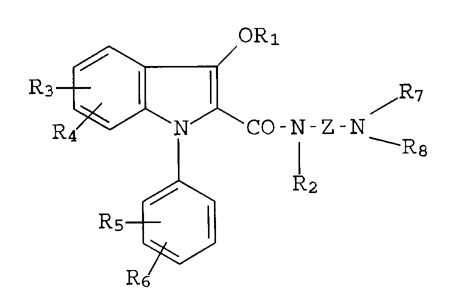 wherein
R₁ is an aliphatic or aromatic acyl group or a benzyl group,
R₂ is hydrogen or an alkyl group,
R₃ to R₅ are hydrogen, a halogen, an alkyl group or an alkoxy group,
R₆ is hydrogen, a halogen, an alkyl group or an alkoxy group, and, when R₅ is hydrogen, a nitro or trifluoromethyl group, and
R₇ and R₈ are hydrogen or alkyl or together form a heterocyclic group.

EP-A-639,573 has overlapping priority and filing dates with the present application. It discloses a compound of formula: 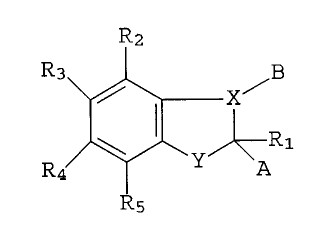 wherein
X is N or CR₆,
Y is O, S or NR₇,
A and B form a bond or are hydrogen when X is CR₆ and Y is NR₇,
one of R₁ and R₆ is a -CO-N=C(NH₂)₂ group and the others are hydrogen, F, Cl, Br, I, C₁-C₆ alkyl, up to two of R₁ to R₆ are CN, NO₂, N₃, C₁-C₄ alkoxy or CF₃ and up to one of R₁ to R₆ is R₈-CₙH₂ₙ-Z- wherein n is 0 to 10 and Z and R₈ are various groups, and
R₇ is hydrogen, C₁-C₁₀ alkyl, C₂-C₁₀ alkenyl or R₈-CₙH₂ₙ-.

An object of the present invention is to provide novel indoloylguanidine derivatives which inhibit the sodium/proton (Na⁺/H⁺) exchanger activity and are therefore useful for the treatment and prevention of diseases caused by increased sodium/proton (Na⁺/H⁺) exchanger activity, for example, hypertension, arrhythmia, angina pectoris, cardiac hypertrophy, organ disorders associated with ischemic reperfusion such as cardiac ischemic reperfusion injury, disorders induced by surgical treatment such as organ transplantation or percutaneous transluminal coronary angioplasty (PTCA), cerebro-ischemic disorders such as disorders associated with cerebral infarction, disorders caused after cerebral apoplexy as sequelae, or cerebral edema; or diseases caused by excessive cell proliferation such as proliferation of fibroblast, proliferation of smooth muscle cells or proliferation of mesangium cells, which diseases are, e.g. atherosclerosis, pulmonary fibrosis, hepatic fibrosis, renal fibrosis, glomerular nephrosclerosis, organ hypertrophy, prostatic hypertrophy, diabetic complications or recurrent stricture after PTCA.

Another object of the present invention is to provide compositions comprising the indoloylguanidine derivatives as the active component which inhibit the sodium/proton (Na⁺/H⁺) exchanger activity and are useful for the prevention and treatment of diseases caused by abnormal sodium/proton (Na⁺/H⁺) exchanger activity.

The present invention provides a compound which is an indoloylguanidine derivative of formula (1): 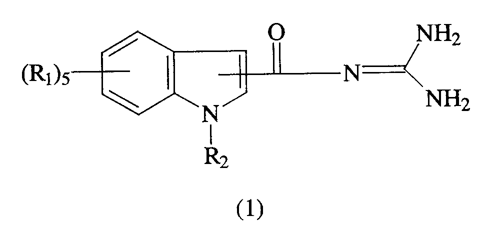 wherein
each R₁, which may be the same or different, is independently selected from hydrogen, an alkyl group which is unsubstituted or substituted by hydroxy, amino, cyano, carboxyl, alkoxycarbonyl, an aromatic group, an alkoxy group, a group -NR₆R₇ or a group -CONR₄R₅ wherein each of R₄ and R₅ independently represents hydrogen or an alkyl group, or R₄ and R₅ are combined together to form a saturated 5- to 7-membered cyclic amino group which may contain other heteroatoms in the ring, an alkenyl group, an alkynyl group, a cycloalkyl group, a halogen, a nitro group, a straight or branched alkanoyl group having up to 8 carbon atoms, an arylalkanoyl group having up to 10 carbon atoms, a carboxyl group, an alkoxycarbonyl group, an aromatic group selected from tolyl, naphthyl and heteroaryl, a group of formula: -OR₃, -NR₆R₇ or -SO₂NR₆R₇, or a group of formula -S(O)ₙR₄₀ selected from ethylsulphonyl, propylsulphonyl, isopropylsulphonyl and the corresponding alkylsulphinyl and alkylthio groups;
R₂ is a hydrogen atom, an alkyl group, a substituted alkyl group, a cycloalkyl group, a hydroxy group, an alkoxy group or a group of formula -CH₂R₂₀;
R₃ is hydrogen, an alkyl group, a substituted alkyl group, a cycloalkyl group or a group shown by formula:
   -CH₂R₃₀, wherein R₃₀ represents an alkenyl group or an alkynyl group;
each of R₆ and R₇, which may be the same or different, is independently selected from hydrogen, an alkyl group, a substituted alkyl group, a cycloalkyl group, an acyl group and a group of formula: -CH₂R₆₀, wherein R₆₀ represents an alkenyl group or an alkynyl group; or R₆ and R₇, together with the nitrogen atom to which they are attached, form a saturated 5- to 7- membered cyclic amino group which optionally includes at least one other hetero atom in the ring;
R₄₀ is an alkyl group or a substituted alkyl group; n is 0, 1 or 2;
   and
R₂₀ is an alkenyl group or an alkynyl group; and wherein the substituents R₁ and the guanidinocarbonyl group -C(=O)-N=C(NH₂)₂ are each, independently, attached to any one of the 5- and 6- membered rings of the indole nucleus;
   or 6-(3-aminopropoxy)-1-methyl-4-trifluoromethyl-2-indoloylguanidine, or 6-(3-dimethylaminopropoxy)-1-methyl-4-trifluoromethyl-2-indoloylguanidine; or a pharmaceutically acceptable acid addition salt thereof.

Typically the guanidinocarbonyl group is bonded at one of positions 2 and 3 of the indole nucleus (i.e. in the 5-membered ring) and the groups R₁ are bonded at the other of positions 2 and 3 and at each of positions 5, 6, 7 and 8 of the indole nucleus (i.e. in the 6-membered ring).

Among the indoloylguanidine derivatives of formula (1), the compounds represented by formula (2) are particularly preferred: 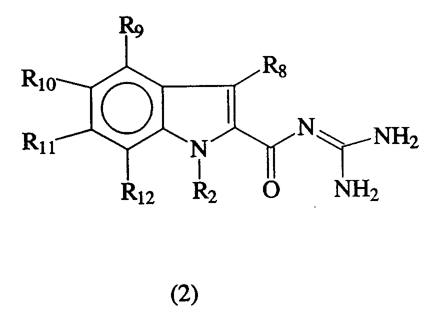 wherein:
each of R₈, R₉, R₁₀, R₁₁, and R₁₂, which may be the same or different, is independently selected from hydrogen, an alkyl group which is unsubstituted or substituted by hydroxy, amino, cyano, carboxyl, alkoxycarbonyl, an aromatic group, an alkoxy group, a group -NR₆R₇ or a group -CONR₄R₅ wherein each of R₄ and R₅ independently represents hydrogen or an alkyl group, or R₄ and R₅ are combined together to form a saturated 5- to 7-membered cyclic amino group which may contain other heteroatoms in the ring, an alkenyl group, an alkynyl group, a cycloalkyl group, a halogen, a nitro group, a straight or branched alkanoyl group having up to 8 carbon atoms, an arylalkanoyl group having up to 10 carbon atoms, a carboxyl group, an alkoxycarbonyl group, an aromatic group selected from tolyl, naphthyl and heteroaryl, a group shown of formula:
   -OR₃, -NR₆R₇ or -SO₂NR₆R₇, or a group of formula -S(O)ₙR₄₀ selected from ethylsulphonyl, propysulphonyl, isopropylsulphony and the corresponding alkylsulphinyl and alkylthio groups;
R₂ is hydrogen, an alkyl group, a substituted alkyl group, a cycloalkyl group, a hydroxy group, an alkoxy group or a group of formula: -CH₂R₂₀
R₃ is hydrogen, an alkyl group, a substituted alkyl group, a cycloalkyl group or a group of formula: -CH₂R₃₀, wherein R₃₀ represents an alkenyl group or an alkynyl group;
each of R₆ and R₇, which may be the same or different, is independently selected from hydrogen, an alkyl group, a substituted alkyl group, a cycloalkyl group, an acyl group and a group of formula: -CH₂R₆₀, wherein R₆₀ is an alkenyl group or an alkynyl group; or R₆ and R₇, together with the nitrogen atom to which are they are attached, form a saturated 5- to 7-membered cyclic amino group which optionally includes at least one other heteroatom in the ring;
R₄₀ is an alkyl group or a substituted alkyl group;
n is 0, 1 or 2; and
R₂₀ is an alkenyl group or an alkynyl group; or a pharmaceutically acceptable acid addition salt thereof.

The respective groups in the indoloylguanidine derivatives of the present invention are described below in detail.

The alkyl group refers to a straight or branched alkyl group having carbon atoms of 8 or less, for example, methyl, ethyl, propyl, 2-propyl, butyl, 2-butyl, 2-methylpropyl, 1,1-dimethylethyl, pentyl, hexyl, heptyl and octyl.

The alkenyl group refers to an alkenyl group having carbon atoms up to 6, e.g., vinyl, allyl, isopropenyl, 1-propenyl, butenyl, pentenyl and hexenyl.

The alkynyl group refers to an alkynyl group having 2 to 6 carbon atoms, e.g., ethynyl, propargyl, butynyl and pentynyl.

The cycloalkyl group refers to a cycloalkyl group having 3 to 7 carbon atoms, e.g., cyclopropyl, cyclobutyl, cyclopentyl, cyclohexyl and cycloheptyl.

Typical examples of the halogen atom include fluorine, chlorine and bromine.

The acyl group refers to a straight or branched alkanoyl group having carbon atoms up to 8, e.g., acetyl, propanoyl and 2-methylpropanoyl; an arylalkanoyl group having carbon atoms up to 10, e.g., phenylacetyl and phenylpropanoyl; and an aroyl group having carbon atoms of 11 or less, e.g., benzoyl, 1-naphthoyl and 2-naphthoyl.

The alkoxycarbonyl group refers to a straight or branched alkoxycarbonyl group having carbon atoms up to 6, e.g., methoxycarbonyl, ethoxycarbonyl, propoxycarbonyl and 2-propoxycarbonyl.

The aromatic group refers to an aryl or heteroaryl group which may have a substituent. Examples of the aryl group are those having carbon atoms up to 10, e.g., phenyl, tolyl or naphthyl, and examples of the heteroaryl group are a 5- or 6-membered aromatic group containing 1 to 4 nitrogen atoms or a 5- or 6-membered aromatic ring containing 1 to 2 nitrogen atoms and one oxygen atom or one sulfur atom, e.g., 2-, 3- or 4-pyridyl, 2- or 3-furyl, 2- or 3-thienyl, 1-, 3- or 4-oxazolyl, and 3-, 4- or 5-isoxazolyl.

Examples of the substituent in the substituted aryl or heteroaryl group include hydroxy group, an alkoxy group, a halogen atom, nitro and a group shown by formula: -NR₆R₇.

The alkoxy group refers to a straight or branched alkoxy group having carbon atoms up to 6, e.g., methoxy, ethoxy, isopropoxy and tert-butoxy.

As the saturated 5- to 7-membered cyclic amino group which is formed by combining R₆ and R₇ together and may contain other hetero atoms therein, there are, for example, a 5- to 7-membered cyclic group containing 1 to 3 nitrogen atoms and a 5- to 7-membered cyclic group containing one nitrogen atom and one oxygen atom. Specific examples of such cyclic amino group include 1-pyrrolidinyl, piperidino, 1-piperazinyl, morpholino and 4-methylmorpholino.

Examples of the substituent in the substituted alkyl group include a cycloalkyl group, a halogen atom, hydroxy, an alkoxy group, cyano, carboxyl, an alkoxycarbonyl group, an acyl group, an aromatic group, or a group shown by formula: -CONR₄R₅ or -NR₆R₇, wherein each of R₄ and R₅ independently represents hydrogen atom or an alkyl group, or R₄ and R₅ are combined together to form a saturated 5- to 7-membered cyclic amino group which may contain other hetero atoms in the ring. Particularly where R₁, R₂ and R₃ represent a substituted alkyl group, examples of the substituent include a cycloalkyl group, a halogen atom, hydroxy, an alkoxy group, carboxyl, an alkoxycarbonyl group, an acyl group, an aromatic group or a group shown by formula: -CONR₄R₅ or -NR₆R₇. Where R₆ and R₇ represent a substituted alkyl group, examples of the substituent include a cycloalkyl group, hydroxy, an alkoxy group, carboxyl, an alkoxycarbonyl group, an acyl group, an aryl group or a group shown by formula: -CONR₄R₅ or -NR₄R₅. As the alkyl moiety in the substituted alkyl group, the same examples as those for the alkyl group described above are given.

As such a substituted alkyl group, there are, for example, an alkyl group having 1 to 5 carbon atoms which is substituted with a cycloalkyl having 3 to 6 carbon atoms, a polyhaloalkyl group having 1 to 5 carbon atoms, a hydroxyalkyl group having 1 to 6 carbon atoms, an alkoxyalkyl group having 2 to 6 carbon atoms, a cyanoalkyl group having 2 to 6 carbon atoms, a carboxyalkyl group having 2 to 6 carbon atoms, an alkoxycarbonylalkyl group having 3 to 8 carbon atoms, an alkanoylalkyl group having 3 to 8 carbon atoms, an aroylalkyl group having carbon atoms up to 16, a phenyl- or naphthyl-C₁ to C₅ alkyl group which may be substituted, a carbamoyl-C₁ to C₃ alkyl group in which the nitrogen atom may be substituted with one or two C₁ to C₃ alkyl, an amino-C₁ to C₅ alkyl group in which the nitrogen atom may be substituted with one or two C₁ to C₃ alkyl or C₇ to C₁₁ aralkyl, and a saturated 5- to 7-membered cyclic amino-C₁ to C₃ alkyl group.

Representative examples of the substituted alkyl group include:
in the case of R₁: a hydroxyalkyl group having 1 to 6 carbon atoms such as hydroxymethyl, hydroxyethyl or 1-hydroxyethyl; and an aminoalkyl group having 1 to 5 carbon atoms such as aminomethyl, aminoethyl or 1-aminoethyl;
in the case of R₂: a hydroxyalkyl group having 1 to 6 carbon atoms such as hydroxyethyl, hydroxypropyl, hydroxybutyl, 2-hydroxypropyl or 3,4-dihydroxybutyl; an alkoxyalkyl group having 1 to 6 carbon atoms such as methoxyethyl, ethoxyethyl or methoxypropyl; a carboxyalkyl group having 2 to 6 carbon atoms such as carboxyethyl or carboxypropyl; an alkoxycarbonylalkyl group having 3 to 7 carbon atoms such as methoxycarbonylmethyl, ethoxycarbonylmethyl or methoxycarbonylethyl; a phenyl- or naphthyl-C₁ to C₅ alkyl group (wherein a phenyl or naphthyl group may be substituted with a substituent, e.g., a C₁ to C₃ alkyl group, a halogen atom, nitro, amino, hydroxy or a C₁ to C₃ alkoxy group) such as benzyl, phenylethyl, phenylpropyl, phenylbutyl or, 1- or 2-naphthylmethyl; a carbamoyl-C₁ to C₃ alkyl group in which the nitrogen atom may be substituted with one or two C₁ to C₃ alkyl groups, such as carbamoylmethyl, carbamoylethyl or dimethylcarbamoylmethyl; or, an amino-C₁ to C₅ alkyl group in which the nitrogen atom may be substituted with one or two C₁ to C₃ alkyl, such as aminoethyl, aminopropyl, dimethylaminoethyl or diethylaminoethyl;
in the case of R₃ and R₄₀: a hydroxyalkyl group having 1 to 6 carbon atoms such as hydroxyethyl, hydroxypropyl, 2-hydroxypropyl, hydroxybutyl or 2,3-dihydroxybutyl; a carboxyalkyl group having 2 to 6 carbon atoms such as carboxymethyl or carboxyethyl; a phenyl-C₁ to C₅ alkyl group such as benzyl, phenylethyl or phenylpropyl; a carbamoyl-C₁ to C₃ alkyl group such as carbamoylmethyl or carbamoylethyl; an amino-C₁ to C₅ alkyl group containing one or two nitrogen atoms in which the nitrogen atom may be substituted with one or two C₁ to C₃ alkyl or C₇ to C₁₁ aralkyl groups, such as aminoethyl, aminopropyl, dimethylaminoethyl, dimethylaminopropyl or benzylmethyl-aminoethyl; or a saturated 5- to 7-membered cyclic amino-C₁ to C₃ alkyl group such as 1-pyrrolidinylethyl or piperadinoethyl; and
in the case of R₆ and R₇: a phenyl-C₁ to C₅ alkyl group such as phenylethyl.

Examples of the saturated 5- to 7-membered cyclic amino group which is formed by combining R₄ and R₅ together and may contain other hereto atoms in the ring thereof include the same groups as exemplified for the aforesaid cyclic amino groups formed by R₆ and R₇.

In one aspect the invention provides indoloylguanidine derivatives of formula (1) wherein each R₁, which may be the same or different, is independently selected from hydrogen, an alkyl group which is unsubstituted or substituted by hydroxy, amino, cyano, carboxyl, alkoxycarbonyl, an aromatic group, an alkoxy group, a group -NR₆R₇ or a group -CONR₄R₅ wherein each of R₄ and R₅ independently represents hydrogen or an alkyl group, or R₄ and R₅ are combined together to form a saturated 5- to 7-membered cyclic amino group which may contain other heteroatoms in the ring, an alkenyl group, a cycloalkyl group, a halogen, a nitro group, an alkanoyl group, carboxyl group, a tolyl, naphthyl or heteroaryl group, an ethylsulfonyl, propylsulphonyl or isopropylsulphonyl group, and a group of formula -OR₃ or -NR₆R₇;
R₃ is hydrogen, an alkyl group or a substituted alkyl group;
each of R₆ and R₇, which may be the same or different, is independently selected from hydrogen, an alkyl group, a substituted alkyl group, an alkanoyl group or an aroyl group; or R₆ and R₇, together with the nitrogen atom to which they are attached, form a saturated 5- to 7-membered cyclic amino group which optionally includes at least one other hereto atom in the ring.

In a second aspect the invention provides indoloylguanidine derivatives of formula (1) wherein each R₁, which may be the same or different is independently selected from hydrogen, an alkyl group, a substituted alkyl group wherein the substituent is a hydroxy group or a group -NR₆R₇ as defined above, an alkenyl group, a cycloalkyl group, a halogen, a nitro group, an alkanoyl group, a carboxyl group, an ethylsulphonyl, propylsulphenyl or isopropylsulphonyl group or a group of formula -OR₃₁ wherein R₃₁ is hydrogen an alkyl group, or an alkyl group substituted with a hydroxy group, a carboxyl group, a phenyl group, a carbamoyl group, a mono- or di-alkylcarbomoyl group or a group of formula -NR₆R₇ wherein R₆ and R₇ are as defined above.

In a third aspect the invention provides an indoloylguanidine derivative of formula (1) wherein each R₁, which may be the same or different, is independently selected from hydrogen, an alkyl group, an alkenyl group, an alkynyl group, a cycloalkyl group, a halogen, a nitro group, a carboxyl group, an alkoxycarbonyl group and a group of formula -OR₃, -NR₆R₇ or -SO₂NR₆R₇.

In a fourth aspect the invention provides indoloylguanidine derivatives of formula (1) wherein each R₁, which may be the same or different, is independently selected from an alkyl group, an alkenyl group, a halogen, a nitro group and a group of formula -OR₃₂, wherein R₃₂ is hydrogen, an alkyl group or an alkyl group substituted by a hydroxy group, a carbamoyl group, a mono- or di-alkylcarbamoyl group or a group of formula -NR₆R₇ wherein R₆ and R₇ are as defined above.

In a fifth aspect the invention provides an indoloylguanidine derivative of formula (1) wherein R₂ is hydrogen, an alkyl group, a substituted alkyl group, a hydroxy group or an alkoxy group.

In a sixth aspect the invention provides an indoloylguanidine derivative of formula (1) which is a 2-indoloylguanidine compound.

In a seventh aspect the invention provides an indoloylguanidine derivative of formula (1) wherein each R₁, which may be the same or different, is independently selected from:
hydrogen,
an alkyl group having up to 8 carbon atoms,
a cycloalkyl group having 3 to 7 carbon atoms,
a halogen,
a nitro group, and
a group of formula: -OR₃ or -NR₆R₇;
R₂ represents
hydrogen,
an alkyl group having up to 8 carbon atoms, or
a substituted alkyl group having up to 8 carbon atoms in which the substituent is hydroxy, cyano, carboxyl, an alkoxycarbonyl group having up to 6 carbon atoms, an aryl group having up to 10 carbon atoms or carbamoyl;
R₃ represents
hydrogen,
an alkyl group having up to 8 carbon atoms,
a substituted alkyl group having up to 8 carbon atoms in which the substituent is a cycloalkyl group having 3 to 7 carbon atoms, an aryl group having up to 10 carbon atoms, a 5 or 6-membered heteroaryl group containing 1 to 4 nitrogen atoms or a 5 or 6-membered heteroaryl group containing 1 or 2 nitrogen atoms and one oxygen atom or one sulfur atom,
a cycloalkyl group having 3 to 7 carbon atoms, or
a group of formula: -CH₂R₃₀, wherein
R₃₀ represents an alkenyl group having up to 6 carbon atoms or an alkynyl group having 2 to 6 carbon atoms;
each of R₆ and R₇, which may be the same or different, is independently selected from:
hydrogen,
an alkyl group having up to 8 carbon atoms,
a substituted alkyl group having up to 8 carbon atoms in which the substituent is a cycloalkyl group having 3 to 7 carbon atoms, an aryl group having up to 10 carbon atoms, a 5 or 6-membered heteroaryl group containing 1 to 4 nitrogen atoms or a 5 or 6-membered heteroaryl group containing 1 or 2 nitrogen atoms and one oxygen atom or one sulfur atom,
a cycloalkyl group having 3 to 7 carbon atoms,
a group of formula: -CH₂R₆₀, wherein R₆₀ represents an alkenyl group having up to 6 carbon atoms or an alkynyl group having 2 to 6 carbon atoms, or
R₆ and R₇, together with the nitrogen atom to which they are attached, form a saturated 5- to 7-membered cyclic amino group containing 1 to 3 nitrogen atoms or a saturated 5- to 7-membered cyclic amino group containing one nitrogen atom and one oxygen atom;
and wherein the substituents R₁ and the guanidinocarbonyl group -C(=O)-N=C(NH₂)₂ are each, independently, attached to any one of the 5- and 6-membered rings of the indole nucleus.

In a preferred embodiment of this aspect each R₁, which may be identical or different, is independently selected from:
hydrogen,
an alkyl group having up to 8 carbon atoms,
a cycloalkyl group having 3 to 7 carbon atoms,
a halogen,
a nitro group, and
a group of formula: -OR₃, or -NR₆R₇,
R₂ represents:
hydrogen,
an alkyl group having up to 8 carbon atoms, or
a substituted alkyl group having up to 8 carbon atoms in which the substituent is hydroxy, cyano, carboxyl, an alkoxycarbonyl group having up to 6 carbon atoms, an aryl group having up to 10 carbon atoms or carbamoyl;
R₃ represents:
hydrogen,
an alkyl group having up to 8 carbon atoms, or
a cycloalkyl group having 3 to 7 carbon atoms,
each of R₆ and R₇, which may be the same of different, is independently selected from:
hydrogen,
an alkyl group having up to 8 carbon atoms,
a cycloalkyl group having 3 to 7 carbon atoms, and
R₆ and R₇, together with the nitrogen atom to which they are attached, form a saturated 5- to 7-membered cyclic amino group containing 1 to 3 nitrogen atoms or a saturated 5- to 7-membered cyclic amino group containing one nitrogen atom and one oxygen atom.

Preferably in formula (1) R₁ represents hydrogen, methyl or methoxy and R₂ represents methyl.

Preferred examples of indoloylguanidine derivatives of formula (1) include 1-methyl-2-indoloylguanidine and 1,4-dimethyl-2-indoloylguanidine.

In a ninth aspect the invention provides an indoloylguanidine derivative of formula (2) wherein R₈ is hydrogen and R₁₀ is hydrogen or a halogen.

Preferably in formula (2) R₉ is hydrogen, an alkyl group, a cycloalkyl, an alkenyl group, a halogen atom, a nitro group, an ethylsulfonyl, propylsulphonyl or isopropylsulphonyl group or a group of formula: -OR₃₃ wherein R₃₃ is hydrogen, an alkyl group or an aralkyl group.

In a further preferred embodiment of formula (2) each of R₁₁ and R₁₂, which may be the same or different, is independently selected from hydrogen, an alkyl group, a substituted alkyl group wherein the substituent is a hydroxy group or a group -NR₆R₇, an alkenyl group, a cycloalkyl group, a halogen, a nitro group, or a group of formula: -OR₃ or -NR₆R₇.

In particularly preferred indoloylguanidine derivatives of formula (2) R₂ is hydrogen, an alkyl group, a substituted alkyl group, a hydroxy group or an alkoxy group.

The invention further provides a process for the preparation of an indoloylguanidine derivative of formula (I) as defined above, or a salt thereof, the process comprising
(a) reacting a derivative of indolecarboxylic acid of the following formula (3): 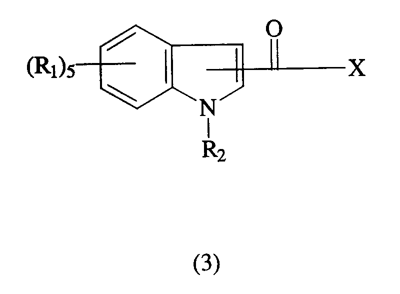 wherein R₁ and R₂ are as defined above and X is a leaving group, with guanidine in an inert solvent; or
(b) reacting an indolecarboxylic acid of the following formula (4): 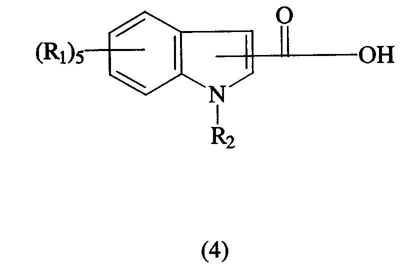 wherein R₁ and R₂ are as defined above, with guanidine in an inert solvent; or
(c) subjecting a compound of formula (5): 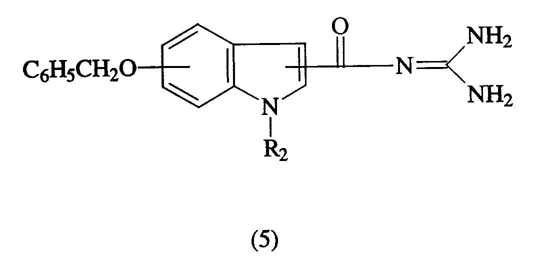 wherein R₂ is as defined above, to debenzylation, so as to obtain an indoloylguanidine derivative of formula (Ia): 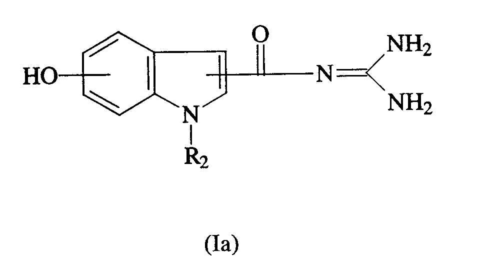 wherein R₂ is as defined above; or
(d) reducing a compound of formula (6): 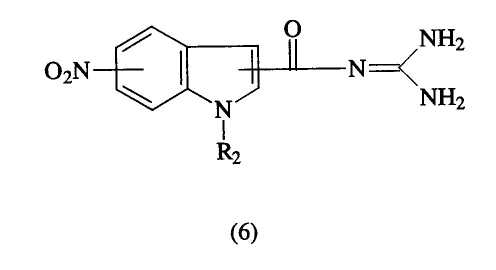 wherein R₂ is as defined above, so as to obtain an indoloylguanidine derivative of formula (Ib): 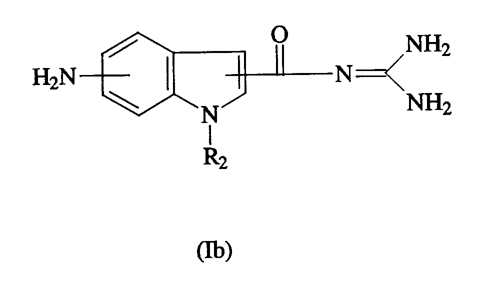 wherein R₂ is as defined as above; and/or, if desired,
(e) converting a compound of formula (I), prepared by any of the above processes (a) to (d), into a pharmaceutically acceptable salt.

In process variant (a), where the indolecarboxylic acid derivatives (3) contain reactive groups such as hydroxy or amino, these groups are previously protected by their protective groups. These protective groups are removed after the reaction is completed. The desired indoloylguanidine derivative (1) can thus be prepared.

As the reactive derivatives of the carboxylic acid, there are acid halides, acid anhydrides (including mixed acid anhydrides) and ester derivatives. Specific examples are acid chlorides and acid bromides as the acid halides; as the mixed acid anhydrides, there are mixed acid anhydrides with alkyloxy chloride type such as ethyloxycarbonyl chloride or isobutyloxycarbonyl chloride and those with α-polyalkyl-substituted carboxylate type such as diethylacetyl chloride or trimethylacetyl chloride; as the ester derivatives there are activated esters such as p-nitrophenyl esters, N-hydroxysuccinimide esters or pentafluorophenyl esters, and ordinary esters such as methyl esters or ethyl esters. These reactive derivatives of the carboxylic acids can be readily obtained from the corresponding carboxylic acids in a conventional manner.

In the case of performing the reaction between the acid halides or the acid anhydrides (including the mixed acid anhydrides) and guanidine, the reaction can be carried out in a solvent under cooling or at room temperature, in the presence of a base or an excess of guanidine. Inorganic bases such as sodium hydroxide, potassium hydroxide, sodium carbonate, potassium carbonate or sodium hydrogencarbonate, or organic bases such as triethylamine or pyridine may be used as the base. Examples of the solvent include aromatic hydrocarbons such as benzene, toluene or xylene, ethers such as tetrahydrofuran or 1,4-dioxane, halogenated hydrocarbons such as dichloromethane, chloroform or 1,2-dichloroethane, amides such as dimethylformamide or dimethylacetamide, basic solvents such as pyridine, or a mixture of these solvents.

Where the ester derivatives are reacted, the reaction is carried out in a solvent usually at a elevated temperature, in the presence of an equimolar amount of or an excess of guanidine. In the case of using the activated esters, the reaction is performed preferably in an ethers such as tetrahydrofuran, 1,2-dimethoxyethane or 1,4-dioxane, an ester type solvent such as ethyl acetate, dimethylformamide or a solvent mixture thereof. In the case of using other esters, it is preferred to perform the reaction in an alcohol type solvent such as methanol, ethanol or isopropanol, an ether type solvent such as tetrahydrofuran, 1,2-dimethoxyethane or 1,4-dioxane, dimethylformamide or a solvent mixture thereof. After removal of the solvent, if necessary and desired, the reaction system may be heated at about 130°C for a short period of time.

In process variant (b) the indolecarboxylic acid of formula (4) may be reacted with guanidine in an inert solvent either at room temperature or with heating, preferably in the presence of a condensing agent. The reaction may be depicted as follows:

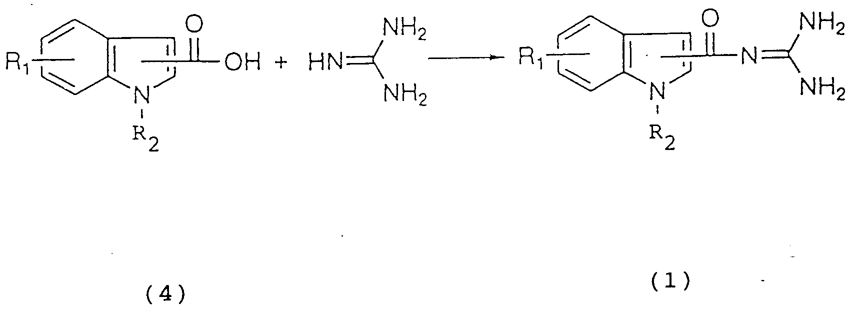

In this reaction, where the indolecarboxylic acid derivatives (4) contain reactive groups such as hydroxy or amino, these groups are previously protected by their protective groups. These protective groups are removed after the reaction is completed. The desired indoloylguanidine derivatives (1) can thus be prepared.

The reaction is carried out in a solvent, e.g., aromatic hydrocarbons such as benzene, toluene or xylene, ethers such as tetrahydrofuran or 1,4-dioxane, halogenated hydrocarbons such as dichloromethane, chloroform or 1,2-dichloroethane, amides such as dimethylformamide or dimethylacetamide, basic solvents such as pyridine, or a mixture of these solvents, in the presence of a condensing agent, e.g., dicyclohexylcarbodiimide (DCC), diisopropylcarbodiimide (DIPC), 1-ethyl-3-(3-dimethylaminopropyl)carbodiimide (WSC), benzotriazol-1-yl-tris(dimethylamino)phosphonium hexafluorophosphate (BOP), diphenylphosphonylazide (DPPA) or N,N-carbonyldiimidazole, cf., Angew. Chem. Int. Ed. Engl., Vol. 1, 351 (1962), and, if desired, in the presence of an additive such as N-hydroxysuccinimide (HONSu), 1-hydroxybenzotriazole (HOBt), 3-hydroxy-4-oxo-3,4-dihydro-1,2,3-benzotriazine (HOObt), etc.

In process variant (c) the debenzylation is carried out in a manner similar to the processes described in publications, such as catalytic hydrogenation using a palladium/carbon catalyst, c.f.,J.Chem. Soc., 1953, 4058 or decomposition under acidic conditions using hydrochloric acid/acetic acid c.f., J. Amer. Chem. Soc., Vol. 73, 5765 (1951).

In process variant (d) the nitroindoloylguanidine derivative of formula (6) may be reduced under conditions such as acidic conditions using zinc, iron, tin or tin (II) chloride, c.f,. Ann,. 641, 81 (1961), J. Amer. Chem. Soc., Vol. 66, 1781 (1944); reducing using sulfides such as sodium dithionite (Na₂S₂O₄), c.f., J. Amer. Chem. Soc, Vol. 72, 1361 (1950); catalytic hydrogenation using catalysts such as palladium/carbon, c.f., Synth. Commun., 1 47 (1971) or Raney nickel, c.f., Org. Synth., IV, 226 (1963).

As the protective groups for the hydroxy, amino or carboxyl group reactive with the reaction in the process variants (a) or (b) described hereinabove, there may be used protective groups conventionally used in the field of organic synthesis chemistry. Introduction and removal of these protective groups can be made in a conventional manner, e.g., Protective Groups in Organic Synthesis, John Wiley & Sons, 1991.

Examples of the protective group for the hydroxy group include methoxymethyl and tetrahydro-pyranyl. Examples of the protective group for the amino group include tert-butyloxycarbonyl and the like. These protective groups for the hydroxy group can be removed by conducting the reaction in a solvent such as hydrated methanol, hydrated ethanol or hydrated tetrahydrofuran in the presence of an acid, e.g., hydrochloric acid, sulfuric acid or acetic acid. The amino protective groups can be removed by performing the reaction in a solvent such as hydrated tetrahydrofuran, methylene chloride, chloroform or hydrated methanol, in the presence of an acid, e.g., hydrochloric acid or trifluoroacetic acid.

For protecting the carboxyl group, the protection is effected in the form of tert-butyl esters, ortho-esters or acid amides. Such protective groups are removed, in the case of the tert-butyl esters, e.g., by performing the reaction in a hydrated solvent in the presence of hydrochloric acid; in the case of the orthoesters, the protective groups are removed, e.g., by treating the protected compounds with an acid in a solvent such as hydrated methanol, hydrated tetrahydrofuran or hydrated 1,2-dimethoxyethane and then with an alkali such as sodium hydroxide. In the case of the acid amides, the protective groups are removed, e.g., by conducting the reaction in a solvent such as water, hydrated methanol or hydrated tetrahydrofuran in the presence of an acid such as hydrochloric acid or sulfuric acid.

The indolecarboxylic acids which are the starting compounds in the process variants (a) and (b) described hereinabove are commercially available. Examples of such commercially available indolecarboxylic acids are indole-5-carboxylic acid, 5-chloro-2-indole carboxylic acid, indole-3-carboxylic acid, indole-2 carboxylic acid, indole-4-carboxylic acid, 5-methoxy-2-indolecarboxylic acid. Alternatively, the indolecarboxylic acids may be prepared by known methods.

According to, e.g., the method of Reissert (Reissert's indole synthesis), there can be prepared 4-chloro-2-indolecarboxylic acid, cf., J. Chem. Soc., 1955, 3490; 6-n-amyl-2-indolecarboxylic acid, cf., J. Amer. Chem. Soc., Vol. 75, 4921 (1953); 7-indole-carboxylic acid, cf., J. Amer. Chem. Soc., Vol. 77, 5700 (1955);, 5-cyano-2-indolecarboxylic acid, cf., J. Org. Chem., Vol. 18, 354 (1953); 6-cyano-2-indolecarboxylic acid, cf., J. Chem. Soc., 1924, 2285; 6-benzyloxy-2-indolecarboxylic acid, cf., J. Chem. Soc., 1937, 1726 and the like.

The method of Fischer (Fischer's indole synthesis) gives nitro-2-indolecarboxylic acids in J. Amer. Chem. Soc., Vol. 80, 4621 (1958), 7-chloro-2-indolecarboxylic acid in J. Chem. Soc., 1955, 3499, 4-trifluoromethyl-2-indolecarboxylic acid in J. Amer. Chem. Soc., Vol. 79, 1745 (1957) and the like.

The 2-indolecarboxylic acids may also be prepared by known methods using benzaldehyde derivatives as the starting compounds, see, e.g., Tetrahedron, Vol. 42, 3259 (1986).

The 4-indolecarboxylic acids, 5-indolecarboxylic acids and 6-indolecarboxylic acids can be prepared based on the methods described in, e.g., J. Chem. Tech. Biotechnol., Vol. 36, 562 (1986), Tetrahedron Letters, Vol. 27, 1653 (1986), etc.

The 1-hydroxyindolecarboxylic acids can be prepared based on the method described in Chem. Ber., Vol. 56, 1024 (1923).

The compounds of formula (1) prepared as described above are illustratively given below.
1-methyl-2-indoloylguanidine
1-methyl-3-indoloylguanidine
1-methyl-4-indoloylguanidine
1-methyl-5-indoloylguanidine
1-methyl-6-indoloylguanidine
4-chloro-1-methyl-2-indoloylguanidine
5-chloro-1-methyl-2-indoloylguanidine
6-chloro-1-methyl-2-indoloylguanidine
7-chloro-1-methyl-2-indoloylguanidine
5-chloro-2-indoloylguanidine
1,4-dimethyl-2-indoloylguanidine
1,5-dimethyl-2-indoloylguanidine
1,6-dimethyl-2-indoloylguanidine
1,7-dimethyl-2-indoloylguanidine
4-methoxy-1-methyl-2-indoloylguanidine
5-methoxy-1-methyl-2-indoloylguanidine
6-methoxy-1-methyl-2-indoloylguanidine
7-methoxy-1-methyl-2-indoloylguanidine
1-methyl-4-nitro-2-indoloylguanidine
1-methyl-5-nitro-2-indoloylguanidine
1-methyl-6-nitro-2-indoloylguanidine
1-methyl-7-nitro-2-indoloylguanidine
4-amino-1-methyl-2-indoloylguanidine
5-amino-1-methyl-2-indoloylguanidine
6-amino-1-methyl-2-indoloylguanidine
7-amino-1-methyl-2-indoloylguanidine
1-benzyl-2-indoloylguanidine
1-benzyl-3-indoloylguanidine
1-benzyl-5-indoloylguanidine
1-isopropyl-2-indoloylguanidine
1-isopropyl-3-indoloylguanidine
1-isopropyl-5-indoloylguanidine
2-indoloylguanidine
3-indoloylguanidine
5-indoloylguanidine
4-hydroxy-1-methyl-2-indoloylguanidine
5-hydroxy-1-methyl-2-indoloylguanidine
6-hydroxy-1-methyl-2-indoloylguanidine
7-hydroxy-1-methyl-2-indoloylguanidine
1-(3-dimethylaminopropyl)-4-trifluoromethyl-2-indoloylguanidine
1-(3-diethylaminopropyl)-4-trifluoromethyl-2-indoloylguanidine
1-[3-(N-pyrrolidinyl)propyl]-4-trifluoromethyl-2-indoloylguanidine
6-(3-aminopropoxy)-1-methyl-4-trifluoromethyl-2-indoloylguanidine
6-(3-dimethylaminopropoxy)-1-methyl-4-trifluoromethyl-2-indoloylguanidine
6-(3-diethylaminopropoxy)-1-methyl-4-trifluoromethyl-2-indoloylguanidine
6-(2-aminoethoxy)-1-methyl-4-trifluoromethyl-2-indoloylguanidine
6-(2-dimethylaminoethoxy)-1-methyl-4-trifluoromethyl-2-indoloylguanidine
6-(2-diethylaminoethoxy)-1-methyl-4-trifluoromethyl-2-indoloylguanidine
1-methyl-6-[3-(N-pyrrolidinyl)propoxy]-4-trifluoromethyl-2-indoloylguanidine
1-methyl-6-[2-(N-pyrrolidinyl)ethoxy]-4-trifluoromethyl-2-indoloylguanidine
1-(3-dimethylaminopropyl)-4-methoxy-2-indoloylguanidine
1-(3-diethylaminopropyl)-4-methoxy-2-indoloylguanidine
1-(3-aminopropyl)-4-methoxy-2-indoloylguanidine
4-methoxy-1-[3-(N-pyrrolidinyl)propoyl]-2-indoloylguanidine
6-(3-aminopropoxy)-4-methoxy-1-methyl-2-indoloylguanidine
6-(3-dimethylaminopropoxy)-4-methoxy-1-methyl-2-indoloylguanidine
6-(3-diethylaminopropoxy)-4-methoxy-1-methyl-2-indoloylguanidine
6-(2-aminoethoxy)-4-methoxy-1-methyl-2-indoloylguanidine
6-(2-dimethylaminoethoxy)-4-methoxy-1-methyl-2-indoloylguanidine
6-(2-diethylaminoethoxy)-4-methoxy-1-methyl-2-indoloylguanidine
4-methoxy-1-methyl-6-[3-(N-pyrrolidinyl)-propoxy]-2-indoloylguanidine
4-methoxy-1-methyl-6-[2-(N-pyrrolidinyl)-ethoxy]-2-indoloylguanidine
7-(3-aminopropoxy)-4-methoxy-1-methyl-2-indoloylguanidine
7-(3-dimethylaminopropoxy)-4-methoxy-1-methyl-2-indoloylguanidine
7-(3-diethylaminopropoxy)-4-methoxy-1-methyl-2-indoloylguanidine
4-methoxy-1-methyl-7-[3-(N-pyrrolidinyl)-propoxy]-2-indoloylguanidine
1-(3-dimethylaminopropyl)-4-isopropoxy-2-indoloylguanidine
1-(3-diethylaminopropyl)-4-isopropoxy-2-indoloylguanidine
1-(3-aminopropyl)-4-isopropoxy-2-indoloylguanidine
4-isopropoxy-1-[3-(N-pyrrolidinyl)propyl]-2-indoloylguanidine
6-(3-aminopropoxy)-4-isopropoxy-1-methyl-2-indoloylguanidine
6-(3-dimethylaminopropoxy)-4-isopropoxy-1-methyl-2-indoloylguanidine
6-(3-diethylaminopropoxy)-4-isopropoxy-1-methyl-2-indoloylguanidine
6-(2-aminoethoxy)-4-isopropoxy-1-methyl-2-indoloylguanidine
6-(2-dimethylaminoethoxy)-4-isopropoxy-1-methyl-2-indoloylguanidine
6-(2-diethylaminoethoxy)-4-isopropoxy-1-methyl-2-indoloylguanidine
4-isopropoxy-1-methyl-6-[3-(N-pyrrolidinyl)propoxy]-2-indoloylguanidine
4-isopropoxy-1-methyl-6-[2-(N-pyrrolidinyl)ethoxy]-2-indoloylguanidine
7-(3-aminopropoxy)-4-isopropoxy-1-methyl-2-indoloylguanidine
7-(3-dimethylaminopropoxy)-4-isopropoxy-1-methyl-2-indoloylguanidine
7-(3-diethylaminopropoxy)-4-isopropoxy-1-methyl-2-indoloylguanidine
4-isopropoxy-1-methyl-7-[3-(N-pyrrolidinyl)propoxy]-2-indoloylguanidine
1-(3-aminopropyl)-4-methyl-2-indoloylguanidine
1-(3-dimethylaminopropyl)-4-methyl-2-indoloylguanidine
1-(3-diethylaminopropyl)-4-methyl-2-indoloylguanidine
4-methyl-1-[3-(N-pyrrolidinyl)propyl]-2-indoloylguanidine
6-(3-aminopropoxy)-1,4-dimethyl-2-indoloylguanidine
1,4-dimethyl-6-(3-dimethylaminopropoxy)-2-indoloylguanidine
6-(3-diethylaminopropoxy)-1,4-dimethyl-2-indoloylguanidine
1,4-dimethyl-6-(2-diethylaminoethoxy)-2-indoloylguanidine
6-(2-diethylaminoethoxy)-1,4-dimethyl-2-indoloylguanidine
6-(2-aminoethoxy)-1,4-dimethyl-2-indoloylguanidine
1,4-dimethyl-6-[3-(N-pyrrolidinyl)propoxy]-2-indoloylguanidine
1,4-dimethyl-6-[2-N-pyrrolidinyl)ethoxy]-2-indoloylguanidine
7-(3-aminopropoxy)-1,4-dimethyl-2-indoloylguanidine
1,4-dimethyl-7-(3-dimethylaminopropoxy)-2-indoloylguanidine
7-(3-diethylaminopropoxy)-1,4-dimethyl-2-indoloylguanidine
1,4-dimethyl-7-[3-(N-pyrrolidinyl)propoxy]-2-indoloylguanidine
4-tert-butyl-1-methyl-2-indoloylguanidine
1-(3-aminopropyl)-4-tert-butyl-2-indoloylguanidine
4-tert-butyl-1-(3-dimethylaminopropyl)-2-indoloylguanidine
4-tert-butyl-1-(3-diethylaminopropyl)-2-indoloylguanidine
4-tert-butyl-1-[3-(N-pyrrolidinyl)propyl]-2-indoloylguanidine
6-(3-dimethylaminopropoxy)-1-methyl-2-indoloylguanidine
6-(3-diethylaminopropoxy)-1-methyl-2-indoloylguanidine
6-(2-aminoethoxy)-1-methyl-2-indoloylguanidine
6-(2-dimethylaminoethoxy)-1-methyl-2-indoloylguanidine
6-(2-diethylaminoethoxy)-1-methyl-2-indoloylguanidine
1-methyl-6-[3-(N-pyrrolidinyl)propoxy]-2-indoloylguanidine
1-methyl-6-[2-(N-pyrrolidinyl)ethoxy]-2-indoloylguanidine
7-(3-dimethylaminopropoxy)-1-methyl-2-indoloylguanidine
7-(3-diethylaminopropoxy)-1-methyl-2-indoloylguanidine
1-methyl-7-[3-(N-pyrrolidinyl)propoxy]-2-indoloylguanidine

The compounds represented by formula (1) may be converted into acid addition salts with pharmaceutically acceptable inorganic acids or organic acids, if necessary and desired. Examples of such acid addition salts are salts with inorganic acids such as hydrochloric acid, hydrobromic acid, sulfuric acid or phosphoric acid; salts with organic acids such as formic acid, acetic acid, fumaric acid, maleic acid, oxalic acid, citric acid, malic acid, tartaric acid, aspartic acid or glutamic acid; salts with sulfonic acids such as methanesulfonic acid, benzenesulfonic acid, p-toluenesulfonic acid, hydroxybenzenesulfonic acid, dihydroxybenzenesulfonic acid, etc.

The compounds of the present invention inhibit the sodium/proton (Na⁺/H⁺) exchanger system and are thus useful for the treatment and prevention of diseases caused by increased sodium/proton (Na⁺/H⁺) exchanger activity, for example, hypertension, arrhythmia, angina pectoris, cardiac hypertrophy, organ disorders associated with ischemic reperfusion (e.g., myocardial ischemic reperfusion disturbance, disorders caused by surgical treatment (e.g., organ transplantation or PTCA), cerebroischemic disorders (e.g., disorders associated with cerebral infarction, disorders caused after cerebral apoplexy as sequelae or cerebral edema), or diseases (e.g., atherosclerosis, pulmonary fibrosis, hepatic fibrosis, renal fibrosis, glomerular nephrosclerosis, organ hypertrophy, prostatic hypertrophy, diabetic complications or recurrent stricture after PTCA) caused by excessive cell proliferation such as proliferation of fibroblast, proliferation of smooth muscle cells or proliferation of mesangium cells).

The present invention further provides a compound of the invention as defined above for use in a method of treatment of the human or animal body by therapy, and the use of a compound of the invention as defined above in the manufacture of a medicament for the treatment of a disease caused by increased Na⁺/H⁺ exchanger activity.

The compounds of the present invention can be prepared in the form of pharmaceutical preparations which are suitable for oral or parenteral administration. These pharmaceutical preparations can be administered orally in the form of powders, granules, tablets, capsules, syrup or suspensions; alternatively, parenterally in the form of injections using its solution, emulsion or suspension. The pharmaceutical preparations may also be administered rectally in the form of suppositories.

These pharmaceutical compositions can be prepared by mixing the compound of the present invention as the active ingredient with a conventionally acceptable carrier, a recipient, a binder, a stabilizer and a diluent. In the case of using the compound of the present invention in the form of injection, a pharmaceutically acceptable buffer, a dissolution aid or an isotonic agent may also be incorporated in the composition.

Dosage and time of administration may vary depending upon the disease, condition, age, body weight and mode of administration but the composition is administered in a daily dose of 0.1 to 2000 mg, preferably

The present invention further provides a compound of the invention as defined above for use in a method of treatment of the human or animal body by therapy, and the use of a compound of the invention as defined above in the manufacture of a medicament for the treatment of a disease caused by increased Na⁺/H⁺ exchanger activity.

### Reference Example 1

### Preparation of 7-chloro-2-indolecarboxylic acid (Fischer's indole synthesis)

### a) Preparation of ethyl 2-(2-chlorophenyl)hydrazonopropionate

To a solution of 14.4 g (0.10 mol) of ethyl 2-methylacetacetate in 100 ml of ethanol was added dropwise 50 g of 50% potassium hydroxide aqueous solution at 0°C. After 70 g of ice was added to the solution, a diazonium salt solution prepared by mixing 12.8 g (0.10 mol) of o-chloroaniline, 13.6 g (0.20 mol) of sodium nitrite and 60 g of conc. hydrochloric acid was added to the mixture at once. The reaction mixture was stirred at 0°C for 30 minutes. The precipitates were cellected and dried under reduced pressure to give 9.10 g (37.7%) of the desired ethyl 2-(2-chlorophenyl)hydrazonopropionate.

### b) Preparation of ethyl 7-chloro-2-indolecarboxylate

After 8.00 g (33.2 mmol) of ethyl 2-(2-chlorophenyl)hydrazonopropionate obtained above was added to 20 g of polyphosphoric acid, the mixture was gradually heated to 190°C, which was kept for 5 minutes. The reaction mixture was cooled to 60°C and water was then added thereto. The mixture was extracted 3 times with ethyl acetate. The combined extracts were washed with water. After drying over anhydrous magnesium sulfate, the solvent was distilled off under reduced pressure. The resulting residue was purified by silica gel column chromatography to obtain 3.40 g (45.7%) of the desired ethyl 7-chloro-2-indolecarboxylate.
¹H NMR (CDCl₃) δ: 1.40-1.46 (3H, m), 4,43 (2H, dd, J=7.3, 14.2Hz), 7.09 (1H, t, J=7.9Hz), 7.25 (1H, d, J=2.3Hz), 7.32 (1H, dd, J=1.0, 7.6Hz), 7.58-7.61 (1H, m), 9.02 (1H, br-s).

The following compounds were prepared by carrying out the reaction in a manner similar to Reference Example 1.
(1) Ethyl 5-nitro-2-indolecarboxylate:
   ¹H NMR (CDCl₃) δ: 1.42-1.47 (3H, m), 4.45 (2H, dd, J=7.3, 14.2Hz), 7.38 (1H, dd, J=0.7, 2.0Hz), 7.50 (1H, d, J=9.3Hz), 8.21-8.25 (1H, m), 8.69 (1H, d, J=2.0Hz), 9.3 (1H, br-s).
(2) Ethyl 7-nitro-2-indolecarboxylate:
   ¹H NMR (CDCl₃) δ: 1.42-1.48 (3H, m), 4.43-4.51 (2H, m), 7.25-7.28) (1H, m), 7.37 (1H, d, J=2.3Hz), 8.04-8.08 (1H, m), 8.31 (1H, dd, J=1.0, 7.9Hz), 10.4 (1H, br-s).
(3) Ethyl 4-methoxy-2-indolecarboxylate:
   ¹H NMR (CDCl₃) δ: 1.38-1.43 (3H, m), 3.96 (3H, s), 4.36-4.44 (2H, m), 6.51 (1H, d, J=7.9Hz), 7.01 (1H, d, J=8.3Hz), 7.22 (1H, d, J=7.9Hz), 7.34-7.35 (1H, m), 8.9 (1H, br-s).
(4) Ethyl 6-methoxy-2-indolecarboxylate:
   ¹H NMR (CDCl₃) δ: 1.38-1.43 (3H, m), 3.85 (3H, s), 4.39 (2H, dd, J=7.3, 14.2Hz), 6.80-6.84 (2H, m), 7.16-7.17 (1H, m), 7.52-7.56 (1H, m), 8.9 (1H, br-s).
(5) Ethyl 4-nitro-2-indolecarboxylate:
   ¹H NMR (CDCl₃) δ: 1.44-1.49 (3H, m), 4.43-4.51 (2H, m), 7.41-7.47 (1H, m), 7.77-7.80 (1H, m), 7.92 (1H, dd, J=1.0, 2.3Hz), 8.20 (1H, dd, J=0.7, 7.9Hz), 9.4 (1H, br-s).
(6) Ethyl 6-nitro-2-indolecarboxylate:
   ¹H NMR (CDCl₃) δ: 1.46 (3H, t, J=7.3Hz), 4.48 (2H, dd, J=7.3, 14.2Hz), 7.29-7.30 (1H, m), 7.78 (1H, d, J=8.9Hz), 8.05 (1H, dd, J=2.0, 8.9Hz), 8.42 (1H, t, J=1.0Hz), 9.6 (1H, br-s).
(7) Ethyl 4-trifluoromethyl-2-indolecarboxylate:
   ¹H NMR (CDCl₃) δ: 1.42-1.47 (3H, m), 4.45 (2H, dd, J=6.9, 14.2Hz), 7.35-7.41 (2H, m), 7.46-7.49 (1H, m), 7.62 (1H, d, J=8.3Hz), 9.32 (1H, br-s).
(8) Ethyl 6-trifluoromethyl-2-indolecarboxylate:
   ¹H NMR (CDCl₃) δ: 1.41-1.47 (3H, m), 4.41-4.49 (2H, m), 7.26-7.27 (1H, m), 7.36-7.40 (1H, m), 7.73-7.81 (2H, m), 9.26 (1H, br-s).
(9) Ethyl 7-phenyl-2-indolecarboxylate:
   ¹H NMR (CDCl₃) δ: 1.28-1.43 (3H, m), 4.41 (2H, dd, J=6.9, 14.2Hz), 7.20-7.26 (1H, m), 7.35-7.57 (6H, m), 7.66-7.70 (2H, m), 9.11 (1H, br-s).
(10) Ethyl 4-acetyl-2-indolecarboxylate:
   ¹H NMR (CDCl₃) δ: 1.41-1.47 (3H, m), 2.72 (3H s), 4.40-4.48 (2H, m), 7.38 (1H, dd, J=7.3, 8.2Hz), 7.66 (1H, dd, J=1.0, 8.3Hz), 7.78 (1H, dd, J=1.0, 7.3Hz), 7.99-8.00 (1H, m), 9.42 (1H, br-s).

### Reference Example 2

### Preparation of 4-methyl-2-indolecarboxylic acid (Reissert's indole synthesis)

### a) Preparation of (6-methyl-2-nitrophenyl)pyruvic acid

A solution of 15.1 g (0.10 mol) of 2-methyl-3-nitrotoluene and 14.6 g (0.10 mol) of diethyl oxalate in 10 ml of ethanol was added to a solution of 11.2 g (0.10 mol) of potassium tert-butoxide in 50 ml of ethanol. After stirring at room temperature for 1.5 hour, the reaction mixture was refluxed for 1.5 hour. After 60 ml of water was added to the reaction mixture, the mixture was refluxed for further an hour. After cooling, ice water was poured onto the reaction mixture followed by washing twice with ethyl acetate. The aqueous layer was acidified with conc. hydrochloric acid and then extracted three times with chloroform. The combined extracts were washed with water. After drying over anhydrous magnesium sulfate, the solvent was distilled off under reduced pressure to give 10.4 g (46.6%) of the desired 6-methyl-2-nitrophenylpyruvic acid.

### b) Preparation of 4-methyl-2-indolecarboxylic acid

A 5% aqueous ammonia of 10.4 g (46.6 mmol) of 6-methyl-2-nitrophenylpyruvic acid obtained above was added to a suspension of 96.4 g (0.33 mol) of ferric sulfate heptahydrate in 324 ml of water containing 37 ml of 28% aqueous ammonia. The mixture was refluxed for 10 minutes. After insoluble matters were filtered off, the filtrate was acidified with conc. hydrochloric acid followed by extracting three times with ethyl acetate. The combined extracts were washed with saturated sodium chloride solution and dried over anhydrous magnesium sulfate. The solvent was distilled off under reduced pressure to give 4.30 g (24.5%, yield based on 2-methyl-3-nitrotoluene) of the desired 4-methyl-2-indolecarboxylic acid.
¹H NMR (DMSO-d₆) δ: 2.49 (3H, s), 6.83 (1H, d, J=6.3Hz), 7.08-7.14 (2H, m), 7.25 (1H, d, J=8.3Hz), 11.7 (1H, br-s), 12.8 (1H, br-s).

The following compounds were prepared in a manner similar to Reference Example 2.
(1) 4-Chloro-2-indolecarboxylic acid:
   ¹H NMR (DMSO-d₆) δ: 7.06 (1H, d, J=2.0Hz), 7.16 (1H, d, J=7.6Hz), 7.22-7.28 (1H, m), 7.42 (1H, d, J=7.9Hz), 12.2 (1H, br-s), 13.2 (1H, br-s).
(2) 6-Chloro-2-indolecarboxylic acid:
   ¹H NMR (DMSO-d₆) δ: 7.04-7.10 (2H, m), 7.43 (1H, d, J=0.7Hz), 7.65 (1H, d, J=8.6Hz), 11.9 (1H, br-s), 13.0 (1H, br-s).
(3) 5-Methyl-2-indolecarboxylic acid:
   ¹H NMR (DMSO-d₆) δ: 2.36 (3H, s), 6.98 (1H, dd, J=1.0, 2.0Hz), 7.04-7.08 (1H, m), 7.30-7.33 (1H, m), 7.40 (1H, s), 11.6 (1H, br-s), 12.9 (1H, br-s).
(4) 6-Methyl-2-indolecarboxylic acid:
   ¹H NMR (DMSO-₆) δ: 2.40 (3H, s), 6.87-6.90 (1H, m), 7.00-7.01 (1H, m), 7.21 (1H, s), 7.50 (1H, d, J=8.3Hz), 11.6 (1H, br-s), 12.7 (1H, br-s).
(5) 7-Methyl-2-indolecarboxylic acid:
   ¹H NMR (DMSO-₆) δ: 2.52 (3H, s), 6.93-7.02 (2H, m), 7.09 (1H, d, J=2.0Hz), 11.5 (1H, br-s), 12.8 (1H, br-s).
(6) 7-Benzyloxy-2-indolecarboxylic acid:
   ¹H NMR (DMSO-₆) δ: 5.27 (2H, s), 6.86 (1H, d, J=7.3Hz), 6.94-7.00 (1H, m), 7.07 (1H, dd, J=2.0, 7.3Hz), 7.17-7.23 (1H, m), 7.31-7.43 (3H, m), 7.65 (2H, d, J=6.9Hz), 11.82 (1H, br-s), 12.81 (1H, br-s).
(7) 4-Benzyloxy-2-indolecarboxylic acid:
   ¹H NMR (DMSO-₆) δ: 5.24 (2H, s), 6.62 (1H, d, J=6.9Hz), 7.00-7.17 (3H, m), 7.31-7.44 (3H, m), 7.50-7.53 (2H, m), 11.78 (1H, br-s), 12.85 (1H, br-s).

### Reference Example 3

### Preparation of Methyl 6-indolecarboxylate

### a) Preparation of methyl 4-chloro-3-nitrobenzoate

To a solution of 10.0 g (49.6 mmol) of 4-chloro-3-nitrobenzoic acid in 100 ml of methanol was added dropwise 11.8 g (99.2 mmol) of thionyl chloride at 0°C. The reaction mixture was refluxed for 2 hours and the solvent was then distilled off under reduced pressure. Ice water was added to the resulting residue. The mixture was made basic by the addition of concentrated ammonium hydroxide. The mixture was extracted three times with ethyl acetate. The combined extracts were washed with water and dried over anhydrous magnesium sulfate. The solvent was then distilled off under reduced pressure to give 10.9 g (>99%) of the desired methyl 4-chloro-3-nitrobenzoate.

### b) Preparation of methyl 3-nitro-4-trimethylsilylethynylbenzoate

A mixture of 10.7 g (49.6 mmol) of methyl 4-chloro-3-nitrobenzoate obtained above, 8.77 g (89.3 mmols) of trimethylsilylacetylene, 0.4 g of dichloro-bis(triphenylphosphine)palladium and 120 ml of triethylamine was heated at 75°C for 3 hours with stirring. The reaction mixture was cooled. After insoluble matters were filtered off, the extract was distilled off under reduced pressure. The resulting residue was purified by silica gel column chromatography to obtain 8.40 g (61.0%) of methyl 3-nitro-4-trimethylsilylethynylbenzoate.

### c) Preparation of methyl 4-(2,2-dimethoxyethyl)-3-nitrobenzoate

To a methanol solution of 2.92 g (54.1 mmol) of sodium methoxide was added 3.00 g (10.8 mmol) of methyl 3-nitro-4-trimethylsilylethynylbenzoate prepared above. The mixture was refluxed for 30 minutes. After cooling to 0°C, 5.52 g (54.1 mmol) of acetic acid was added to the reaction mixture and the solvent was then distilled off under reduced pressure. Ice water was poured onto the resulting residue followed by extraction three times with dichloromethane. After drying over anhydrous magnesium sulfate, the solvent was distilled off under reduced pressure and the residue was purified by silica gel column chromatography to give 2.40 g (82.4%) of methyl 4-(2,2-dimethoxyethyl)-3-nitrobenzoate.

### d) Preparation of methyl 3-amino-4-(2,2-dimethoxyethyl)benzoate

To a mixture of 4.40 g (16.3 mmol) of methyl 4-(2,2-dimethoxyethyl)-3-nitrobenzoate in a solvent mixture of 200 ml of methanol and 2 ml of acetic acid was added 0.50 g of 5% palladium-carbon to perform catalytic hydrogenation at ambient temperature under normal pressure and then treat the reaction mixture in a conventional manner. Thus, 4.16 g of methyl 3-amino-4-(2,2-dimethoxyethyl)benzoate was obtained.

### e) Preparation of methyl 6-indolecarboxylate

After 4.00 g (16.7 mmol) of methyl 3-amino-4-(2,2-dimethoxyethyl)benzoate obtained above was added to a solution of 5 ml of 1N hydrochloric acid in 15 ml of ethanol, the mixture was heated at 60°C for an hour. The reaction mixture was poured onto ice water followed by extraction three times with ethyl acetate. The combined extracts were then washed with water. After drying over anhydrous magnesium sulfate, the solvent was then distilled off under reduced pressure to give 3.00 g (>99%) of methyl 6-indolecarboxylate.
¹H NMR (CDCl₃) δ: 3.95 (3H, s), 7.13-7.45 (4H, m), 7.68-7.72 (1H, m), 8.94 (1H, br-s).

### Reference Example 4

### Preparation of methyl 1-methyl-2-indolecarboxylate

After 2.00 g (12.4 mmol) of 2-indole-carboxylic acid was added to a suspension of 0.99 g (24.8 mmol) of 60% sodium hydride in 40 ml of dimethylformamide, the mixture was stirred at room temperature until the mixture became a transparent solution. A solution of 7.05 g (49.6 mmol) of methyl iodide in 10 ml of dimethylformamide was then added dropwise to the transparent solution at room temperature followed by stirring at the same temperature for 5 hours. The reaction mixture was poured onto ice water. The resulting mixture was then extracted three times with ethyl acetate. The combined extracts were washed with water. After drying over anhydrous magnesium sulfate, the solvent was distilled off under reduced pressure and the residue was recrystallized from n-hexane to give 1.70 g (72.4%) of methyl 1-methyl-2-indolecarboxylate.
¹H NMR (CDCl₃) δ: 3.91 (3H, s), 4.08 (3H, s), 7.12-7.18 (1H, m), 7.30 (1H, s), 7.32-7.41 (2H, m), 7.66-7.70 (1H, m).

The following compounds were prepared in a manner similar to Reference Example 4.
(1) Methyl 1-methyl-5-indolecarboxylate:
   ¹H NMR (CDCl₃) δ: 3.82 (3H, s), 3.93 (3H, s), 6.58 (1H, dd, J=1.0, 3.3Hz), 7.11 (1H, d, J=3.3Hz), 7.32 (1H, d, J=8.6Hz), 7.91-7.95 (1H, m), 8.39-8.40 (1H, m).
(2) Methyl 1-methyl-3-indolecarboxylate:
   ¹H NMR (CDCl₃) δ: 3.82 (3H, s), 3.91 (3H, s), 7.24-7.37 (3H, m), 7.77 (1H, s), 8.14-8.20 (1H, m).
(3) Methyl 1-methyl-4-indolecarboxylate:
   ¹H NMR (CDCl₃) δ: 3.84 (3H, s), 3.98 (3H, s), 7.10-7.11 (1H, m), 7.20 (1H, d, J=3.0Hz), 7.24-7.29 (1H, m), 7.53 (1H, d, J=8.2Hz), 7.91 (1H, dd, J=1.0, 7.6Hz).
(4) Methyl 4-chloro-1-methyl-2-indolecarboxylate:
   ¹H NMR (CDCl₃) δ: 3.60 (3H, s), 3.75 (3H, s), 6.80-6.83 (1H, m), 6.89-6.95 (2H, m), 7.05 (1H, d, J=0.7Hz).
(5) Methyl 5-chloro-1-methyl-2-indolecarboxylate:
   ¹H NMR (CDCl₃) δ: 3.64 (3H, s), 3.78 (3H, s), 6.93 (1H, s), 6.97-7.02 (2H, m), 7.36 (1H, t, J=1.3Hz).
(6) Methyl 6-chloro-1-methyl-2-indolecarboxylate:
   ¹H NMR (CDCl₃) δ: 3.91 (3H, s), 4.04 (3H, s), 7.09-7.13 (1H, m), 7.25-7.26 (1H, m), 7.38-7.39 (1H, m), 7.56-7.59 (1H, m).
(7) Methyl 7-chloro-1-methyl-2-indolecarboxylate:
   ¹H NMR (CDCl₃) δ: 3.91 (3H, s), 4.47 (3H, s), 6.99 (1H, m), 7.26-7.30 (2H, m), 7.52-7.56 (1H, m).
(8) Methyl 1,4-dimethyl-2-indolecarboxylate:
   ¹H NMR (CDCl₃) δ: 2.56 (3H, s), 3.92 (3H, s), 4.07 (3H, s), 6.93-6.96 (1H, m), 7.17-7.29 (2H, m), 7.33 (1H, d, J=0.7Hz).
(9) Methyl 1,5-dimethyl-2-indolecarboxylate:
   ¹H NMR (CDCl₃) δ: 2.44 (3H, s), 3.90 (3H, s), 4.05 (3H, s), 7.16-7.29 (3H, m), 7.42-7.45 (1H, m).
(10) Methyl 1,6-dimethyl-2-indolecarboxylate:
   ¹H NMR (CDCl₃) δ: 2.51 (3H, s), 3.90 (3H, s), 4.05 (3H, s), 6.99 (1H, dd, J=1.0, 8.3Hz), 7.12-7.16 (1H, m), 7.24-7.26 (1H, m), 7.55 (1H, d, J=8.2Hz), 7.42-7.45 (1H, m).
(11) Methyl 1,7-dimethyl-2-indolecarboxylate:
   ¹H NMR (CDCl₃) δ: 2.80 (3H, s), 3.89 (3H, s), 4.35 (3H, s), 6.97 (2H, m), 7.25-7.27 (1H, m), 7.26 (1H, s), 7.48 (1H, d, J=7.3Hz).
(12) Methyl 1-methyl-5-methoxy-2-indolecarboxylate:
   ¹H NMR (CDCl₃) δ: 3.85 (3H, s), 3.90 (3H, s), 4.05 (3H, s), 7.00-7.09 (2H, m), 7.19-7.30 (2H, m).
(13) Benzyl 1-benzyl-5-indolecarboxylate:
   ¹H NMR (CDCl₃) δ: 5.33 (2H, s), 5.38 (2H, s), 6.64 (1H, d, J=3.3Hz), 7.06-7.49 (12H, m), 7.92 (1H, dd, J=1.7, 8.9Hz), 8.45-8.46 (1H, m).
(14) Isopropyl 1-isopropyl-5-indolecarboxylate:
   ¹H NMR (CDCl₃) δ: 1.38 (6H, d, J=6.3Hz), 1.53 (6H, d, J=6.6Hz), 4.62-4.75 (1H, m), 5.21-5.35 (1H, m), 6.60 (1H, d, J=3.3Hz), 7.27 (1H, d, J=3.3Hz), 7.36 (1H, d, J=8.6Hz), 7.90 (1H, dd, J=1.7, 8.6Hz), 8.38 (1H, d, J=1.7Hz).

### Reference Example 5

### Preparation of methyl 1-methyl-6-indolecarboxylate

The reaction was carried out in a manner similar to Reference Example 4 except for using 3.00 (17.1 mmol) of methyl 6-indolecarboxylate, 0.68 g (17.1 mmol) of 60% sodium hydroxide, 4.86 g (34.4 mmol) of methyl iodide and 60 ml of dimethylformamide. Thus 2.75 g (86.9%) of methyl 1-methyl-6-indolecarboxylate was obtained.
¹H NMR (CDCl₃) δ: 3.86 (3H, s), 3.95 (3H, s), 6.51-6.53 (1H, m), 7.21 (1H, d, J=3.3Hz), 7.63 (1H, d, J=8.6Hz), 7.78-7.82 (1H, m), 8.10 (1H, s).

The following compounds were prepared in a manner similar to Reference Example 5.
(1) Ethyl 4-methoxy-1-methyl-2-indolecarboxylate:
   ¹H NMR (CDCl₃) δ: 1.39 (3H, t, J=7.3Hz), 3.96 (3H, s), 4.06 (3H, s), 4.35 (2H, dd, J=7.3, 14.2Hz), 6.50 (1H, d, J=7.6Hz), 6.98 (1H, d, J=8.6Hz), 7.24-7.30 (1H, m), 7.42 (1H, d, J=0.7Hz).
(2) Ethyl 6-methoxy-1-methyl-2-indolecarboxylate:
   ¹H NMR (CDCl₃) δ: 1.37-1.42 (3H, m), 3.89 (3H, s), 4.03 (3H, s), 4.31-4.39 (2H, m), 6.75 (1H, s), 6.80-6.84 (1H, m), 7.25 (1H, s), 7.53 (1H, d, J=8.9Hz).
(3) Ethyl 1-methyl-4-nitro-2-indolecarboxylate:
   ¹H NMR (CDCl₃) δ: 1.45 (3H, t, J=7.3Hz), 4.17 (3H, s), 4.39-4.47 (2H, m), 7.41-7.48 (1H, m), 7.74-7.77 (1H, m), 7.96 (1H, d, J=1.0Hz), 8.18-8.21 (1H, m).
(4) Ethyl 1-methyl-6-nitro-2-indolecarboxylate:
   ¹H NMR (CDCl₃) δ: 1.41-1.46 (3H, m), 4.17 (3H, s), 4.38-4.46 (2H, m), 7.34 (1H, d, J=1.0Hz), 7.75 (1H, dd, J=0.7, 8.9Hz), 8.03 (1H, dd, J=2.0, 8.9Hz), 8.39 (1H, d, J=2.0Hz).
(5) Ethyl 1-methyl-5-nitro-2-indolecarboxylate:
   ¹H NMR (CDCl₃) δ: 1.41-1.46 (3H, m), 4.14 (3H, s), 4.41 (2H, dd, J=7.3, 14.2Hz), 7.42-7.46 (2H, m), 8.22-8.26 (1H, m), 8.66 (1H, d, J=2.0Hz).
(6) Ethyl 1-methyl-7-nitro-2-indolecarboxylate:
   ¹H NMR (CDCl₃) δ: 1.43 (3H, t, J=7.3Hz), 4.00 (3H, s), 4.37-4.45 (2H, m), 7.20 (1H, t, J=7.9Hz), 7.43 (1H, s), 7.85-7.93 (2H, m).
(7) Methyl 1-benzyl-2-indolecarboxylate:
   ¹H NMR (CDCl₃) δ: 3.86 (3H, s), 5.84 (2H, s), 7.02-7.06 (2H, m), 7.13-7.44 (7H, m), 7.70-7.73 (1H, m).
(8) Methyl 1-benzyl-3-indolecarboxylate:
   ¹H NMR (CDCl₃) δ: 3.91 (3H, s), 5.34 (2H, s), 7.13-7.17 (2H, m), 7.20-7.36 (6H, m), 7.85 (1H, s), 8.17-8.21 (1H, m).
(9) Methyl 1-isopropyl-3-indolecarboxylate:
   ¹H NMR (CDCl₃) δ: 1.56 (6H, d, J=6.9Hz), 3.92 (3H, s), 4.64-4.74 (1H, m), 7.24-7.31 (2H, m), 7.39-7.42 (2H, m), 7.96 (1H, s), 8.15-8.20 (1H, m).
(10) Ethyl 1,3-dimethyl-2-indolecarboxylate:
   ¹H NMR (CDCl₃) δ: 1.42-1.47 (3H, m), 2.59 (3H, s), 4.01 (3H, s), 4.37-4.45 (2H, m), 7.10-7.18 (1H, m), 7.31-7.38 (2H, m), 7.64-7.67 (1H, m).
(11) Ethyl 1-methyl-4-methylsulfonyl-2-indolecarboxylate:
   ¹H NMR (CDCl₃) δ: 1.41-1.46 (3H, m), 3.14 (3H, s), 4.16 (3H, s), 4.41 (2H, dd, J=7.3, 14.2Hz), 7.48 (1H, dd, J=7.3, 8.3Hz), 7.68-7.71 (2H, m), 7.81-7.84 (1H, m).
(12) Ethyl 1-methyl-6-methylsulfonyl-2-indolecarboxylate:
   ¹H NMR (CDCl₃) δ: 1.41-1.46 (3H, m), 3.11 (3H, s), 4.16 (3H, s), 4.37-4.45 (2H, m), 7.34 (1H, d, J=0.7Hz), 7.62-7.70 (2H, m), 7.83 (1H, dd, J=0.7, 8.6 Hz), 8.07 (1H, d, J=0.7Hz).
(13) Methyl 4-fluoro-1-methyl-2-indolecarboxylate:
   ¹H NMR (CDCl₃) δ: 3.92 (3H, s), 4.09 (3H, s), 6.77-6.83 (1H, m), 7.16 (1H, d, J=8.3Hz), 7.23-7.31 (1H, m), 7.36 (1H, s).
(14) Methyl 4-bromo-1-methyl-2-indolecarboxylate:
   ¹H NMR (CDCl₃) δ: 3.93 (3H, s), 4.08 (3H, s), 7.16-7.26 (1H, m), 7.31-7.35 (3H, m).
(15) Methyl 1-(2-naphthylmethyl)-2-indolecarboxylate:
   ¹H NMR (CDCl₃) δ: 3.86 (3H, s), 6.00 (2H, s), 7.14-7.32 (3H, m), 7.37-7.43 (5H, m), 7.66-7.78 (4H, m).
(16) Methyl 1-(2-phenylethyl)-2-indolecarboxylate:
   ¹H NMR (CDCl₃) δ: 3.06 (2H, t, J=7.9Hz), 3.90 (3H, s), 4.74-4.80 (2H, m), 7.11-7.33 (9H, m), 7.66-7.69 (1H, m).
(17) Methyl 1-(4-bromobenzyl)-2-indolecarboxylate:
   ¹H NMR (CDCl₃) δ: 3.87 (3H, s), 5.79 (2H, s), 6.92 (2H, dd, J=2.0, 6.6Hz), 7.15-7.23 (1H, m), 7.31-7.38 (5H, m), 7.70-7.74 (1H, m).
(18) Methyl 1-(4-nitrobenzyl)-2-indolecarboxylate:
   ¹H NMR (CDCl₃) δ: 3.87 (3H, s), 5.93 (2H, s), 7.14-7.41 (5H, m), 7.42 (1H, d, J=0.7Hz), 7.73-7.77 (1H, m), 8.09-8.14 (2H, m).
(19) Methyl 1-(3-phenylpropyl)-2-indolecarboxylate:
   ¹H NMR (CDCl₃) δ: 2.06-2.22 (2H, m), 2.69 (2H, d, J=8.0Hz), 3.90 (3H, s), 4.60 (2H, t, J=8.0Hz), 7.05-7.40 (9H, m), 7.66 (1H, d, J=8.0Hz).
(20) Methyl 1-(2-methoxyethyl)-2-indolecarboxylate:
   ¹H NMR (CDCl₃) δ: 3.28 (3H, s), 3.73 (2H, t, J=5.9Hz), 3.91 (3H, s), 4.74 (2H, t, J=5.9Hz), 7.14 (1H, ddd, J=1.0, 6.9, 7.4Hz), 7.31 (1H, d, J=0.7Hz), 7.36 (1H, dd, J=1.3, 6.9Hz), 7.48 (1H, dd, J=0.7, 8.6Hz), 7.66 (1H, dd, J=1.1, 8.3Hz).
(21) Methyl 1-(2-diethylaminoethyl)-2-indolecarboxylate:
   ¹H NMR (CDCl₃) δ: 1.03 (6H, t, J=7.3Hz), 2.61 (4H, q, J=7.3Hz), 2.70-2.82 (2H, m), 3.91 (3H, s), 4.58-4.70 (2H, m), 7.14 (1H, ddd, J=1.3, 6.7, 8.6Hz), 7.27 (1H, d, J=1.0Hz), 7.34 (1H, ddd, J=1.0, 6.7, 7.1Hz), 7.43 (1H, dd, J=1.0, 8.6Hz), 7.61-7.71 (1H, m).
(22) Ethyl 4-chloro-1-(2-diethylaminoethyl)-2-indolecarboxylate:
   ¹H NMR (CDCl₃) δ: 1.00 (6H, t, J=7.3Hz), 1.42 (3H, t, J=7.3Hz),2.59 (4H, q, J=7.3Hz), 2.69-2.80 (2H, m), 4.38 (2H, q, J=7.3Hz), 4.56-4.68 (2H, m), 7.14 (1H, dd, J=1.0, 7.3Hz), 7.18-7.28 (1H, m), 7.29-7.35 (1H, m), 7.37 (1H, d, J=0.7Hz).
(23) Preparation of methyl 1-[2-(2-tetrahydropyranyl)oxyethyl]-2-indolecarboxylate:
   The reaction was carried out in a manner similar to Reference Example 5 except for using 2.0 g (11.4 mmol) of methyl 2-indolecarboxylate, 0.55 g (13.7 mmol) of 60% sodium hydroxide, 3.63 g (13.7 mmol) of 2-(2-iodoethoxy)tetrahydropyran (prepared from 2-iodoethanol and 3,4-dihydro-2H-pyran) and 50 ml of dimethylformamide. Thus, 2.87 g (83.0%) of methyl 1-[2-(2-tetrahydropyranyl)oxyethyl]-2-indolecarboxylate was obtained.
   ¹H NMR (CDCl₃) δ: 1.27-1.75 (6H, m), 3.26-3.54 (2H, m), 3.75 (1H, dt, J=4.6, 10.2Hz), 4.03 (1H, dt, J=4.6, 10.2Hz), 4.47 (1H, t, J=3.0Hz), 4.80 (2H, t, J=3.7Hz), 7.13 (1H, t, J=7.0Hz), 7.22-7.38 (2H, m), 7.53 (1H, d, J=8.0Hz), 7.65 (1H, d, J=8.0Hz).
(24) Methyl 1-[3-(2-tetrahydropyranyl)oxypropyl]-2-indolecarboxylate:
   The title compound was prepared in a manner similar to Reference Example 5 (23) except that 2-(3-iodopropoxy)tetrahydropyran was used in place of 2-(2-iodoethoxy)tetrahydrophyran.
   ¹H NMR (CDCl₃) δ: 1.42-1.97 (6H, m), 2.11 (2H, dt, J=5.9, 11.2Hz), 3.33 (1H, dt, J=7.9, 8.3Hz), 3.40-3.55 (1H, m), 3.72-3.88 (2H, m), 3.94 (3H, s), 4.52 (1H, dd, J=3.0, 4.3Hz), 4.69 (2H, dt, J=0.9, 1.7Hz), 7.14 (1H, ddd, J=1.0, 7.0, 7.9Hz), 7.27-7.37 (2H, m), 7.48 (1H, dd, J=0.9, 8.5Hz), 7.66 (1H, dt, J=1.0, 7.9Hz).
(25) Synthesis of methyl 1-(3-tert-butoxycarbonylaminopropyl)-2-indolecarboxylate:
   The reaction was carried out in a manner similar to Reference Example 5 except for using 5.00 g (28.5 mmol) of methyl 2-indolecarboxylate, 1.26 g (31.4 mmol) of 60% sodium hydroxide, 12.3 g (43.2 mmol) of tert-butyl N-(3-iodopropyl)carbamate (prepared from 3-iodopropylamine and di-tert-butyl dicarbonate) and 60 ml of dimethylformamide. Thus, 2.54 g (27%) of methyl 1-(3-tert-butoxycarbonylaminopropyl)-2-indolecarboxylate was obtained.
   ¹H NMR (CDCl₃) δ: 1.45 (9H, s), 1.90-2.10 (2H, m), 3.00-3.20 (2H, m), 3.91 (3H, s), 4.62 (2H, t, J=6.9Hz), 4.98 (1H, br-s), 7.06-7.20 (1H, m), 7.28-7.44 (3H, m), 7.68 (1H, d, J=7.3Hz).
(26) Methyl 1-(2-tert-butoxycarbonylaminoethyl)-2-indolecarboxylate:
   The title compound in a manner similar to Reference Example 5 (25) except that tert-butyl N-(2-iodopropyl)carbamate was used in place of tert-butyl N-(3-iodopropyl)carbamate.
   ¹H NMR (CDCl₃) δ: 1.41 (9H, s), 3.53 (2H, t, J=5.9Hz), 3.90 (3H, s), 4.68 (2H, t, J=6.3Hz), 4.60-4.80 (1H, m), 7.15 (1H, ddd, 1H, J=1.0, 6.9, 7.4Hz), 7.27-7.38 (2H, m), 7.48 (1H, d, J=8.3Hz), 7.66 (1H, d, J=7.9Hz).
(27) Ethyl 1-methyl-4-(2-tetrahydropyranyl)oxymethyl-2-indolecarboxylate:
   The title compound was prepared in a manner similar to Reference Example 5.
   ¹H NMR (CDCl₃) δ: 1.39-1.44 (3H, m), 1.53-1.91 (6H, m), 3.50-3.61 (1H, m), 3.84-4.03 (1H, m), 4.09 (3H, s), 4.34-4.42 (2H, m), 4.75 (1H, t, J=3.6Hz), 4.83 (1H, d, J=12.2Hz), 5.08 (1H, d, J=12.2Hz), 7.18 (1H, t, J=4.0Hz), 7.32-7.33 (2H, m), 7.42 (1H, s).
(28) Ethyl 4-chloro-1-[4-(2-tetrahydropyranyl)oxybutyl]-2-indolecarboxylate:
   The title compound was prepared in a manner similar to Reference Example 5 (23) except taht 2-(4-iodobutoxy)tetrahydropyran and ethyl 4-chloro-2-indolecarboxylate was used in place of 2-(2-iodoethoxy)tetrahydropyran and methyl 2-indolecarboxylate.
   ¹H NMR (CDCl₃) δ: 1.42 (3H, t, J=7.3Hz), 1.41-2.00 (10H, m), 3.32-3.56 (2H, m), 3.68-3.90 (2H, m), 4.38 (2H, q, J=7.3Hz), 4.55 (1H, t, J=4.0Hz), 4.60 (2H, t, J=7.6Hz), 7.13 (1H, dd, J=1.0, 7.6Hz), 7.22 (1H, d, J=8.2Hz), 7.32 (1H, d, J=8.3Hz), 7.39 (1H, s).
(29) Methyl 1-(3:4-isopropylidenedioxybutyl)-2-indolecarboxylate:
   The title compound was prepared in a manner similar to Reference Example 5 except that 3,4-isopropylidenedioxybutyl iodide was used in place of methyl iodide.
   ¹H NMR (CDCl₃) δ: 1.34 (3H, s), 1.46 (3H, s), 1.90-2.18 (2H, m), 3.52 (1H, dd, J=6.9, 7.9Hz), 3.91 (3H, s), 3.97 (1H, dd, J=5.9, 7.9Hz), 4.01-4.17 (1H, m), 4.58-4.80 (2H, m), 7.15 (1H, ddd, J=1.0, 6.9, 7.4Hz), 7.31 (1H, d, J=0.7Hz), 7.35 (1H, ddd, J=1.3, 6.9, 7.6Hz), 7.47-7.55 (1H, m), 7.63-7.70 (1H, m).
(30) Methyl-1-[2-[1-(4-methyl-2,6,7-trioxabicyclo[2.2.2]octyl)]ethyl]-2-indolecarboxylate:
   The title compound was prepared in a manner similar to Reference Example 5 except that 2-[1-(4-methyl-2,6,7-trioxabicyclo[2.2.2]octyl]ethyl iodide was used in place of methyl iodide.
   ¹H NMR (CDCl₃) δ: 0.79 (3H, s), 2.17 (2H, ddd, J=2.6, 5.3, 7.9Hz), 3.89 (6H, s), 3.90 (3H, s), 5,87 (2H, ddd, J=2.3, 5.6, 7.9Hz), 7.12 (1H, ddd, J=1.0, 6.9, 7.4Hz), 7.27 (1H, d, J=0.7Hz), 7.32 (1H, ddd, J=1.3, 6.9, 7.6Hz), 7.48 (1H, dd, J=0.7, 8.6Hz), 7.65 (1H, ddd, J=1.0, 1.5, 8.3Hz).

   The following compounds were prepared in a manner similar to Reference Example 5.
(31) Methyl 4-benzyloxy-1-methyl-2-indolecarboxylate:
   ¹H NMR (CDCl₃) δ: 3.89 (3H, s), 4.06 (3H, s), 5.22 (2H, s), 6.57 (1H, d, J=7.6Hz), 6.99 (1H, d, J=8.6Hz), 7.22-7.28 (1H, m), 7.30-7.51 (6H, m).
(32) Methyl 6-benzyloxy-1-methyl-2-indolecarboxylate:
   ¹H NMR (CDCl₃) δ: 3.89 (3H, s), 4.02 (3H, s), 5.15 (2H, s), 6.85 (1H, d, J=2.31Hz), 6.91 (1H, dd, J=2.3, 8.6Hz), 7.24 (1H, d, J=1.0Hz), 7.34-7.44 (3H, m), 7.47-7.52 (2H, m), 7.53-7.57 (1H, m).
(33) Methyl 7-benzyloxy-1-methyl-2-indolecarboxylate:
   ¹H NMR (CDCl₃) δ: 3.89 (3H, s), 4.38 (3H, s), 5.19 (2H, s), 6.78 (1H, d, J=8.6Hz), 6.97-7.03 (1H, m), 7.24-7.27 (2H, m), 7.33-7.51 (5H, m).

### Reference Example 6

### Preparation of methyl 2-indolecarboxylate

To 300 ml of a methanol solution of 30.0 g (186.2 mmol) of 2-indolecarboxylic acid was dropwise added 44.3 g (372.3 mmol) of thionyl chloride at 0°C. The reaction mixture was refluxed for 2 hours and the solvent was then distilled off under reduced pressure. Ice water was poured onto the resulting residue. The mixture was made basic by the addition of concentrated ammonium hydroxide. The mixture was extracted three times with ethyl acetate. The extract was washed with water and dried over anhydrous magnesium sulfate. The solvent was then distilled off under reduced pressure to give 32.34 g (99.2%) of methyl 2-indolecarboxylate.
¹H NMR (CDCl₃) δ: 3.95 (3H, s), 7.13-7.45 (4H, m), 7.69 (1H, dd, J=1.0, 7.9Hz), 8.91 (1H, br-s).

The following compounds were prepared in a manner similar to Reference Example 6.
(1) Methyl 3-indolecarboxylate:
   ¹H NMR (CDCl₃) δ: 3.93 (3H, s), 7.24-7.31 (2H, m), 7.38-7.45 (1H, m), 7.93 (1H, d, J=3.0Hz), 8.17-8.22 (1H, m), 8.63 (1H, br-s).
(2) Methyl 4-fluoro-2-indolecarboxylate:
   ¹H NMR (CDCl₃) δ: 3.96 (3H, s), 6.78-6.85 (1H, m), 7.18-7.30 (3H, m), 8.99 (1H, br-s).
(3) Methyl 4-bromo-2-indolecarboxylate:
   ¹H NMR (CDCl₃) δ: 3.97 (3H, s), 7.17 (1H, dd, J=7.6, 8.3Hz), 7.28 (1H, dd, J=1.0, 2.3Hz), 7.32-7.39 (2H, m), 9.05 (1H, br-s).
(4) Methyl 7-benzyloxy-2-indolecarboxylate:
   ¹H NMR (CDCl₃) δ: 3.93 (3H, s), 5.21 (2H, s), 6.80 (1H, d, J=6.9Hz), 7.01-7.08 (1H, m), 7.19 (1H, dd, J=2.3, 4.3Hz), 7.24-7.31 (1H, m), 7.35-7.51 (5H, m), 9.07 (1H, br-s).
(5) Methyl 4-benzyloxy-2-indolecarboxylate:
   ¹H NMR (CDCl₃) δ: 3.93 (3H, s), 5.22 (2H, s), 6.58 (1H, d, J=7.6Hz), 7.03 (1H, d, J=8.3Hz), 7.19-7.26 (1H, m), 7.31-7.44 (4H, m), 7.50 (2H, d, J=7.3Hz), 8.84 (1H, br-s).

### Reference Example 7

### Preparation of methyl 5-indolecarboxylate

A mixture of 1.00 g (6.21 mmol) of 5-indolecarboxylic acid and 50 ml of 10% hydrogen chloride/methanol was refluxed for 2 hours. The reaction mixture was then poured onto ice water followed by neutralization with sodium bicarbonate. The mixture was extracted three times with ethyl acetate. The combined extracts were washed with aqueous sodium bicarbonate solution. After drying over anhydrous magnesium sulfate, the solvent was distilled off under reduced pressure to give 0.42 g (38.6%) of methyl 5-indole-carboxylate.
¹H NMR (CDCl₃) δ: 3.93 (3H, s), 6.64-6.66 (1H, m), 7.26-7.29 (1H, m), 7.40 (1H, dd, J=0.7, 8.6Hz), 7.91 (1H, dd, J=1.7, 8.6Hz), 8.3-8.6 (2H, m).

### Reference Example 8

### Preparation of methyl 1-isopropyl-5-indolecarboxylate

A mixture of 2.20 g (8.97 mmol) of isopropyl 1-isopropyl-5-indolecarboxylate, 100 ml of 2N sodium hydroxide solution and 100 ml of ethanol was refluxed for an hour. The solvent was then distilled off under reduced pressure. Thereafter water was added to the residue and the resulting mixture was acidified with conc. hydrochloric acid. The precipitated solid was filtered and dried under reduced pressure to give 2.00 g of crude 1-isopropyl-5-indole-carboxylic acid. The reaction was carried out in a manner similar to Reference Example 4 using 2.00 g of the crude 1-isopropyl-5-indolecarboxylic acid, 0.44 g (11.1 mmol) of 60% sodium hydride, 2.87 g (20.2 mmol) of methyl iodide and 50 ml of dimethylformamide. Thus, 1.64 g (84.2%; yield based on isopropyl 1-isopropyl-5-indolecarboxylic acid) was obtained.
¹H NMR (CDCl₃) δ: 1.54 (6H, d, J=6.9Hz), 3.93 (3H, s), 4.65-4.75 (1H, m), 6.61 (1H, d, J=3.3Hz), 7.28 (1H, d, J=3.3Hz), 7.37 (1H, d, J=8.6Hz), 7.9 (1H, dd, J=1.7, 8.6Hz), 8.39 (1H, d, J=1.7Hz).

### Reference Example 9

### Preparation of 7-chloro-2-indolecarboxylic acid

A mixture of 3.40 g (15.2 mmol) of ethyl 7-chloro-2-indolecarboxylate, 100 ml of 2N sodium hydroxide solution and 100 ml of ethanol was refluxed for an hour. The solvent was then distilled off under reduced pressure. Thereafter ice water was added to the residue and the resulting mixture was acidified with conc. hydrochloric acid and extracted three times with ethyl acetate. After drying over anhydrous magnesium sulfate, the solvent was distilled off under reduced pressure to give 2.85 g (95.8%) of 7-chloro-2-indolecarboxylic acid.
¹H NMR (DMSO-d₆) δ: 7.04-7.09 (1H, m), 7.19 (1H, d, J=2.0Hz), 7.30 (1H, dd, J=1.0, 7.6Hz), 7.62 (1H, d, J=8.3Hz), 11.9 (1H, br-s), 13.1 (1H, br-s).

### Reference Example 10

### Preparation of 1-isopropyl-2-indolecarboxylic acid

The reaction was carried out in a manner similar to Reference Example 4, using 6.00 g (34.2 mmol) of methyl 2-indolecarboxylate, 1.36 g (34.2 mmol) of 60% sodium hydroxide, 6.40 g (37.7 mmol) of isopropyl iodide and 100 ml of dimethylformamide. The mixture of methyl 1-isopropyl-2-indolecarboxylate and isopropyl 1-isopropyl-2-indolecarboxylate was obtained. The reaction was carried out in a manner similar to Reference Example 9, using the thus obtained mixture, 150 ml of 2N sodium hydroxide solution and 150 ml of ethanol. Thus 3.71 g (53.3%) of 1-isopropyl-2-indolecarboxylic acid was obtained.
¹H NMR (DMSO-d₆) δ: 1.58 (6H, d, J=6.9Hz), 5.74-5.85 (1H, m), 7.05-7.11 (1H, m), 7.19-7.28 (2H, m), 7.64-7.72 (2H, m), 12.9 (1H, br-s).

### Reference Example 11

### Preparation of methyl 1-methyl-7-indolecarboxylate

### a) Preparation of ethyl 7-carbomethoxy-1-methyl-2-indolecarboxylate

After 5.00 g (20.2 mmol) of ethyl 7-carbomethoxy-2-indolecarboxylate obtained in a manner similar to Reference Example 1 was added to a suspension of 0.81 g (20.2 mmol) of 60% sodium hydride in 80 ml of dimethylformamide, the mixture was stirred at room temperature until the mixture became a transparent solution. A solution of 5.74 g (40.4 mmol) of methyl iodide was then added dropwise to the transparent solution at room temperature followed by stirring at 50°C for an hour. The reaction solution was poured onto ice water. The resulting mixture was then extracted three times with ethyl acetate and the combined extracts were washed with water. After drying over anhydrous magnesium sulfate, the solvent was distilled off under reduced pressure and the residue was isolated and purified by silica gel column chromatography to give 5.20 g (98.5%) of ethyl 7-carbomethoxy-1-methyl-2-indolecarboxylate.

### b) Preparation of 1-methylindole-2,7-dicarboxylic acid

A mixture of 5.20 g (19.9 mmol) of ethyl 7-carbomethoxy-1-methyl-2-indolecarboxylate, 90 ml of 2N sodium hydroxide and 150 ml of ethanol was refluxed for 3 hours. The reaction mixture was concentrated under reduced pressure and ice water was added to the residue. 2N hydrochloric acid was added to acidify the reaction mixture. The precipitated solid was filtered and dried under reduced pressure to give 4.70 g (>99%) of 1-methylindole-2,7-dicarboxylic acid.

### c) Preparation of 1-methyl-7-indolecarboxylic acid

A mixture of 4.60 g (21.0 mmol) of 1-methylindole-2,7-dicarboxylic acid, 0.5 g of copper (II) oxide and 50 ml of quinoline was stirred for an hour with heating at 180°C. After cooling, the reaction mixture was poured onto 200 ml of 2N hydrochloric acid. The mixture was extracted three times with ethyl acetate and the combined extracts were washed with saturated sodium chloride solution. After drying over anhydrous magnesium sulfate, the solvent was distilled off under reduced pressure. The residue was isolated and purified by silica gel column chromatography to give 1.82 g (49.0%) of 1-methyl-7-indolecarboxylic acid.

### d) Preparation of methyl 1-methyl-7-indolecarboxylate

To 70 ml of a methanol solution of 1.82 g (10.4 mmol) of 1-methyl-7-indolecarboxylic acid was added dropwise 3.09 g (26.0 mmol) of thionyl chloride at 0°C. The reaction mixture was refluxed for 2 hours and the solvent was then distilled off under reduced pressure. Ice water was poured onto the resulting residue and ammonium hydroxide was added to render the mixture alkaline. The mixture was extracted three times with ethyl acetate. The combined extracts were washed with water and dried over anhydrous magnesium sulfate. The solvent was then distilled off under reduced pressure. The residue was isolated and purified by silica gel column chromatography to give 1.16 g (59.0%) of methyl 1-methyl-7-indolecarboxylate.
¹H NMR (CDCl₃) δ: 3.88 (3H, s), 3.96 (3H, s), 6.54 (1H, d, J=3.3Hz), 7.10 (1H, t, J=7.6Hz), 7.67 (1H, d, J=7.3Hz), 7.75-7.78 (1H, m).

### Reference Example 12

### Preparation of ethyl 7-benzyloxy-4-chloro-2-indolecarboxylate

### a) Preparation of 3-benzyloxy-6-chloro-2-nitrotoluene

A mixture of 1.50 g (8.00 mmols) of 4-chloro-3-methyl-2-nitrophenol, 1.50 g (8.80 mmols) of benzyl bromide, 2.43 g (17.6 mmols) of potassium carbonate and 70 ml of acetone was refluxed for 2 hours. Thereafter insoluble matters were filtered off and the filtrate was concentrated under reduced pressure. The residue was isolated and purified by silica gel column chromatography to give 2.22 g (>99%) of 3-benzyloxy-6-chloro-2-nitrotoluene.

### b) Preparation of ethyl (3-benzyloxy-6-chloro-2-nitrophenyl)pyruvate

Diethyl oxalate, 1.20 g (7.92 mmol), was added dropwise to a suspension of 0.67 g (7.92 mmol) of potassium ethoxide in diethyl ether (50 ml) at room temperature. Subsequently 2.00 g (7.20 mmol) of 3-benzyloxy-6-chloro-2-nitrotoluene was added to the mixture followed by stirring for 4 hours at room temperature. The reaction solution was poured onto 1N hydrochloric acid and the mixture was extracted twice with diethyl ether. The combined extracts were washed with saturated sodium chloride solution. After drying over anhydrous magnesium sulfate, the solvent was distilled off under reduced pressure. The residue was isolated and purified by silica gel column chromatography to give 1.60 g (53.5%) of ethyl (3-benzyloxy-6-chloro-2-nitrophenyl)pyruvate.

### c) Preparation of ethyl 7-benzyloxy-4-chloro-2-indolecarboxylate

A mixture of 1.60 g (4.24 mmol) of ethyl (3-benzyloxy-4-chloro-2-nitrophenyl)pyruvate, 22.9 g (29.7 mmol) of 20% titanium trichloride solution and 60 ml of acetone was stirred at room temperature for 3 hours. The reaction mixture was poured onto ice water and the mixture was extracted three times with ethyl acetate. The combined extracts were washed with saturated sodium hydrogencarbonate solution. After drying over anhydrous magnesium sulfate, the solvent was distilled off under reduced pressure. The residue was purified by silica gel column chromatography to give 0.50 g (35.8%) of ethyl 7-benzyloxy-4-chloro-2-indole-carboxylate.
¹H NMR (CDCl₃) δ: 1.39-1.44 (3H, m), 4.40 (2H, dd, J=6.9, 14.2Hz), 5.18 (2H, s), 6.69 (1H, d, J=8.3Hz), 7.01 (1H, d, J=8.3Hz), 7.26-7.27 (1H, m), 7.35-7.48 (5H, m), 9.15 (1H, br-s).

### Reference Example 13

### Preparation of ethyl 6-benzyloxy-4-chloro-2-indolecarboxylate

### a) Preparation of ethyl 3-(4-benzyloxy-2-chlorophenyl)-2-azidopropenoate

An ethanol solution, 70 ml, containing 5.40 g (21.9 mmol) of 4-benzyloxy-2-chlorobenzaldehyde and 11.3 g (87.6 mmol) of ethylazide acetate was gradually added dropwise to 70 ml of an ethanol solution of 5.95 g (87.6 mmol) of sodium ethoxide at -10°C. After stirring at -10°C for further 5 hours, the reaction temperature was slowly elevated to room temperature. The reaction mixture was poured onto 200 ml of saturated ammonium chloride aqueous solution and the mixture was extracted three times with ethyl acetate. The combined extracts were then washed with saturated ammonium chloride solution and next with saturated sodium chloride solution. After drying over anhydrous magnesium sulfate, the solvent was distilled off under reduced pressure. The resulting residue was purified by silica gel column chromatography to give 4.50 g (57.5%) of ethyl 3-(4-benzyloxy-2-chlorophenyl)-2-azidopropenoate.

### b) Preparation of ethyl 6-benzyloxy-4-chloro-2-indolecarboxylate

A solution of 4.50 g (12.6 mmols) of ethyl 3-(4-benzyloxy-2-chlorophenyl)-2-azidopropenoate in 100 ml of toluene was refluxed for 3 hours. The reaction mixture was concentrated under reduced pressure. The resulting residue was purified by silica gel column chromatography to give 3.73 g (89.8%) of ethyl 6-benzyloxy-4-chloro-2-indolecarboxylate.
¹H NMR (CDCl₃) δ: 1.38-1.43 (3H, m), 4.35-4.43 (2H, m), 5.09 (2H, s), 6.79 (1H, dd, J=0.7, 2.0Hz), 6.95 (1H, d, J=2.0Hz), 7.23-7.24 (1H, m), 7.31-7.45 (5H, m), 8.94 (1H, br-s).

### Reference Example 14

### Preparation of methyl 1-(2-carbamoylethyl)-2-indolecarboxylate

### a) Preparation of methyl 1-(2-cyanoethyl)-2-indolecarboxylate

After 3.63 g (68.4 mmol) of acrylonitrile and 2.2 ml of 40% methanol solution of N-benzyltrimethylammonium hydroxide was added to a solution of 10.0 g (57.1 mmol) of methyl 2-indolecarboxylate in 150 ml of 1,4-dioxane, the mixture was stirred at 55°C for an hour. The reaction mixture was concentrated under reduced pressure. The resulting residue was added to a mixture of 5 ml of acetic acid and 500 ml of water. The aqueous layer was extracted twice with methylene chloride and the combined extracts were washed with water. After drying over anhydrous magnesium sulfate, the solvent was distilled off under reduced pressure. The residue was purified by silica gel column chromatography to give 13.0 g of methyl 1-(2-cyano-ethyl)-2-indolecarboxylate.

### b) Preparation of methyl 1-(2-carbamoylethyl)-2-indolecarboxylate

A mixture of 3.12 g (13.7 mmol) of methyl 1-(2-cyanoethyl)-2-indolecarboxylate, 30 ml of 10% sodium carbonate solution, 30 ml of 30% hydrogen peroxide and 100 ml of acetone was stirred at room temperature for 4 hours. Next, the reaction mixture was cooled to 0°C and 10% sodium sulfite solution was added dropwise to decompose an excess of the peroxide. The most of acetone in the reaction mixture was then distilled off and the concentrate was extracted three times with ethyl acetate. The combined extracts were washed with saturated sodium chloride solution. After drying over anhydrous magnesium sulfate, the solvent was distilled off under reduced pressure. The resulting residue was purified by silica gel column chromatography to give 2.30 g (68%) of methyl 1-(2-carbamoylethyl)-2-indolecarboxylate.
¹H NMR (CDCl₃) δ: 2.75 (2H, ddd, J=1.7, 5.9, 7.6Hz), 3.92 (3H, s), 4.85 (2H, ddd, J=1.7, 5.9, 7.6Hz), 5.37 (1H, br-s), 5.72 (1H, br-s), 7.16 (1H, ddd, J=1.0, 6.9, 7.4Hz), 7.32 (1H, d, J=1.0Hz), 7.37 (1H, ddd, J=1.0, 7.3, 7.8Hz), 7.53 (1H, dd, J=0.8, 8.4Hz), 7.67 (1H, dt, J=1.0, 7.9Hz).

The following compound was prepared in a manner similar to Reference Example 14.
(1) Ethyl 1-(2-carbamoylethyl)-4-chloro-2-indolecarboxylate:
   ¹H NMR (CDCl₃) δ: 1.43 (3H, t, J=7.3Hz), 2.65-2.82 (1H, m), 4.39 (2H, q, J=7.3Hz), 4.84 (2H, ddd, J=1.0, 6.3, 7.3Hz), 5.45 (1H, br-s), 5.68 (1H, br-s), 7.14 (1H, d, J=7.9Hz), 7.26 (1H, dd, J=7.6, 8.2Hz), 7.41 (1H, d, J=1.0Hz), 7.45 (1H, d, J=8.6Hz).

### Reference Example 15

### Preparation of methyl 7-carbamoylmethoxy-1-methyl-2-indolecarboxylate

### a) Preparation of methyl 7-hydroxy-1-methyl-2-indolecarboxylate

In a solvent mixture of 50 ml of tetrahydrofuran and 50 ml of methanol was dissolved 2.31 g (7.82 mmol) of methyl 7-benzyloxy-1-methyl-2-indolecarboxylate. After 0.5 g of 10% palladium/carbon was added to the solution, catalytic hydrogenation was performed at ambient temperature under normal pressure. After completion of the reaction, the catalyst was filtered off and the filtrate was concentrated under reduced pressure. The resulting residue was purified by silica gel column chromatography to give 1.63 g (>99%) of methyl 7-hydroxy-1-methyl-2-indolecarboxylate.

### b) Preparation of methyl 7-carbamoylmethoxy-1-methyl-2-indolecarboxylate

After 0.50 g (2.44 mmol) of methyl 7-hydroxy-1-methyl-2-indolecarboxylate was added to a suspension of 0.01 g (2.44 mmol) of 60% sodium hydride in 25 ml of dimethyl-formamide, the mixture was stirred at room temperature until the mixture became a transparent solution. Then 0.25 g (2.68 mmols) of 2-chloroacetamide was added dropwise to the transparent solution at room temperature followed by stirring at 50°C for an hour. The reaction mixture was poured onto ice water. The resulting mixture was then extracted three times with ethyl acetate. The combined extracts were washed with water. After drying over anhydrous magnesium sulfate, the solvent was distilled off under reduced pressure. The residue was purified by silica gel column chromatography to give 0.54 g (84.4%) of methyl 7-carbamoylmethoxy-1-methyl-2-indolecarboxylate.
¹H NMR (CDCl₃) δ: 3.91 (3H, s), 4.41 (3H, s), 4.67 (2H, s), 5.70 (1H, br-s), 6.41 (1H, br-s), 6.70-6.73 (1H, m), 7.03 (1H, t, J=7.9Hz), 7.26 (1H, s), 7.31-7.34 (1H, m).

The following compounds were synthesized in a manner similar to Reference Example 15.
(1) Methyl 1-methyl-7-(2-phenylethoxy)-2-indolecarboxylate:
   ¹H NMR (CDCl₃) δ: 3.17-3.22 (2H, m), 3.87 (3H, s), 4.24 (3H, s), 4.31-4.36 (2H, m), 6.68 (1H, d, J=7.6Hz), 6.94-7.00 (1H, m), 7.18-7.35 (7H, m).
(2) Methyl 1-methyl-7-(3-phenylpropoxy)-2-indolecarboxylate:
   ¹H NMR (CDCl₃) δ: 2.16-2.26 (2H, m), 2.84-2.90 (2H, m), 3.89 (3H, s), 4.07-4.12 (2H, m), 4.43 (3H, s), 6.64 (1H, d, J=6.9Hz), 6.97 (1H, t, J=7.9Hz), 7.18-7.23 (5H, m), 7.25-7,33 (2H, m).
(3) Ethyl 7-carbamoylmethoxy-4-chloro-1-methyl-2-indolecarboxylate:
   ¹H NMR (CDCl₃) δ: 1.33-1.36 (3H, m), 4.29-4,37 (5H, m), 4.60 (2H, s), 6.69 (1H, d, J=8.3Hz), 7.06 (1H, dd, J=0.7, 8.2Hz), 7.15 (1H, d, J=0.7Hz), 7.38 (1H, br-s), 7.54 (1H, br-s).
(4) Ethyl 4-chloro-7-(2-dimethylaminoethoxy)-1-methyl-2-indolecarboxylate:
   ¹H NMR (CDCl₃) δ: 1.39-1.44 (3H, m), 2.36 (6H, s), 2.82 (2H, t, J=5.9Hz), 4.17 (2H, t, J=5.9Hz), 4.33-4.40 (5H, m), 6.59 (1H, d, J=8.3Hz), 6.96 (1H, d, J=7.9Hz), 7.30 (1H, s).
(5) Ethyl 6-carbamoylmethoxy-1-methyl-2-indolecarboxylate:
   ¹H NMR (CDCl₃) δ: 1.38-1.43 (3H, m), 4.03 (3H, s), 4.32-4.40 (2H, m), 4.60 (2H, s), 5.61 (1H, br-s), 6.59 (1H, br-s), 6.79 (1H, d, J=2.3Hz), 6.84 (1H, dd, J=2.3, 8.6Hz), 7.26-7.27 (1H, m), 7.57-7.60 (1H, m).
(6) Ethyl 4-chloro-1-methyl-7-[2-(N-pyrrolidinyl)-ethoxy]-2-indolecarboxylate:
   ¹H NMR (CDCl₃) δ: 1.39-1.44 (3H, m), 1.79-1.84 (4H, m), 2.63-2.68 (4H, m), 2.97-3.02 (2H, m), 4.20-4.24 (2H, m), 4.33-4.41 (5H, m), 6.60 (1H, d, J=8.6Hz), 6.97 (1H, d, J=8.3 Hz), 7.31 (1H, s).
(7) Methyl 7-(3-tert-butoxycarbonylaminopropoxy)-1-methyl-2-indolecarboxylate:
   ¹H NMR (CDCl₃) δ: 1.43 (9H, s), 2.09 (2H, t, J=6.3Hz), 3.35-3.42 (2H, m), 3.89 (3H, s), 4.13-4.18 (2H, m), 4.39 (3H, s), 4.73 (1H, br-s), 6.69 (1H, d, J=7.9Hz), 6.96-7.02 (1H, m), 7.21-7.26 (2H, m).
(8) Ethyl 7-(3-tert-butoxycarbonylaminopropoxy)-4-chloro-1-methyl-2-indolecarboxylate:
   ¹H NMR (CDCl₃) δ: 1.38-1.44 (12H, m), 2.03-2.13 (2H, m), 3.33-3.40 (2H, m), 4.12 (2H, t, J=5.9Hz), 4.33-4.41 (5H, m), 4.70 (1H, br-s), 6.58 (1H, d, J=8.3Hz), 6.96 (1H, d, J=8.3Hz), 7.31 (1H, s).
(9) Ethyl 6-(3-tert-butoxycarbonylaminopropoxy)-1-methyl-2-indolecarboxylate:
   ¹H NMR (CDCl₃) δ: 1.37-1.42 (3H, m), 1.45 (9H, s), 2.02 (2H, dd, J=6.3, 12.5Hz), 3.33-3.40 (2H, m), 4.02 (3H, s), 4.10 (2H, t, J=5.9Hz), 4.35 (2H, dd, J=6.9, 14.2Hz), 4.78 (1H, br-s), 6.76 (1H, s), 6.78-6.83 (1H, m), 7.24-7.26 (1H, m), 7.53 (1H, d, J=8.6Hz).
(10) Ethyl 7-(2-tert-butoxycarbonylaminoethoxy)-4-chloro-1-methyl-2-indolecarboxylate:
   ¹H NMR (CDCl₃) δ: 1.39-1.44 (3H, m), 1.45 (9H, s), 3.63 (2H, dd, J=5.3, 10.6Hz), 4.13 (2H, t, J=5.3Hz), 4.30-4.41 (5H, m), 4.63-4.89 (1H, m), 6.58 (1H, d, J=8.3Hz), 6.97 (1H, d, J=8.3Hz), 7.31 (1H, s).
(11) Methyl 1-methyl-7-[2-(2-tetrahydropyranyl)oxyethoxy]-2-indolecarboxylate:
   ¹H NMR (CDCl₃) δ: 1.52-1.85 (6H, m), 3.50-3.58 (1H, m), 3.83-3.90 (5H, m), 4.11-4.19 (1H, m), 4.26-4.30 (2H, m), 4.42 (3H, s), 4.73-4.75 (1H, m), 6.70-6.73 (1H, m), 7.01 (1H, t, J=7.9Hz), 7.22-7.26 (2H, m).
(12) Ethyl 4-chloro-1-methyl-7-[2-(2-tetrahydropyranyl)oxyethoxy]-2-indolecarboxylate:
   ¹H NMR (CDCl₃) δ: 1.42 (3H, t, J=7.3Hz), 1.52-1.85 (6H, m), 3.51-3.56 (1H, m), 3.81-3.91 (2H, m), 4.10-4.17 (1H, m), 4.23-4.27 (2H, m), 4.33-4.40 (5H, m), 4.72-4.73 (1H, m), 6.61 (1H, d, J=8.2Hz), 6.96 (1H, d, J=8.3Hz), 7.30 (1H, s).
(13) Ethyl 4-chloro-7-(2:3-isopropylidenedioxypropoxy)-1-methyl-2-indolecarboxylate:
   ¹H NMR (CDCl₃) δ: 1.39-1.47 (9H, m), 3.91-3.99 (1H, m), 4.06-4.23 (3H, m), 4.33-4.41 (5H, m), 4.51-4.63 (1H, m), 6.60 (1H, d, J=8.3Hz), 6,97 (1H, d, J=8.3Hz), 7.31 (1H, s).
(14) Ethyl 4-chloro-1-methyl-7-[4-(2-tetrahydropyranyl)oxybutoxy]-2-indolecarboxylate:
   ¹H NMR (CDCl₃) δ: 1.39-1.44 (3H, m), 1.52-1.85 (8H, m), 1.87-2.04 (2H, m), 3.44-3.55 (2H, m), 3.79-3.91 (2H, m), 4.10 (2H, t, J=6.3Hz), 4.33-4.41 (5H, m), 4.58-4.61 (1H, m), 6,56 (1H, d, J=8.3Hz), 6.96 (1H, d, J=8.3Hz), 7.30 (1H, s).

### Reference Example 16

### Preparation of ethyl 4-carboxy-1-methyl-2-indolecarboxylate

### a) Preparation of ethyl 4-hydroxymethyl-1-methyl-2-indolecarboxylate

In a solvent mixture of 20 ml of 2N hydrochloric acid and 60 ml of tetrahydrofran was dissolved 4.00 g (12.6 mmol) of ethyl 1-methyl-4-(2-tetrahydropyranyl)-oxymethyl-2-indolecarboxylate. The solution was stirred at 50°C for an hour. The reaction mixture was poured onto ice water and the aqueous layer was extracted three times with ethyl acetate. The combined extracts were washed with saturated sodium hydrogencarbonate solution. After drying over anhydrous magnesium sulfate, the solvent was distilled off under reduced pressure. The residue was purified by silica gel column chromatography to give 2.90 g (99%) of ethyl 4-hydroxymethyl-1-methyl-2-indolecarboxylate.

### b) Preparation of ethyl 4-carboxy-1-methyl-2-indolecarboxylate

In 30 ml of acetone was dissolved 0.70 g (3.00 mmols) of ethyl 4-hydroxymethyl-1-methyl-2-indolecarboxylate. After 3.3 ml of Jones' reagent, which was prepared by dissolving 26.7 g of chromium (VI) oxide in a mixture of 23 ml of conc. sulfuric acid and 40 ml of water and adding water to make the whole volume 100 ml, was added dropwise to the above solution at room temperature, the mixture was stirred at room temperature for an hour. The reaction mixture was poured onto ice water and the aqueous layer was extracted three times with chloroform. The combined extracts were washed with saturated sodium hydrogencarbonate solution. After drying over anhydrous magnesium sulfate, the solvent was distilled off under reduced pressure. The residue was purified by silica gel column chromatography to give 0.38 g (51.2%) of ethyl 4-carboxy-1-methyl-2-indolecarboxylate.
¹H NMR (DMSO-d₆) δ: 1.34-1.40 (3H, m), 4.08 (3H, s), 4.35 (2H, dd, J=7.3, 14.2Hz), 7.41-7.47 (1H, m), 7.72 (1H, s), 7.82-7.89 (2H, m), 12.7 (0.5H, br-s).

### Reference Example 17

### Preparation of ethyl 6-benzyloxy-4-methyl-2-indolecarboxylate

### a) Preparation of 4-benzyloxy-2-methylbenzoic acid

A mixture of 5.00 g (18.9 mmol) of 5-benzyloxy-2-bromotoluene, 0.46 g (18.9 mmol) of metallic magnesium, a catalytic amount of iodine and 20 ml of tetrahydrofuran was refluxed for 2 hours. After cooling to -50°C, carbon dioxide was bubbled into the reaction solution for 30 minutes. The reaction temperature was then elevated to room temperature and stirring was continued at the same temperature for further 2 hours. Next the reaction mixture was poured into 1N hydrochloric acid followed by extraction twice with ethyl acetate. The combined extracts were washed with water. After drying over anhydrous magnesium sulfate, the solvent was distilled off under reduced pressure. The resulting residue was purified by silica gel column chromatography to give 1.40 g of 4-benzyloxy-2-methylbenzoic acid.

### b) Preparation of methyl 4-benzyolxy-2-methylbenzoate

Using 1.40 g (5.78 mmol) of 4-benzyloxy-2-methylbenzoic acid, 1.37 g (11.6 mmol) of thionyl chloride and 50 ml of methanol, the reaction was carried out in a manner similar to Reference Example 6 to obtain 0.77 g of methyl 4-benzyloxy-2-methylbenzoate.

### c) Preparation of 4-benzyloxy-2-methylbenzyl alcohol

A suspension of 0.11 g (2.93 mmol) of lithium aluminum hydride in 20 ml of tetrahydrofuran was cooled to 0°C. A solution of 0.75 g (2.93 mmol) of methyl 4-benzyloxy-2-methylbenzoate in 20 ml of tetrahydrofuran was added dropwise to the suspension at 0°C. After stirring at 0°C for 2 hours, the reaction mixture was treated in a conventional manner to give 0.66 g of 4-benzyloxy-2-methylbenzyl alcohol.

### d) Preparation of 4-benzyloxy-2-methylbenzaldehyde

A mixture of 0.70 g (3.07 mmol) of 4-benzyolxy-2-methylbenzyl alcohol, 2.67 g (30.7 mmol) of manganese dioxide, 0.5 ml of methanol and 20 ml of chloroform was stirred at room temperature for 11 hours. Then insoluble matters were filtered off and the filtrate was concentrated under reduced pressure. The resulting residue was purified by silica gel column chromatography to give 0.60 g of 4-benzyloxy-2-methylbenzaldehyde.

### e) Preparation of ethyl 3-(4-benzyloxy-2-methylphenyl)-2-azidopropenoate

The reaction was carried out in a manner similar to Reference Example 13 a) except for using 2.80 g (12.4 mmol) of 4-benzyloxy-2-methylbenzaldehyde, 6.39 g (49.5 mmol) of ethyl azidacetate, 3.37 g (49.5 mmol) of sodium ethoxide and 50 ml of ethanol. Ethyl 3-(4-benzyloxy-2-methylphenyl)-2-azidopropenoate was thus obtained in the yield of 3.24 g.

### f) Preparation of ethyl 6-benzyloxy-4-methyl-2-indolecarboxylate

The reaction was carried out in a manner similar to Reference Example 13 b) except for using 3.22 g of ethyl 3-(4-benzyloxy-2-methylphenyl)-2-azido-propenoate and 100 ml of toluene. Ethyl 6-benzyloxy-4-methyl-2-indolecarboxylate was thus obtained in the yield of 2.66 g.
¹H NMR (CDCl₃) δ: 1.38-1.43 (3H, m), 2.51 (3H, s), 4.38 (2H, dd, J=6.9, 14.2Hz), 5.09 (2H, s), 6.72 (2H, s), 7.19 (1H, d, J=2.3Hz), 7.29-7.46 (5H, m), 8.71 (1H, br-s)

The following compound was prepared in a manner similar to Reference Example 17.
(1) Ethyl 6-benzyloxy-4-trifluoromethyl-2-indolecarboxylate:
   ¹H NMR (CDCl₃) δ: 1.39-1.44 (3H, 4.37-4.45 (2H, m), 5.14 (2H, s), 7.05 (1H, s), 7.23-7.24 (1H, m), 7.30 (1H, s), 7.35-7.47 (5H, m), 8.92 (1H, br-s)

### Example 1

### Preparation of 1-methyl-2-indoloylguanidine

After 8.58 g (89.8 mmol) of guanidine hydrochloride was added to 70 ml of a methanol solution of 4.85 g (89.8 mmol) of sodium methoxide, the mixture was stirred at room temperature. The precipitated sodium chloride was filtered off to obtain the solution. Then 1.70 g (8.97 mmol) of methyl 1-methyl-2-indolecarboxylate was added to the thus obtained solution. Subsequently methanol was distilled off under reduced pressure. The resulting residue was heated at 130°C for 5 minutes and then allowed to stand at room temperature for an hour. Thereafter water was poured onto the reaction solution and the mixture was extracted three times with ethyl acetate. The combined extracts were washed with water. After drying over anhydrous magnesium sulfate, the solvent was distilled off under reduced pressure. The resulting residue was isolated and purified by silica gel column chromatography to give the desired 1-methyl-2-indoloylguanidine. The compound was dissolved in chloroform and treated with hydrogen chloride/ether. Thus 0.70 g (30.8%) of 1-methyl-2-indoloylguanidine hydrochloride was obtained.
M.P.: 250°C or higher
¹H NMR (DMSO-d₆) δ: 4.04 (3H, s), 7.12-7.21 (1H, m), 7.31-7.44 (1H, m), 7.61 (1H, d, J=8.6Hz), 7.73 (1H, d, J=7.9Hz), 7.89 (1H, s), 8.5 (2H, br-s), 8.7 (2H, br-s), 11.9 (1H, br-s).

### Example 2

### Preparation of 1-methyl-5-indoloylguanidine

The reaction was carried out in a manner similar to Example 1 except for using 1.00 g (5.29 mmol) of methyl 1-methyl-5-indolecarboxylate, 5.05 g (52.9 mmol) of guanidine hydrochloride and 50 ml of a methanol solution of 2.85 g (52.9 mmol) of sodium methoxide. Thus 0.92 g (68.9%) of 1-methyl-5-indoloylguanidine hydrochloride was obtained.
M.P.: 260°C or higher
¹H NMR (DMSO-d₆) δ: 3.86 (3H, s), 6.62-6.64 (1H, m), 7.50 (1H, d, J=3.3Hz), 7.61 (1H, d, J=8.9Hz), 7.91-7.95 (1H, m), 8.44 (2H, br-s), 8.47 (1H, d, J=1.3Hz), 8.7 (2H, br-s), 11.7 (1H, br-s).

### Example 3

### Preparation of 1-methyl-3-indoloylguanidine

The reaction was carried out in a manner similar to Example 1 except for using 1.00 g (5.29 mmol) of methyl 1-methyl-3-indolecarboxylate, 5.05 g (52.9 mmol) of guanidine hydrochloride and 50 ml of a methanol solution of 2.85 g (52.9 mmol) of sodium methoxide. Thus 0.48 g (35.9%) of 1-methyl-3-indoloylguanidine hydrochloride was obtained.
M.P.: 252-253°C.
¹H NMR (DMSO-d₆) δ: 3.91 (3H, s), 7.25-7.37 (2H, m), 7.58-7.61 (1H, m), 8.15 (1H, dd, J=1.3, 6.6Hz), 8.3 (2H, br-s), 8.6 (2H, br-s), 8.78 (1H, s), 11.8 (1H, br-s).

### Example 4

### Preparation of 1-methyl-4-indoloylguanidine

The reaction was carried out in a manner similar to Example 1 except for using 0.85 g (4.49 mmol) of methyl 1-methyl-4-indolecarboxylate, 4.29 g (44.9 mmol) of guanidine hydrochloride and 50 ml of a methanol solution of 2.43 g (44.9 mmol) of sodium methoxide. Thus 0.75 g (66.1%) of 1-methyl-4-indoloylguanidine hydrochloride was obtained.
M.P.: 186-187°C.
¹H NMR (DMSO-d₆) δ: 3.88 (3H, s), 6.97 (1H, d, J=3.0Hz), 7.92-7.35 (1H, m), 7.56 (1H, d, J=3.0Hz), 7.84 (1H, d, J=7.9Hz), 7.98 (1H, d, J=7.6Hz), 8.5 (2H, br-s), 8.7 (2H, br-s), 11.7 (1H, br-s).

### Example 5

### Preparation of 4-chloro-1-methyl-2-indoloylguanidine

The reaction was carried out in a manner similar to Example 1 except for using 2.00 g (8.94 mmol) of methyl 4-chloro-1-methyl-2-indolecarboxylate, 8.54 g (89.4 mmol) of guanidine hydrochloride and 50 ml of a methanol solution of 4.83 g (89.4 mmol) of sodium methoxide. Thus 1.06 g (41.3%) of 4-chloro-1-methyl-2-indoloylguanidine hydrochloride was obtained.
M.P.: 288-290°C.
¹H NMR (DMSO-d₆) δ: 4.05 (3H, s), 7.24 (1H, d, J=7.6Hz), 7.35-7.41 (1H, m), 7.62 (1H, d, J=8.6Hz), 7.98 (1H, s), 8.56 (2H, br-s), 8.63 (2H, br-s), 12.0 (1H, br-s).

The compounds of Examples 6 through 81 were prepared in a manner similar to Example 1.

### Example 6

### 5-Chloro-1-methyl-2-indoloylguanidine hydrochloride:

Yield: 43.6%, M.P.: 281-282°C
¹H NMR (DMSO-d₆) δ: 4.03 (3H, s), 7.39 (1H, dd, J=2.0, 8.9Hz), 7.67 (1H, d, J=8.9Hz), 7.77 (1H, s), 7.81 (1H, d, J=1.7Hz), 8.5 (2H, br-s), 8.6 (2H, br-s), 11.9 (1H, br-s).

### Example 7

### 6-Chloro-1-methyl-2-indoloylguanidine hydrochloride:

Yield: 59.6%, M.P.: 290-294°C
¹H NMR (DMSO-d₆) δ: 4.02 (3H, s), 7.17 (1H, dd, J=2.0, 8.6Hz), 7.74-7.77 (2H, m), 7.84 (1H, s), 8.5 (2H, br-s), 8.6 (2H, br-s), 11.9 (1H, br-s).

### Example 8

### 7-Chloro-1-methyl-2-indoloylguanidine hydrochloride:

Yield: 56.5%, M.P.: 243-244°C
¹H NMR (DMSO-d₆) δ: 4.33 (3H, s), 7.11-7.17 (1H, m), 7.41 (1H, d, J=7.6Hz), 7.71 (1H, d, J=7.9Hz), 7.81 (1H, s), 8.5 (2H, br-s), 8.6 (2H, br-s), 12.0 (1H, br-s).

### Example 9

### 1,4-Dimethyl-2-indoloylguanidine hydrochloride:

Yield: 32.5%, M.P.: 279-280°C
¹H NMR (DMSO-d₆) δ: 2.53 (3H, s), 4.02 (3H, s), 6.96 (1H, d, J=6.9Hz), 7.26-7.32 (1H, m), 7.41 (1H, d, J=8.3Hz), 7.99 (1H, s), 8.5 (2H, br-s), 8.7 (2H, br-s), 11.9 (1H, br-s).

### Example 10

### 1,5-Dimethyl-2-indoloylguanidine hydrochloride:

Yield: 30.5%, M.P.: 281-282°C
¹H NMR DMSO-d₆) δ: 2.41 (3H, s), 4.00 (3H, s), 7.23 (1H, d, J=8.9Hz), 7.48-7.51 (2H, m), 7.79 (1H, s), 8.5 (2H, br-s), 8.7 (2H, br-s), 11.9 (1H, br-s).

### Example 11

### 1,6-Dimethyl-2-indoloylguanidine hydrochloride:

Yield: 63.1%, M.P.: 267-269°C
¹H NMR (DMSO-d₆) δ: 2.47 (3H, s), 3.99 (3H, s), 7.02 (1H, d, J=8.3Hz), 7.41 (1H, s), 7.61-8.00 (2H, m), 8.4 (2H, br-s), 8.5 (2H, br-s), 11.6 (1H, br-s).

### Example 12

### 1,7-Dimethyl-2-indoloylguanidine hydrochloride:

Yield: 27.3%, M.P.: 271-273°C
¹H NMR (DMSO-d₆) δ: 2.78 (3H, s), 4.25 (3H, s), 6.99-7.11 (2H, m), 7.53 (1H, d, J=7.6Hz), 7.70 (1H, s), 8.4 (2H, br-s), 8.6 (2H, br-s), 11.8 (1H, br-s).

### Example 13

### 5-Methoxy-1-methyl-2-indoloylguanidine hydrochloride:

Yield: 50.1%, M.P.: 235-236°C
¹H NMR (DMSO-d₆) δ: 3.80 (3H, s), 4.01 (3H, s), 7.03-7.07 (1H, m), 7.16 (1H, d, J=2.3Hz), 7.52 (1H, d, J=8.9Hz), 7.75 (1H, s), 8.4 (2H, br-s), 8.7 (2H, br-s), 11.8 (1H, br-s).

### Example 14

### 1-Methyl-6-indoloylguanidine hydrochloride:

Yield: 62.1%, M.P.: 297-298°C
¹H NMR (DMSO-d₆) δ: 3.94 (3H, s), 6.55 (1H, dd, J=0.7, 3.0Hz), 7.61 (1H, d, J=3.0Hz), 7.67-7.78 (2H, m), 8.4 (2H, br-s), 8.6 (1H, br-s), 8.9 (2H, br-s), 12.0 (1H, br-s).

### Example 15

### 1-Benzyl-2-indoloylguanidine hydrochloride:

Yield: 54.9%, M.P.: 228-229°C
¹H NMR (DMSO-d₆) δ: 5.86 (2H, s), 7.03 (2H, d, J=6.6Hz), 7.17-7.39 (4H, m), 7.57 (1H, d, J=8.3Hz), 7.78 (1H, d, J=7.9Hz), 7.98 (1H, s), 8.4 (2H, br-s), 8.6 (2H, br-s), 11.9 (1H, br-s).

### Example 16

### 1-Benzyl-3-indoloylguanidine hydrochloride:

Yield: 66.2%, M.P.: 252-253°C
¹H NMR (DMSO-d₆) δ: 5.53 (2H, s), 7.23-7.37 (7H, m), 7.62-7.66 (1H, m), 8.15-8.18 (1H, m), 8.3 (2H, br-s), 8.6 (2H, br-s), 8.95 (1H, s), 11.8 (1H, br-s).

### Example 17

### 1-Isopropyl-3-indoloylguanidine hydrochloride:

Yield: 49.7%, M.P.: 221-223°C
¹H NMR (DMSO-d₆) δ: 1.51 (6H, d, J=6.6Hz), 4.85-4.90 (1H, m), 7.24-7.34 (2H, m), 7.67 (1H, d, J=7.6Hz), 8.14-8.17 (1H, m), 8.3 (2H, br-s), 8.6 (2H, br-s), 9.12 (1H, s), 11.9 (1H, br-s).

### Example 18

### 2-Indoloylguanidine hydrochloride:

Yield: 61.9%, M.P.: 192-194°C
¹H NMR (DMSO-d₆) δ: 7.09-7.14 (1H, m), 7.28-7.34 (1H, m), 7.49 (1H, d, J=8.3Hz), 7.71 (1H, d, J=8.3Hz), 8.5 (2H, br-s), 8.7 (2H, br-s), 12.06 (1H, br-s), 12.13 (1H, br-s).

### Example 19

### 3-Indoloylguanidine hydrochloride:

Yield: 42.2%, M.P.: 287°C
¹H NMR (DMSO-d₆) δ: 7.20-7.29 (2H, m), 7.53 (1H, dd, J=1.7, 6.6Hz), 8.12-8.16 (1H, m), 8.3 (2H, br-s), 8.7 (2H, br-s), 8.83 (1H, d, J=3.3Hz), 11.8 (1H, br-s), 12.2 (1H, br-s).

### Example 20

### 5-Indoloylguanidine hydrochloride:

Yield: 55.9%, M.P.: 219-222°C
¹H NMR (DMSO-d₆) δ: 6.61-6.63 (1H, m), 7.50-7.56 (2H, m), 7.85-7.89 (1H, m), 8.45 (2H, br-s), 8.49 (1H, d, J=1.7Hz), 8.75 (2H, br-s), 11.6 (1H, br-s), 11.7 (1H, br-s).

### Example 21

### 1-Isopropyl-5-indoloylguanidine hydrochloride:

Yield: 72.5%, M.P.: 219°C
¹H NMR (DMSO-d₆) δ: 1.48 (6H, d, J=6.6Hz), 4.81-4.88 (1H, m), 6.67 (1H, d, J=3.3Hz), 7.68-7.71 (2H, m), 7.89-7.93 (1H, m), 8.3-8.6 (3H, m), 8.7 (2H, br-s), 11.7 (1H, br-s).

### Example 22

### 4-Methoxy-1-methyl-2-indoloylguanidine hydrochloride:

Yield: 54.5%, M.P.: 281-282°C
¹H NMR (DMSO-d₆) δ: 3.93 (3H, s), 4.01 (3H, s), 6.62 (1H, d, J=7.9Hz), 7.16 (1H, d, J=8.6Hz), 7.30-7.36 (1H, m), 7.83 (1H, s), 8.5 (2H, br-s), 8.6 (2H, br-s), 11.7 (1H, br-s).

### Example 23

### 6-Methoxy-1-methyl-2-indoloylguanidine hydrochloride:

Yield: 75.5%, M.P.: 272°C
¹H NMR (DMSO-d₆) δ: 3.87 (3H, s), 4.00 (3H, s), 6.81 (1H, dd, J=2.0, 8.9Hz), 7.05 (1H, d, J=2.0Hz), 7.59 (1H, d, J=8.9Hz), 7.84 (1H, s), 8.4 (2H, br-s), 8.7 (2H, br-s), 11.8 (1H, br-s).

### Example 24

### 1-Methyl-4-nitro-2-indoloylguanidine hydrochloride:

Yield: 97.7%, M.P.: 292-293°C
¹H NMR (DMSO-d₆) δ: 4.14 (3H, s), 7.59-7.65 (1H, m), 8.16 (1H, m), 8.20-8.28 (2H, m), 8.5 (4H, br-s), 11.8 (1H, br-s).

### Example 25

### 1-Methyl-6-nitro-2-indoloylguanidine hydrochloride:

Yield: 68.4%, M.P.: 279-283°C
¹H NMR (DMSO-d₆) δ: 4.15 (3H, s), 7.89 (1H, s), 7.95-8.03 (2H, m), 8.51-8.66 (5H, m), 12.1 (1H, br-s).

### Example 26

### 1-Methyl-7-nitro-2-indoloylguanidine hydrochloride:

Yield: 66.8%, M.P.: 268-270°C
¹H NMR (DMSO-d₆) δ: 3.83 (3H, s), 7.36 (1H, t, J=7.9Hz), 7.98 (1H, s), 8.06 (1H, dd, J=1.0, 7.9Hz), 8.19 (1H, dd, J=1.0, 7.9Hz), 8.44-8.74 (4H, m), 12.2 (1H, br-s).

### Example 27

### 1-Methyl-5-nitro-2-indoloylguanidine hydrochloride:

Yield: 73.6%, M.P.: 294-295°C
¹H NMR (DMSO-d₆) δ: 4.09 (3H, s), 7.86-7.91 (2H, m), 8.23 (1H, dd, J=2.3, 9.2Hz), 8.49 (4H, br-s), 8.83 (1H, d, J=2.3Hz), 11.9 (1H, br-s).

### Example 28

### 1-Methyl-7-indoloylguanidine hydrochloride:

Yield: 37.4%, M.P.: 203-204°C
¹H NMR (DMSO-d₆) δ: 3.78 (3H, s), 6.60 (1H, d, J=3.3Hz), 7.16 (1H, t, J=7.6Hz), 7.44 (1H, d, J=3.0Hz), 7.53 (1H, d, J=7.6Hz), 7.85 (1H, d, J=7.9Hz), 8.44 (2H, br-s), 8.52 (2H, br-s), 11.90 (1H, br-s).

### Example 29

### 5-Fluoro-1-methyl-2-indoloylguanidine hydrochloride:

Yield: 60.8%, M.P.: 278-281°C
¹H NMR (DMSO-d₆) δ: 4.03 (3H, s), 7.25-7.33 (1H, m), 7.54 (1H, dd, J=2.3, 9.6Hz), 7.69 (1H, dd, J=4.6, 9.2Hz), 7.82 (1H, s), 8.51 (2H, br-s), 8.69 (2H, br-s), 11.98 (1H, br-s).

### Example 30

### 5-Ethoxy-1-methyl-2-indoloylguanidine hydrochloride:

Yield: 30.9%, M.P.: 234-236°C
¹H NMR (DMSO-d₆) δ: 1.35 (3H, t, J=6.9Hz), 3.99 (3H, s), 4.05 (2H, dd, J=6.9, 14.2Hz), 7.05 (1H, dd, J=2.3, 9.2Hz), 7.16 (1H, d, J=2.3Hz), 7.54 (1H, d, J=8.9Hz), 7.73 (1H, s), 8.42 (2H, br-s), 8.65 (2H, br-s), 11.81 (1H, br-s).

### Example 31

### 5-Benzyloxy-1-methyl-2-indoloylguanidine hydrochloride:

Yield: 45.2%, M.P.: 249-251°C
¹H NMR (DMSO-d₆) δ: 3.99 (3H, s), 5.14 (2H, s), 7.12-7.16 (1H, m), 7.28-7.58 (7H, m), 7.67 (1H, s), 8.28-8.68 (4H, m), 11.71 (1H, br-s).

### Example 32

### 7-Benzyloxy-1-methyl-2-indoloylguanidine hydrochloride:

Yield: 53.5%, M.P.: 221-222°C
¹H NMR (DMSO-d₆) δ: 4.27 (3H, s), 5.26 (2H, s), 6.97-7.08 (2H, m), 7.27-7.56 (5H, m), 7.72 (1H, s), 8.43 (2H, br-s), 8.60 (2H, br-s), 11.80 (1H, br-s).

### Example 33

### 1-(2-Naphthylmethyl)-2-indoloylguanidine hydrochloride:

Yield: 56.4%, M.P.: 254-255°C
¹H NMR (DMSO-d₆) δ: 6.02 (2H, s), 7.17-7.27 (2H, m), 7.32-7.38 (1H, m), 7.43-7.48 (3H, m), 7.60 (1H, d, J=7.9Hz), 7.73-7.86 (4H, m), 8.07 (1H, s), 8.43 (2H, br-s), 8.67 (2H, br-s), 12.04 (1H, br-s).

### Example 34

### 1-(2-Phenylethyl)-2-indoloylguanidine hydrochloride:

Yield: 55.1%, M.P.: 262-264°C
¹H NMR (DMSO-d₆) δ: 2.97-3.03 (2H, m), 4.73-4.79 (2H, m), 7.13-7.24 (6H, m), 7.32-7.38 (1H, m), 7.59 (1H, d, J=7.9Hz), 7.73 (1H, d, J=7.9Hz), 7.84 (1H, s), 8.43 (2H, br-s), 8.62 (2H, br-s), 11.78 (1H, br-s).

### Example 35

### 1-(4-Bromobenzyl)-2-indoloylguanidine hydrochloride:

Yield: 53.3%, M.P.: 260-263°C
¹H NMR (DMSO-d₆) δ: 5.82 (2H, s), 6.99 (2H, d, J=8.3Hz), 7.17-7.23 (1H, m), 7.35-7.40 (1H, m), 7.47 (2H, d, J=8.3Hz), 7.57 (1H, d, J=8.3Hz), 7.79 (1H, d, J=7.9Hz), 8.06 (1H, s), 8.47 (2H, br-s), 8.69 (2H, br-s), 12.07 (1H, br-s).

### Example 36

### 1-(4-Nitrobenzyl)-2-indoloylguanidine hydrochloride:

Yield: 42.7%, M.P.: 245-247°C
¹H NMR (DMSO-d₆) δ: 5.98 (2H, s), 7.20-7.27 (3H, m), 7.39 (1H, t, J=7.3Hz), 7.56 (1H, d, J=8.3Hz), 7.82 (1H, d, J=7.9Hz), 8.05 (1H, s), 8.16 (2H, d, J=8.6Hz), 8.41 (2H, br-s), 8.61 (2H, br-s), 12.02 (1H, br-s).

### Example 37

### 1-(4-Methoxybenzyl)-2-indoloylguanidine hydrochloride:

Yield: 54.8%, M.P.: 239-240°C
¹H NMR (DMSO-d₆) δ: 3.68 (3H, s), 5.78 (2H, s), 6.82 (2H, d, J=8.6Hz), 7.18 (1H, t, J= 7.3Hz), 7.34-7.40 (1H, m), 7.61 (1H, d, J=8.6Hz), 7.77 (1H, d, J=7.9Hz), 7.92 (1H, s), 8.43 (2H, br-s), 8.60 (2H, br-s), 11.89 (1H, br-s).

### Example 38

### 1-(3-Phenylpropyl)-2-indoloylguanidine hydrochloride:

Yield: 39.0%, M.P.: 147-148°C
¹H NMR (DMSO-d₆) δ: 1.97-2.13 (2H, m), 5.62 (2H, t, J=8.0Hz), 4.59 (2H, t, J=7.0Hz), 7.11-7.34 (6H, m), 7.40 (1H, dt, J=1.0, 8.0Hz), 7.57 (1H, d, J=8.0Hz), 7.76 (1H, d, J=8.0Hz), 7.81 (1H, s), 8.25-8.70 (4H, m), 11.75 (1H, br-s).

### Example 39

### 1-(4-Phenylbutyl)-2-indoloylguanidine hydrochloride:

Yield: 51.0%, M.P.: 154-155°C
¹H NMR (DMSO-d₆) δ: 1.43-1.65 (2H, m), 1.65-1.68 (2H, m), 2.57 (2H, t, J=8.0Hz), 4.58 (1H, t, J=7.0Hz), 7.03-7.32 (6H, m), 7.39 (1H, dt, J= 1.0, 8.0Hz), 7.63 (1H, d, J=8.0Hz), 7.73 (1H, d, J=8.0Hz), 7.92 (1H, s), 8.20-9.00 (4H, m), 11.95 (1H, br-s).

### Example 40

### 1-Isopropyl-6-indoloylguanidine hydrochloride:

Yield: 37.7%, M.P.: 218-220°C
¹H NMR (DMSO-d₆) δ: 1.51 (6H, d, J=6.6Hz), 4.92-5.02 (1H, m), 6.59 (1H, d, J=3.0Hz), 7.66-7.81 (3H, m), 8.41 (2H, br-s), 8.66 (1H, s), 8.86 (2H, br-s), 12.04 (1H, br-s).

### Example 41

### 1-Benzyl-6-indoloylguanidine hydrochloride:

Yield: 44.5%, M.P.: 227-228°C
¹H NMR (DMSO-d₆) δ: 5.57 (2H, s), 6.62 (1H, d, J=3.0Hz), 7.24-7.32 (5H, m), 7.69-7.79 (2H, m), 7.81 (1H, d, J=3.0Hz), 8.43 (2H, br-s), 8.71 (1H, s), 8.86 (2H, br-s), 12.06 (1H, br-s).

### Example 42

### 1-Isopropyl-4-indoloylguanidine hydrochloride:

Yield: 49.0%, M.P.: 95-97°C
¹H NMR (DMSO-d₆) δ: 1.48 (6H, d, J=6.6Hz), 4.87 (1H, m), 7.01 (1H, d, J=3.0Hz), 7.26-7.31 (1H, m), 7.72 (1H, d, J=3.3Hz), 7.91 (1H, d, J=8.3Hz), 8.02 (1H, d, J=7.6Hz), 8.54 (2H, br-s), 8.83 (2H, br-s), 11.85 (1H, br-s).

### Example 43

### 1-Benzyl-4-indoloylguanidine hydrochloride:

Yield: 42.6%, M.P.: 203-205°C
¹H NMR (DMSO-d₆) δ: 5.52 (2H, s), 7.03 (1H, d, J=3.0Hz), 7.17-7.32 (6H, m), 7.74 (1H, t, J=1.7Hz), 7.84 (1H, d, J=7.9Hz), 7.98 (1H, d, J=7.6Hz), 8.48 (2H, br-s), 8.77 (2H, br-s), 11.79 (1H, br-s).

### Example 44

### 4-Benzyloxy-1-methyl-2-indoloylguanidine hydrochloride:

Yield: 57.6%, M.P.: 260°C
¹H NMR (DMSO-d₆) δ: 4.01 (3H, s), 5.26 (2H, s), 6.75 (1H, d, J=7.6Hz), 7.20 (1H, d, J=8.6Hz), 7.30-7.54 (6H, m), 7.75 (1H, s), 8.40 (4H, br-s), 11.41 (1H, br-s).

### Example 45

### 1,3-Dimethyl-2-indoloylguanidine hydrochloride:

Yield: 55.5%, M.P.: 228-229°C
¹H NMR (DMSO-d₆) δ: 2.56 (3H, s), 3.84 (3H, s), 7.12-7.18 (1H, m), 7.34-7.40 (1H, m), 7.53 (1H, d, J=8.3Hz), 7.69 (1H, d, J=7.9Hz), 8.61-8.68 (4H, m), 11.67 (1H, br-s).

### Example 46

### 4-Acetyl-1-methyl-2-indoloylguanidine hydrochloride:

Yield: 45.4%, M.P.: 288-289°C
¹H NMR (DMSO-d₆) δ: 2.71 (3H, s), 4.07 (3H, s), 7.50-7.56 (1H, m), 7.91-7.97 (2H, m), 8.25 (1H, s), 8.53 (4H, br-s), 11.71 (1H, br-s).

### Example 47

### 6-Benzyloxy-1-methyl-2-indoloylguanidine hydrochloride:

Yield: 42.7%, M.P.: 269-270°C
¹H NMR (DMSO-d₆) δ: 3.99 (3H, s), 5.20 (2H, s), 6.89 (1H, d, J=10.6Hz), 7.22 (1H, s), 7.35-7.58 (6H, m), 7.62-7.67 (1H, m), 8.4 (4H, br-s), 11.35 (1H, br-s).

### Example 48

### 4-Ethoxy-1-methyl-2-indoloylguanidine hydrochloride:

Yield: 69.8%, M.P.: 262-263°C
¹H NMR (DMSO-d₆) δ: 1.42 (3H, t, J=6.9Hz), 3.99 (3H, s), 4.19 (2H, q, J=6.9Hz), 6.62 (1H, d, J=7.6Hz), 7.16 (1H, d, J=8.6Hz), 7.28-7.34 (1H, m), 7.77 (1H, s), 8.51 (4H, br-s), 11.60 (1H, br-s).

### Example 49

### 1-(2-Carbamoylethyl)-2-indoloylguanidine hydrochloride:

Yield: 30.0%, M.P.: 285-286°C
¹H NMR (DMSO-d₆) δ: 2.55 (2H, t, J=7.3Hz), 4.74 (2H, t, J=7.3Hz), 6.85 (1H, br-s), 7.17 (1H, t, J=6.9Hz), 7.33 (1H, br-s), 7.39 (1H, ddd, J=1.0, 7.3, 7.8Hz), 7.70 (2H, dd, J=8.4, 17.7Hz), 7.82 (1H, s), 8.46 (2H, br-s), 8.64 (2H, br-s), 11.85 (1H, br-s).

### Example 50

### 1-Propyl-2-indoloylguanidine hydrochloride:

Yield: 53.2%, M.P.: 218-219°C
¹H NMR (DMSO-d₆) δ: 0.85 (3H, t, J=7.6Hz), 1.66-1.77 (2H, m), 4.51 (2H, dd, J=6.9, 7.6Hz), 7.10-7.23 (1H, m), 7.32-7.45 (1H, m), 7.65 (1H, d, J=8.6Hz), 7.73 (1H, d, J=7.9Hz), 7.97 (1H, s), 8.52 (2H, br-s), 8.77 (2H, br-s), 12.01 (1H, br-s).

### Example 51

### 1-(2-Methoxyethyl)-2-indoloylguanidine hydrochloride:

Yield: 15.0%, M.P.: 174-176°C
¹H NMR (DMSO-d₆) δ: 3.16 (3H, s), 3.63 (2H, t, J=5.3Hz), 4.72 (2H, t, J=5.3Hz), 7.11-7.22 (1H, m), 7.31-7.44 (1H, m), 7.66 (1H, d, J=8.6Hz), 7.72 (1H, d, J=7.9Hz), 7.89 (1H, s), 8.49 (2H, br-s), 8.70 (2H, br-s), 11.96 (1H, br-s).

### Example 52

### 4-Fluoro-1-methyl-2-indoloylguanidine hydrochloride:

Yield: 53.1%, M.P.: 281-282°C
¹H NMR (DMSO-d₆) δ: 4.04 (3H, s), 6.97 (1H, dd, J=7.6, 10.2Hz), 7.35-7.43 (1H, m), 7.50 (1H, d, J=8.3Hz), 7.89 (1H, s), 8.48-8.60 (4H, m), 11.92 (1H, br-s).

### Example 53

### 4-Bromo-1-methyl-2-indoloylguanidine hydrochloride:

Yield: 58.2%, M.P.: 306-307°C
¹H NMR (DMSO-d₆) δ: 4.04 (3H, s), 7.30-7.36 (1H, m), 7.42 (1H, d, J=7.6Hz), 7.69 (1H, d, J=8.6Hz), 7.78 (1H, s), 8.56 (4H br-s), 11.91 (1H, br-s).

### Example 54

### 4-Isobutyloxy-1-methyl-2-indoloylguanidine hydrochloride:

Yield: 58.1%, M.P.: 245-247°C
¹H NMR (DMSO-d₆) δ: 1.05 (6H, d, J=6.9Hz), 2.06-2.16 (1H, m), 3.90 (2H, d, J=6.3Hz), 3.99 (3H, s), 6.61 (1H, d, J=7.9Hz), 7.16 (1H, d, J=8.6Hz), 7.28-7.34 (1H, m), 7.84 (1H, s), 8.51 (4H, br-s), 11.65 (1H, br-s).

### Example 55

### 4-Isopropoxy-1-methyl-2-indoloylguanidine hydrochloride:

Yield: 62.3% M.P.: 269-270°C
¹H NMR (DMSO-d₆) δ: 1.35 (6H, d, J=5.9Hz), 3.99 (3H, s), 4.75-4.84 (1H, m), 6.65 (1H, d, J=7.6Hz), 7.14 (1H, d, J=8.6Hz), 7.28-7.34 (1H, m), 7.75 (1H, s), 8.53 (4H, br-s), 11.59 (1H, br-s).

### Example 56

### 1-Methyl-7-(2-phenylethoxy)-2-indoloylguanidine hydrochloride:

Yield: 24.3%, M.P.: 155-156°C
¹H NMR (DMSO-d₆) δ: 3.21 (2H, t, J=6.3Hz), 4.13 (3H, s), 4.43 (2H, t, J=6.3Hz), 6.95 (1H, d, J=7.9Hz), 7.08 (1H, t, J=7.9Hz), 7.25-7.44 (6H, m), 7.60 (1H, s), 8.44 (4H, br-s), 11.62 (1H, br-s).

### Example 57

### 1-Methyl-7-(3-phenylpropoxy)-2-indoloylguanidine hydrochloride:

Yield: 46.1%, M.P.: 165-166°C
¹H NMR (DMSO-d₆) δ: 2.12-2.17 (2H, m), 2.79-2.85 (2H, m), 4.09-4.13 (2H, m), 4.31 (3H, s), 6.83 (1H, m), 7.00-7.05 (1H, m), 7.19-7.32 (6H, m), 7.67 (1H, s), 8.56 (4H, br-s), 11.75 (1H, br-s).

### Example 58

### 7-Benzyloxy-4-chloro-1-methyl-2-indoloylguanidine hydrochloride:

Yield: 54.4%, M.P.: 264°C
¹H NMR (DMSO-d₆) δ: 4.27 (3H, s), 5.26 (2H, s), 6.96 (1H, d, J=8.6Hz), 7.11 (1H, d, J=8.3Hz), 7.32-7.54 (5H, m), 7.78 (1H, s), 8.5-8.6 (4H, m), 11.94 (1H, br-s).

### Example 59

### 4-Carboxy-1-methyl-2-indoloylguanidine hydrochloride:

Yield: 40.5%, M.P.: 302-303°C
¹H NMR (DMSO-d₆) δ: 4.07 (3H, s), 7.48-7.54 (1H, m), 7.86-7.95 (2H, m), 8.10 (1H, s), 8.3-8.7 (4H, m), 11.58 (1H, br-s), 13.0 (0.7H, br-s).

### Example 60

### 7-Carbamoylmethoxy-1-methyl-2-indoloylguanidine hydrochloride:

Yield: 56.7%, M.P.: 268-269°C
¹H NMR (DMSO-d₆) δ: 4.32 (3H, s), 4.61 (2H, s), 6.76 (1H, d, J=7.9Hz), 7.03 (1H, t, J=7.9Hz), 7.30 (1H, d, J=7.6Hz), 7.40 (1H, br-s), 7.58 (1H, br-s), 7.68 (1H, s), 8.54 (4H, m), 11.74 (1H, br-s).

### Example 61

### 7-Carbamoylmethoxy-4-chloro-1-methyl-2-indoloylguanidine hydrochloride:

Yield: 29.7%, M.P.: 270-271°C
¹H NMR (DMSO-d₆) δ: 4.33 (3H, s), 4.61 (2H, s), 6.73 (1H, d, J=8.3Hz), 7.10 (1H, d, J=8.3Hz), 7.39 (1H, br-s), 7.58 (1H, br-s), 7.74 (1H, s), 8.57 (4H, br-s), 11.93 (1H, br-s).

### Example 62

### 4-Chloro-7-(2-dimethylaminoethoxy)-1-methyl-2-indoloylguanidine hydrochloride:

Yield: 50.8%, M.P.: 287-288°C
¹H NMR (DMSO-d₆) δ: 2.86 (6H, d, J=5.0Hz), 3.62-3.64 (2H, m), 4.29 (3H, s), 4.51-4.55 (2H, m), 6.92 (1H, d, J=8.2Hz), 7.14 (1H, d, J=8.3Hz), 7.88 (1H, s), 8.6-8.9 (4H, m), 11.01 (1H, br-s), 12.13 (1H, br-s).

### Example 63

### 6-Carbamoylmethoxy-1-methyl-2-indoloylguanidine hydrochloride:

Yield: 26.8%, M.P.: 275°C
¹H NMR (DMSO-d₆) δ: 3.98 (3H, s), 4.53 (2H, s), 6.90-6.95 (1H, m), 7.11 (1H, d, J=2.0Hz), 7.45 (1H, br-s), 7.58 (1H, br-s), 7.65 (1H, d, J=8.9Hz), 7.77 (1H, s), 8.38-8.58 (4H, m), 11.72 (1H, br-s).

### Example 64

### 1-Methyl-6-(2-phenylethoxy)-2-indoloylguanidine hydrochloride:

Yield: 48.6%, M.P.: 219-221°C
¹H NMR (DMSO-d₆) δ: 3.07-3.12 (2H, m), 3.97 (3H, s), 4.29 (2H, t, J=6.9Hz), 6.79-6.83 (1H, m), 7.11 (1H, d, J=2.0Hz), 7.23-7.39 (5H, m), 7.60 (1H, d, J=8.6Hz), 7.74 (1H, s), 8.36-8.56 (4H, m), 11.67 (1H, br-s).

### Example 65

### 1-Methyl-6-(3-phenylpropoxy)-2-indoloylguanidine hydrochloride:

Yield: 72.4%, M.P.: 232-233°C
¹H NMR (DMSO-d₆) δ: 2.02-2.13 (2H, m), 2.75-2.81 (2H, m), 3.97 (3H, s), 4.07 (2H, t, J=6.3Hz), 6.82-6.86 (1H, m), 7.06 (1H, d, J=1.7Hz), 7.16-7.33 (5H, m), 7.61 (1H, d, J=8.9Hz), 7.75 (1H, s), 8.36-8.58 (4H, m), 11.69 (1H, br-s).

### Example 66

### 4-Chloro-1-(2-methoxyethyl)-2-indoloylguanidine hydrochloride:

Yield: 27.0%, M.P.: 147-150°C
¹H NMR (DMSO-d₆) δ: 3.15 (3H, s), 3.63 (2H, t, J=5.3Hz), 4.73 (2H, t, J=5.3Hz), 7.26 (1H, d, J=6.9Hz), 7.31-7.44 (1H, m), 7.66 (1H, d, J=8.6Hz), 7.94 (1H, s), 8.60 (2H, br-s), 8.67 (2H, br-s), 12.05 (1H, br-s).

### Example 67

### 1-(2-carbamoylethyl)-4-chloro-2-indoloylguanidine hydrochloride:

Yield: 11.0%, M.P.: 295°C
¹H NMR (DMSO-d₆) δ:2.56 (2H, t, J=6.9Hz), 4.76 (2H, t, J=6.9Hz), 6.84 (1H, br-s), 7.26 (1H, d, J=7.7Hz), 7.30-7.46 (2H, m), 7.68 (1H, d, J=8.2Hz), 7.89 (1H, s), 8.56 (2H, br-s), 8.62 (2H, br-s), 11.95 (1H, br-s).

### Example 68

### 4-Chloro-1-methyl-7-[2-(N-pyrrolidinyl)ethoxy]-2-indoloylguanidine hydrochloride:

Yield: 53.8%, M.P.: 250°C
¹H NMR (DMSO-d₆) δ: 1.93-2.03 (4H, m), 3.0-3.2 (2H, m), 3.61-3.71 (4H, m), 4.30 (3H, s), 4.51-4.54 (2H, m), 6.92 (1H, d, J=8.2Hz), 7.14 (1H, d, J=8.3Hz), 7.85 (1H, s), 8.6-8.7 (4H, m), 11.20 (1H, br-s), 12.07 (1H, br-s).

### Example 69

### 4-Chloro-7-(3-dimethylaminopropoxy)-1-methyl-2-indoloylguanidine hydrochloride:

Yield: 35.4%, M.P.: 250°C
¹H NMR (DMSO-d₆) δ: 2.24-2.30 (2H, m), 2.78 (6H, s), 3.2-3.3 (2H, m), 4.20 (2H, t, J=5.9Hz), 4.29 (3H, s), 6.85 (1H, d, J=8.3Hz), 7.11 (1H, d, J=8.3Hz), 7.82 (1H, s), 8.5-8.7 (4H, m), 10.74 (1H, br-s), 12.04 (1H, br-s).

### Example 70

### 7-[2-(N-Benzyl-N-methylamino)ethoxy]-4-chloro-1-methyl-2-indoloylguanidine hydrochloride:

Yield: 43.5%, M.P.: 230°C
¹H NMR (DMSO-d₆) δ: 2.80 (3H, s), 3.61 (2H, br-s), 4.20 (3H, s), 4.40-4.57 (4H, m), 6.89 (1H, d, J=8.3Hz), 7.13 (1H, d, J=8.3Hz), 7.45-7.47 (3H, m), 7.6-7.7 (2H, m), 7.82 (1H, s), 8.5-8.7 (4H, m), 11.10 (1H, br-s), 12.04 (1H, br-s).

### Example 71

### 4-Isopropenyl-1-methyl-2-indoloylguanidine hydrochloride:

Yield: 41.5%, M.P.: 235°C
¹H NMR (DMSO-d₆) δ: 2.24 (3H, s), 4.03 (3H, s), 5.35-5.36 (1H, m), 5.48 (1H, d, J=1.0Hz), 7.15 (1H, dd, J=0.7, 7.3Hz), 7.38 (1H, dd, J=7.3, 8.6Hz), 7.56 (1H, d, J=8.6Hz), 8.07 (1H, s), 8.45-8.70 (4H, m), 12.03 (1H, br-s).

### Example 72

### 4-Isopropenyl-1-methyl-2-indoloylguanidine hydrochloride:

Yield: 75.6%, M.P.: 255°C
¹H NMR (DMSO-d₆) δ: 1.35 (6H, d, J=6.9Hz), 3.27-3.37 (1H, m), 4.02 (3H, s), 7.03 (1H, d, J=6.9Hz), 7.31-7.37 (1H, m), 7.44 (1H, d, J=8.6Hz), 8.08 (1H, s), 8.42-8.70 (4H, m), 11.97 (1H, br-s).

### Example 73

### 1-(2-Diethylaminoethyl)-2-indoloylguanidine hydrochloride:

Yield: 19.3%, M.P.: 250°C
¹H NMR (DMSO-d₆) δ: 1.28 (6H, t, J=7.3Hz), 3.10-3.43 (6H, m), 4.88-5.10 (2H, m), 7.23 (1H, t, J=7.6Hz), 7.46 (1H, ddd, J=1.0, 8.3, 8.7Hz), 7.76 (1H, d, J=7.6Hz), 7.94 (1H, d, J=8.7Hz), 8.09 (1H, br-s), 8.61 (2H, br-s), 8.79 (2H, br-s), 11.27 (1H, br-s), 12.3 (1H, br-s).

### Example 74

### 4-Chloro-1-(2-diethylaminoethyl)-2-indoloylguanidine hydrochloride:

Yield: 36.0%, M.P.: 260-261°C
¹H NMR (DMSO-d₆) δ: 1.28 (6H, t, J=7.3Hz), 3.10-3.48 (6H, m), 4.90-5.15 (2H, m), 7.31 (1H, d, J=7.7Hz), 7.45 (1H, dd, J=7.7, 8.3Hz), 7.98 (1H, d, J=8.3Hz), 8.14 (1H, br-s), 8.72 (2H, br-s), 8.75 (2H, br-s), 11.38 (1H, br-s), 12.33 (1H, br-s).

### Example 75

### 1-(2-Dimethylaminoethyl)-2-indoloylguanidine hydrochloride:

Yield: 27.0%, M.P.: 239-242°C
¹H NMR (DMSO-d₆) δ: 2.84 (6H, s), 3.23-3.53 (2H, m), 4.85-5.08 (2H, m), 7.23 (1H, dd, J=7.3, 7.9Hz), 7.41-7.43 (1H, m), 7.77 (1H, d, J=7.9Hz), 7.88 (1H, d, J=8.3Hz), 8.11 (1H, s), 8.64 (2H, br-s), 8.81 (2H, br-s), 11.09 (1H, br-s), 12.26 (1H, br-s).

### Example 76

### 4-Chloro-1-(2-dimethylaminoethyl)-2-indoloylguanidine hydrochloride:

Yield: 26.0%, M.P.: 245-248°C
¹H NMR (DMSO-d₆) δ: 2.84 (6H, s), 3.31-3.52 (2H, m), 4.88-5.08 (2H, m), 7.32 (1H, d, J=7.6Hz), 7.46 (1H, dd, J=7.6, 8.3Hz), 7.91 (1H, d, J=8.3Hz), 8.16 (1H, s), 8.71 (2H, br-s), 8.77 (2H, br-s), 11.19 (1H, m), 12.32 (1H, br-s).

### Example 77

### Preparation of 1-benzyl-5-indoloylguanidine

After 2.24 g (23.4 mmol) of guanidine hydrochloride was added to 50 ml of a methanol solution of 1.26 g (23.4 mmol) of sodium methoxide, 0.80 g (2.34 mmol) of benzyl 1-benzyl-5-indolecarboxylate was added to the resulting mixture. The mixture was then stirred for 30 hours while heating at 50 to 60°C. Methanol was distilled off under reduced pressure and the residue was purified by silica gel column chromatography followed by treatment with 2N hydrochloric acid to give 0.08 g (10.4%) of 1-benzyl-5-indoloylguanidine hydrochloride.
M.P.: 216-222°C
¹H NMR (DMSO-d₆) δ: 5.51 (2H, s), 6.69 (1H, d, J=2.6Hz), 7.20-7.34 (5H, m), 7.62-7.68 (2H, m), 7.88 (1H, dd, J=1.7, 8.9Hz), 8.43-8.48 (3H, m), 8.72 (2H, br-s), 11.7 (1H, br-s).

### Example 78

### Preparation of 7-methoxy-1-methyl-2-indoloylguanidine hydrochloride

a) The reaction was carried out in a manner similar to Reference Example 1-a) except for using 24.6 g (0.20 mmol) of 2-methoxyaniline, 15.2 g (0.22 mol) of sodium nitrite, 84 ml of conc. hydrochloric acid, 28.8 g (0.20 mmol) of ethyl 2-methylacetacetate and 20 ml of ethanol. Crude ethyl 2-(2-methoxyphenylhydrazono)propionate was obtained in an amount of 23.0 g.
b) After 23.0 g of the crude ethyl 2-(2-methoxyphenylhydrazono)propionate obtained above was added to 150 ml of 10% hydrogen chloride/ethanol, the mixture was refluxed for 30 minutes. After cooling, the reaction mixture was poured onto ice water and the mixture was extracted three times with ether. After washing with water and then with aqueous sodium bicarbonate solution, the extract was dried over anhydrous magnesium sulfate. The solvent was then distilled off under reduced pressure. The resulting residue was roughly purified by silica gel column chromatography to give 8.00 g of crude ethyl 7-methoxy-2-indolecarboxylate.
c) The reaction was carried out in a manner similar to Reference Example 5 except for using 8.00 g (36.5 mmol) of the crude ethyl 7-methoxy-2-indolecarboxylate obtained above, 1.44 g (36 mmol) of 60% sodium hydride, 7.76 g (54.7 mmol) of methyl iodide and 50 ml of dimethylformamide. Thus 4.4 g of crude ethyl 7-methoxy-1-methyl-2-indolecarboxylate was obtained.
d) The reaction was carried out in a manner similar to Example 1 except for using 4.40 g (18.9 mmol) of the crude ethyl 7-methoxy-1-methyl-2-indole-carboxylate obtained above, 18.0 g (189 mmol) of guanidine hydrochloride and 150 ml of a methanol solution of 10.2 g (189 mmol) of sodium methoxide. Thus 1.58 g of 7-methoxy-1-methyl-2-indoloylguanidine hydrochloride was obtained; yield: 5.6%, based on 2-methoxyaniline.

M.P.: 252-253°C
¹H NMR (DMSO-d₆) δ: 3.93 (3H, s), 4.28 (3H, s), 6.86 (1H, d, J=7.6Hz), 7.05 (1H, t, J=7.9Hz), 7.26 (1H, d, J=7.6Hz), 7.74 (1H, s), 8.5 (2H, br-s), 8.6 (2H, br-s), 11.8 (1H, br-s).

### Example 79

### Preparation of 1-isopropyl-2-indoloylguanidine

A tetrahydrofuran solution, 60 ml, containing 2.00 g (9.84 mmol) of 1-isopropyl-2-indolecarboxylic acid and 2.39 g (14.8 mmol) of carbonyldiimidazole was stirred at room temperature for 2 hours and then at 45 to 50°C for an hour. After cooling to room temperature, 30 ml of a dimethylformamide solution of 5.64 g (59.0 mmol) of guanidine hydrochloride and 5.97 g (59.0 mmol) of triethylamine was added to the reaction mixture followed by stirring at room temperature for 12 hours. The mixture was then distilled off under reduced pressure and water was added to the resulting residue. After adjusting pH in the range of 5 to 6 with 2N hydrochloric acid, the mixture was extracted three times with ethyl acetate. After drying over anhydrous magnesium sulfate, the extract was acidified with hydrogen chloride/ether. The precipitated crystals were filtered and dried to give 1.31 g (47.4%) of the desired 1-isopropyl-2-indoloyl-guanidine hydrochloride.
M.P.: 150-151°C
¹H NMR (DMSO-d₆) δ: 1.61 (6H, d, J=7.3Hz), 5.46-5.57 (1H, m), 7.15 (1H, t, J=7.9Hz), 7.32-7.38 (1H, m), 7.68-7.78 (3H, m), 8.5 (2H, br-s), 8.7 (2H, br-s), 11.8-11.9 (1H, m).

The reaction was carried out in a manner similar to Example 79 to obtain the compound of Example 80.

### Example 80

### 1-Carbamoylmethyl-2-indoloylguanidine hydrochloride:

Yield: 2.1%, M.P.: 261-262°C
¹H NMR (DMSO-d₆) δ: 5.17 (2H, s), 7.10-7.28 (2H, m), 7.32-7.45 (1H, m), 7.56 (1H, d, J=8.6Hz), 7.59 (1H, br-s), 7.75 (1H, dd, J=0.7, 7.0Hz), 7.81 (1H, s), 8.45 (2H, br-s), 8.61 (2H, br-s), 11.90 (1H, br-s).

### Example 81

### Preparation of 5-chloro-2-indoloylguanidine

The reaction was carried out in a manner similar to Example except for using 2.00 g (10.2 mmol) of 5-chloro-2-indolecarboxylic acid, 1.82 g (11.3 mmol) of carbonyldiimidazole, 5.86 g (61.3 mmol) of guanidine hydrochloride, 6.20 g (61.3 mmol) of triethylamine, 50 ml of tetrahydrofuran and 50 ml of dimethylformamide. Thus 1.85 g (66.2%) of 5-chloro-2-indoloylguanidine hydrochloride was obtained.
M.P.: 250°C or higher
¹H NMR (DMSO-d₆) δ: 7.32 (1H, dd, J=2.0, 8.9Hz), 7.51 (1H, d, J=8.9Hz), 7.82 (2H, s), 8.53 (2H, br-s), 8.68 (2H, br-s), 12.2 (1H, br-s), 12.3 (1H, br-s).

### Example 82

### Preparation of 6-amino-1-methyl-2-indoloylguanidine

After 1.10 g (4.21 mmol) of 1-methyl-6-nitro-2-indoloylguanidine was dissolved in a solvent mixture of 100 ml of tetrahydrofuran and 100 ml of methanol, 0.50 g of 10% palladium/carbon was added to the solution at room temperature in a nitrogen flow, while stirring. Catalytic hydrogenation was then performed at ambient temperature under normal pressure. After completion of the reaction, the catalyst was filtered off and the filtrate was then concentrated under reduced pressure. Hydrogen chloride/methanol was added to the resulting residue to convert the compound into the hydrochloride, whereby 0.73 g (64.7%) of 6-amino-1-methyl-2-indoloyl-guanidine hydrochloride was obtained.
M.P.: 282-283°C
¹H NMR (DMSO-d₆) δ: 4.00 (3H, s), 7.06 (1H, dd, J=1.7, 8.6Hz), 7.39 (1H, s), 7.76 (1H, d, J=8.6Hz), 7.93 (1H, s), 8.5 (2H, br-s), 8.7 (2H, br-s), 9.0-10.3 (2H, br), 12.0 (1H, br-s).

The reaction was carried out in a manner similar to Example 82 to prepare the compounds of Examples 83 through 85 shown below.

### Example 83

### 4-Amino-1-methyl-2-indoloylguanidine hydrochloride:

Yield: >99%, M.P.: 279-283°C
¹H NMR (DMSO-d₆) δ: 4.00 (3H, s), 6.80 (1H, d, J=7.6Hz), 7.20-7.31 (2H, m), 7.84 (1H, s), 8.5 (2H, br-s), 8.6 (2H, br-s), 11.9 (1H, br-s).

### Example 84

### 5-Amino-1-methyl-2-indoloylguanidine hydrochloride:

Yield: 89.8%, M.P.: 301-302°C
¹H NMR (DMSO-d₆) δ: 4.04 (3H, s), 7.35-7.39 (1H, m), 7.72-7.79 (2H, m), 7.93 (1H, s), 8.5 (2H, br-s), 8.7 (2H, br-s), 10.1 (2H, br-s), 12.1 (1H, br).

### Example 85

### 7-Amino-1-methyl-2-indoloylguanidine hydrochloride:

Yield: 66.7%, M.P.: 299-300°C
¹H NMR (DMSO-d₆) δ: 4.28 (3H, s), 7.08-7.14 (1H, m), 7.24 (1H, d, J=7.3Hz), 7.55 (1H, d, J=7.9Hz), 7.76 (1H, s), 8.5 (2H, br-s), 8.6 (2H, br-s), 12.0 (1H, br-s).

### Example 86

### Preparation of 5-hydroxy-1-methyl-2-indoloylguanidine

In 50 ml of methanol was dissolved 0.83 g (2.58 mmol) of 5-benzyloxy-1-methyl-2-indoloylguanidine obtained in Example 31. While stirring at room temperature, 0.30 g of 10% palladium/carbon was added to the solution in a nitrogen flow and catalytic hydrogenation was then conducted at ambient temperature under normal pressure. After completion of the reaction, the catalyst was filtered off and the filtrate was then concentrated under reduced pressure. The resulting residue was purified by silica gel column chromatography to give 5-hydroxy-1-methyl-2-indoloylguanidine. The 5-hydroxy-1-methyl-2-indoloylguanidine was further treated with hydrogen chloride/methanol to give 0.37 g (68.6%) of 5-hydroxy-1-methyl-2-indoloylguanidine hydrochloride.
M.P.: 288-289°C
¹H NMR (DMSO-d₆) δ: 3.96 (3H, s), 6.93-6.98 (2H, m), 7.43-7.47 (1H, m), 7.65 (1H, s), 8.43 (2H, br-s), 8.65 (2H, br-s), 9.18 (1H, s), 11.76 (1H, br-s).

The reaction was carried out in a manner similar to Example 86 to prepare the compounds of Examples 87 through 91 shown below.

### Example 87

### 7-Hydroxy-1-methyl-2-indoloylguanidine hydrochloride:

Yield: 53.0%, M.P.: 244-246°C
¹H NMR (DMSO-d₆) δ: 4.29 (3H, s), 6.71 (1H, d, J=7.6Hz), 6.88-6.94 (1H, m), 7.12 (1H, d, J=7.9Hz), 7.65 (1H, s), 8.42-8.56 (4H, m), 10.08 (1H, s), 11.70 (1H, br-s).

### Example 88

### 4-Hydroxy-1-methyl-2-indoloylguanidine hydrochloride:

Yield: 27.4%, M.P.: 267-268°C
¹H NMR (DMSO-d₆) δ: 3.96 (3H, s), 6.50 (1H, d, J=7.6Hz), 7.00 (1H, d, J=8.3Hz), 7.16-7.22 (1H, m), 7.71 (1H, s), 8.42 (4H, br-s), 10.14 (1H, br-s), 11.51 (1H, br-s).

### Example 89

### 6-Hydroxy-1-methyl-2-indoloylguanidine hydrochloride:

Yield: 73.4%, M.P.: 270-271°C
¹H NMR (DMSO-d₆) δ: 3.90 (3H, s), 6.72-6.76 (1H, m), 6.81 (1H, s), 7.53-7.61 (2H, m), 8.4 (4H, br-s), 9.76 (1H, br-s), 11.39 (1H, br-s).

### Example 90

### 4-Chloro-7-hydroxy-1-methyl-2-indoloylguanidine hydrochloride:

Yield: 23.9%, M.P.: 280°C
¹H NMR (DMSO-d₆) δ: 4.30 (3H, s), 6.70 (1H, d, J=7.9Hz), 6.96 (1H, d, J=8.3Hz), 7.68 (1H, s), 8.54 (4H, br-s), 10.37 (1H, s), 11.79 (1H, br-s).

### Example 91

### 4-Chloro-6-hydroxy-1-methyl-2-indoloylguanidine hydrochloride:

Yield: 40.1%, M.P.: 270°C
¹H NMR (DMSO-d₆) δ: 3.91 (3H, s), 6.83-6.84 (2H, m), 7.77 (1H, s), 8.3-8.7 (4H, m), 10.14 (1H, s), 11.72 (1H, br-s).

### Example 92

### Preparation of 4-acetamido-1-methyl-2-indoloylguanidine

### a) Preparation of ethyl 4-amino-1-methyl-2-indole-carboxylate

In a solvent mixture of 50 ml of tetrahydrofuran and 50 ml of methanol was dissolved 1.37 g (5.52 mmol) of ethyl 1-methyl-4-nitro-2-indole-carboxylate. Thereafter 0.30 g of 10% palladium/carbon was added to the solution and catalytic hydrogenation was then conducted at ambient temperature under normal pressure. After completion of the reaction, the catalyst was filtered off and the filtrate was then concentrated under reduced pressure. The resulting residue was purified by silica gel column chromatography to give 1.2 g (>99%) of ethyl 4-amino-1-methyl-2-indolecarboxylate.

### b) Preparation of ethyl-4-acetamido-1-methyl-2-indole-carboxylate

In 20 ml of pyridine was dissolved 1.2 g (5.52 mmol) of ethyl 4-amino-1-methyl-2-indolecarboxylate. While stirring at room temperature, 10 ml of anhydrous acetic acid was added to the solution. After stirring for 2 hours at room temperature, the reaction mixture was poured onto ice water. The mixture was extracted three times with ethyl acetate. The combined extracts were washed with 1N hydrochloric acid and then with saturated sodium hydrogencarbonate solution. The solvent was then distilled off under reduced pressure and the residue was purified by silica gel column chromatography to give 1.40 g (97.9%) of ethyl 4-acetamido-1-methyl-2-indolecarboxylate.

### c) Preparation of 4-acetamido-1-methyl-2-indoloylguanidine

The reaction was carried out in a manner similar to Example 1 except for using 1.40 g (5.38 mmol) of ethyl 4-acetamido-1-methyl-2-indole-carboxylate, 5.14 g (53.8 mmol) of guanidine hydrochloride and 50 ml of a methanol solution of 2.91 g (53.8 mmol) of sodium methoxide. Thus 1.15 g (69.0%) of 4-acetamido-1-methyl-2-indoloylguanidine hydrochloride was obtained.
M.P.: 277-279°C
¹H NMR (DMSO-d₆) δ: 2.15 (3H, s), 3.99 (3H, s), 7.30-7.35 (2H, m), 7.5-7.6 (1H, m), 7.79 (1H, s), 8.4-8.7 (4H, m), 10.00 (1H, br-s), 11.68 (1H, br-s).

The reaction was carried out in a manner similar to Example 92 to prepare the compounds of Examples 93 through 95 shown below.

### Example 93

### 5-Acetamido-1-methyl-2-indoloylguanidine hydrochloride:

Yield: 49.2%, M.P.: 260-261°C
¹H NMR (DMSO-d₆) δ: 2.06 (3H, s), 3.99 (3H, s), 7.46 (1H, dd, J=2.0, 8.9Hz), 7.56 (1H, d, J=8.9Hz), 7.83 (1H, s), 8.09 (1H, d, J=1.7Hz), 8.47 (2H, br-s), 8.71 (2H, br-s), 9.97 (1H, br-s), 11.92 (1H, br-s).

### Example 94

### 7-Acetamido-1-methyl-2-indoloylguanidine hydrochloride:

Yield: 17.1%, M.P.: 285°C
¹H NMR (DMSO-d₆) δ: 2.10 (3H, s), 4.07 (3H, s), 7.07-7.15 (2H, m), 7.61-7.64 (1H, m), 7.76 (1H, s), 8.45 (2H, br-s), 8.60 (2H, br-s), 9.90 (1H, br-s), 11.86 (1H, br-s).

### Example 95

### 6-Acetamido-1-methyl-2-indoloylguanidine hydrochloride:

Yield: 63.8%, M.P.: 280-281°C
¹H NMR (DMSO-d₆) δ: 2.09 (3H, s), 3.95 (3H, s), 7.18 (1H, dd, J=1.7, 8.6Hz), 7.64 (1H, d, J=8.9Hz), 7.72 (1H, s), 8.09 (1H, s), 8.2-8.8 (4H, m), 10.17 (1H, br-s), 11.75 (1H, br-s).

### Example 96

### Preparation of 1-hydroxy-2-indoloylguanidine

### a) Preparation of methyl 1-hydroxy-2-indolecarboxylate

The reaction was carried out in a manner similar to Reference Example 6 except for using 3.99 g (22.5 mmol) of 1-hydroxy-2-indolecarboxylic acid, 5.36 g (45.0 mmol) of thionyl chloride and 100 ml of methanol. Thus 2.56 g (59.5%) of methyl 1-hydroxy-2-indolecarboxylate was obtained.

### b) Preparation of 1-hydroxy-2-indoloylguanidine

The reaction was carried out in a manner similar to Example 1 except for using 1.00 g (5.23 mmol) of methyl 1-hydroxy-2-indolecarboxylate, 5.00 g (52.3 mmol) of guanidine hydrochloride and 50 ml of a methanol solution of 2.82 g (52.3 mmol) of sodium methoxide. 1-Hydroxy-2-indoloylguanidine hydrochloride was obtained in an amount of 0.56 g (42.0%).
M.P.: 217°C
¹H NMR (DMSO-d₆) δ: 7.13-7.19 (1H, m), 7.37-7.52 (2H, m), 7.69-7.73 (1H, m), 8.45 (2H, br-s), 8.70 (2H, br-s), 11.4-11.8 (2H, m).

### Example 97

### Preparation of 1-methoxy-2-indoloylguanidine

### a) Preparation of methyl 1-methoxy-2-indolecarboxylate

In a nitrogen flow 0.56 g (2.93 mmol) of methyl 1-hydroxy-2-indolecarboxylate was added to a suspension of 0.12 g (2.93 mmol) of 60% sodium hydride in 20 ml of tetrahydrofuran at room temperature. After it was confirmed that the reaction mixture became transparent, 0.83 g (5.86 mmol) of methyl iodide was added to the reaction mixture. The mixture was then refluxed for 2 hours. After cooling to room temperature, the reaction mixture was poured onto ice water followed by extraction three times with ethyl acetate. The combined extracts were washed with saturated sodium chloride solution. After drying over anhydrous magnesium sulfate, the solvent was distilled off under reduced pressure. The resulting residue was purified by silica gel column chromatography to give 0.46 g (76.5%) of methyl 1-methoxy-2-indolecarboxylate.

### b) Preparation of 1-methoxy-2-indoloylguanidine

The reaction was carried out in a manner similar to Example 1 except for using 0.46 g (2.24 mmol) of methyl 1-methoxy-2-indolecarboxylate, 2.14 g (22.4 mmol) of guanidine hydrochloride and 15 ml of a methanol solution of 1.21 g (22.4 mmol) of sodium methoxide. Thus 0.15 g (24.9%) of 1-methoxy-2-indoloylguanidine hydrochloride was obtained.
M.P.: 214°C
¹H NMR (DMSO-d₆) δ: 4.16 (3H, s), 7.21-7.26 (1H, m), 7.44-7.50 (1H, m), 7.62 (1H, d, J=8.6Hz), 7.74-7.79 (2H, m), 8.48 (2H, br-s), 8.66 (2H, br-s), 11.93 (1H, br-s).

### Example 98

### Preparation of 5-benzamido-1-methyl-2-indoloylguanidine

### a) Preparation of ethyl-5-benzamido-1-methyl-2-indole-carboxylate

In 20 ml of pyridine was dissolved 0.80 g (3.67 mmol) of ethyl 5-amino-1-methyl-2-indole-carboxylate. While stirring at room temperature, 0.57 g (4.03 mmol) of benzoyl chloride was added to the solution. After stirring for 2 hours at 70°C, the reaction mixture was cooled to room temperature and then poured onto ice water. The mixture was then extracted three times with ethyl acetate. The combined extracts were washed with 1N hydrochloric acid and then with saturated sodium hydrogencarbonate solution. After drying over anhydrous magnesium sulfate, the solvent was distilled off under reduced pressure. The residue was purified by silica gel column chromatography to give 0.62 g (52.5%) of ethyl 5-benzamido-1-methyl-2-indole-carboxylate.

### b) Preparation of 5-benzamido-1-methyl-2-indoloylguanidine

The reaction was carried out in a manner similar to Example 1 except for using 0.62 g (1.92 mmol) of ethyl 5-benzamido-1-methyl-2-indole-carboxylate, 3.68 g (38.4 mmol) of guanidine hydrochloride and 50 ml of a methanol solution of 2.08 g (38.4 mmol) of sodium methoxide. 5-Benzamido-1-methyl-2-indoloylguanidine hydrochloride was obtained in an amount of 0.38 g (53.1%).
M.P.: 185-190°C
¹H NMR (DMSO-d₆) δ: 4.03 (3H, s), 7.50-7.60 (4H, m), 7.63-7.74 (1H, m), 7.81 (1H, s), 7.96-8.00 (2H, m), 8.25 (1H, d, J=1.7Hz), 8.44 (2H, br-s), 8.62 (2H, br-s), 10.26 (1H, br-s), 11.82 (1H, br-s).

The reaction was carried out in a manner similar to Example 98 to prepare the compounds of Examples 99 through 101 shown below.

### Example 99

### 4-Benzamido-1-methyl-2-indoloylguanidine hydrochloride:

Yield: 54.7%, M.P.: 302-303°C
¹H NMR (DMSO-d₆) δ: 4.04 (3H, s), 7.37-7.64 (6H, m), 7.87 (1H, s), 8.05-8.09 (2H, m), 8.52 (4H, br-s), 10.35 (1H, br-s), 11.70 (1H, br-s).

### Example 100

### 7-Benzamido-1-methyl-2-indoloylguanidine hydrochloride:

Yield: 45.7%, M.P.: 318-319°C
¹H NMR (DMSO-d₆) δ: 4.07 (3H, s), 7.16-7.24 (2H, m), 7.53-7.72 (4H, m), 7.80 (1H, m), 8.04-8.06 (2H, m), 8.45 (2H, br-s), 8.61 (2H, br-s), 10.44 (1H, br-s), 11.88 (1H, br-s).

### Example 101

### 6-Benzamido-1-methyl-2-indoloylguanidine hydrochloride:

Yield: 40.1%, M.P.: 309°C
¹H NMR (DMSO-d₆) δ: 4.00 (3H, s), 7.48-7.62 (4H, m), 7.70-7.75 (2H, m), 7.98-8.01 (2H, m), 8.27 (1H, s), 8.2-8.8 (4H, m), 10.45 (1H, br-s), 11.73 (1H, br-s).

### Example 102

### Preparation of 1-(4-aminobenzyl)-2-indoloylguanidine

The reaction was carried out in a manner similar to Example 82 except for using 0.45 g (1.20 mmol) of 1-(4-nitrobenzyl)-2-indoloylguanidine, 0.50 g of 10% palladium/carbon, 25 ml of tetrahydrofuran and 25 ml of methanol. Thus 0.33 g (79.7%) of 1-(4-amino-benzyl)-2-indoloylguanidine hydrochloride was obtained.
M.P.: 226-228°C
¹H NMR (DMSO-d₆) δ: 5.83 (2H, s), 7.00-7.13 (4H, m), 7.17-7.23 (1H, m), 7.34-7.40 (1H, m), 7.58 (1H, d, J=8.3Hz), 7.79 (1H, d, J=7.9Hz), 8.02 (1H, s), 8.50 (2H, br-s), 8.66 (2H, br-s), 9.0-9.8 (2H, m), 12.01 (1H, br-s).

### Example 103

### Preparation of 1-(2-hydroxyethyl)-2-indoloylguanidine

The reaction was carried out in a manner similar to Example 1 except for using 1.00 g (3.30 mmol) of methyl 1-[2-(2-tetrahydropyranyl)oxyethyl]-2-indolecarboxylate, 3.15 g (33.0 mmol) of guanidine hydrochloride and a methanol solution of 1.78 g (33.0 mmol) of sodium methoxide. 1-[2-(2-Tetrahydropyranyl)-oxyethyl]-2-indoloylguanidine was obtained in an amount of 0.85 g. Thereafter 0.69 g of the thus obtained compound was dissolved in hydrochloric acid/methanol. The solution was stirred at room temperature for 5.5 hours. The reaction mixture was concentrated under reduced pressure and a solvent mixture of methanol and diethyl ether was added to the resulting residue. The precipitates formed were filtered and dried under reduced pressure to give 0.49 g (65%) of 1-(2-hydroxyethyl)-2-indoloylguanidine hydrochloride.
M.P.: 190-193°C
¹H NMR (DMSO-d₆) δ: 3.60-3.82 (2H, m), 4.60 (2H, t, J=5.0Hz), 4.74-4.97 (1H, br-s), 7.17 (1H, dt, J=7.0, 7.8Hz), 7.38 (1H, dt, J=7.0, 7.8Hz), 7.66 (1H, d, J=8.0Hz), 7.72 (1H, d, J=8.0Hz), 7.84 (1H, s), 8.20-8.90 (4H, m), 11.87 (1H, br-s).

The reaction was carried out in a manner similar to Example 103 to prepare the compounds of Examples 104 and 105 shown below.

### Example 104

### 1-(3-Hydroxypropyl)-2-indoloylguanidine hydrochloride:

Yield: 81.0%, M.P.: 206-207°C
¹H NMR (DMSO-d₆) δ: 1.90 (2H, dt, J=6.9, 7.3Hz), 3.39 (2H, t, J=6.3Hz), 4.60 (2H, t, J=6.9Hz), 7.18 (1H, dd, J=7.0, 7.8Hz), 7.41 (1H, dd, J=7.1, 8.5Hz), 7.65 (1H, d, J=8.2Hz), 7.74 (1H, d, J=7.8Hz), 7.88 (1H, s), 8.28-8.85 (4H, m), 11.87 (1H, br-s).

### Example 105

### 1-(4-Hydroxybutyl)-2-indoloylguanidine hydrochloride:

Yield: 84.0%, M.P.: 226°C
¹H NMR (DMSO-d₆) δ: 1.30-1.50 (2H, m), 1.62-1.86 (2H, m), 3.38 (2H, t, J=6.4Hz), 4.43 (1H, br-s), 4.56 (2H, t, J=7.3Hz), 7.17 (1H, t, J=7.4Hz), 7.40 (1H, ddd, J=1.0, 6.9, 7.4Hz), 7.65 (1H, d, J=8.3Hz), 7.73 (1H, d, J=7.9Hz), 7.96 (1H, s), 8.52 (2H, br-s), 8.76 (2H, br-s), 12.00 (1H, s).

### Example 106

### Preparation of 3-methyl-2-indoloylguanidine

### a) Preparation of ethyl 2-phenylhydrazonobutyronate

Ethyl 2-Phenylhydrazonobutyronate was obtained in a manner similar to Reference Example 1 a) except that aniline and ethyl 2-ethylacetacetate were used in place of o-chloroaniline and ethyl 2-methylacetacetate.

### b) Preparation of ethyl 3-methyl-2-indolecarboxylate

After 25.0 g of ethyl 2-henylhydrazonobutyronate was dissolved in 80 ml of hydrochloric acid/methanol, the solution was refluxed for an hour. After cooling to room temperature, the reaction mixture was poured onto ice water. The mixture was then extracted three times with diethyl ether. The combined extracts were washed with water and next with saturated sodium hydrogen-carbonate solution. After drying over anhydrous magnesium sulfate, the solvent was distilled off under reduced pressure. The resulting residue was purified by silica gel column chromatography to give 14.0 g (69.0%) of ethyl 3-methyl-2-indolecarboxylate.

### c) Preparation of 3-methyl-2-indoloylguanidine

The reaction was carried out in a manner similar to Example 1 except for using 1.50 g (7.38 mmol) of ethyl 3-methyl-2-indolecarboxylate, 7.05 g (73.8 mmol) of guanidine hydrochloride and 50 ml of a methanol solution of 3.99 g (73.8 mmol) of sodium methoxide. Thus 1.61 g (86.3%) of 3-methyl-2-indoloylguanidine hydrochloride.
M.P.: 285-286°C
¹H NMR (DMSO-d₆) δ: 2.60 (3H, s), 7.12 (1H, t, J=7.9Hz), 7.31-7.44 (2H, m), 7.70 (1H, d, J=7.9Hz), 8.46 (4H, br-s), 11.78 (1H, br-s), 11.94 (1H, br-s).

### Example 107

### Preparation of 1-methyl-7-(3-phenylpropionamido)-2-indoloylguanidine

### a) Preparation of ethyl 1-methyl-7-(3-phenylpropionamido)-2-indolecarboxylate

A suspension of 0.20 g (0.92 mmol) of ethyl 7-amino-1-methyl-2-indolecarboxylate, 0.14 g (0.94 mmol) of 3-phenylpropionic acid, 0.11 g (0.94 mmol) of 4-dimethylaminopyridine and 0.19 g (0.94 mmol) of dicyclohexylcarbodiimide in 5 ml of methylene chloride was stirred at room temperature for 24 hours. The reaction solution was poured onto ice water and the resulting mixture was extracted three times with ethyl acetate. The combined extracts were washed with 1N hydrochloric acid and next with 5% sodium hydrogencarbonate solution. After drying over anhydrous magnesium sulfate, the solvent was distilled off under reduced pressure. The resulting residue was purified by silica gel column chromatography to give ethyl 1-methyl 7-(3-phenylpropionamido)-2-indole-carboxylate.

### b) Preparation of 1-methyl-7-(3-phenylpropionamido)-2-indoloylguanidine

The reaction was carried out in a manner similar to Example 1 except for using 0.42 g (1.21 mmol) of ethyl 1-methyl-7-(3-phenylpropionamido)-2-indole-carboxylate, 2.31 g (24.2 mmols) of guanidine hydrochloride and a solution of 1.31 g (24.2 mmols) of sodium methoxide in 30 ml of methanol. 1-Methyl-7-(3-phenylpropionamido)-2-indoloylguanidine hydrochloride was obtained in an amount of 0.16 g (34.9%).
M.P.: 279-280°C
¹H NMR (DMSO-d₆) δ: 2.72 (2H, t, J=7.6Hz), 2.96 (2H, t, J=7.6Hz), 3.34 (3H, s), 7.03-7.14 (2H, m), 7.20-7.24 (1H, m), 7.29-7.31 (4H, m), 7.62 (1H, d, J=6.9Hz), 7.69 (1H, s), 8.53 (4H, m), 9.89 (1H, s), 11.76 (1H, br-s).

The compound of Example 108 was prepared in a manner similar to Example 107.

### Example 108

### 1-Methyl-6-(3-phenylpropionamido)-2-indoloylguanidine hydrochloride:

Yield: 34.6%, M.P.: 245-247°C
¹H NMR (DMSO-d₆) δ: 2.66-2.71 (2H, m), 2.91-2.97 (2H, m), 2.91-2.97 (2H, m), 3.95 (3H, s), 7.16-7.30 (6H, m), 7.63-7.71 (2H, m), 8.13 (1H, s), 8.36-8.52 (4H, m), 10.16 (1H, br-s), 11.67 (1H, br-s).

### Example 109

### Preparation of 1-(3-aminopropyl)-2-indoloylguanidine

The reaction was carried out in a manner similar to Example 1 except for using 1.50 g (4.51 mmol) of methyl 1-(3-tert-butoxycarbonylaminopropyl)-2-indole-carboxylate, 4.31 g (45.1 mmol) of guanidine hydrochloride and 60 ml of a methanol solution of 2.44 g (45.1 mmol) of sodium methoxide. 1-(3-tert-Butoxycarbonylaminopropyl)-2-indoloylguanidine hydrochloride was obtained in an amount of 1.57 g. After 1.55 g of the compound was dissolved in hydrochloric acid/methanol, the solution was stirred at 70°C for 3.5 hours. The reaction mixture was concentrated under reduced pressure. The resulting residue was recrystallized from water to give 0.65 g (46.0%) of 1-(3-aminopropyl)-2-indoloylguanidine hydrochloride.
M.P.: 296-297°C
¹H NMR (DMSO-d₆) δ: 2.05 (2H, ddd, J=7.6, 11.4, 14.5Hz), 2.63-2.86 (2H, m), 4.65 (2H, t, J=7.3Hz), 7.19 (1H, t, J=7.9Hz), 7.42 (1H, t, J=7.6Hz), 7.74 (2H, d, J=8.6Hz), 7.83-8.16 (4H, m), 8.27-9.03 (4H, m), 12.00-12.30 (1H, br-s).

The compound of Example 110 was prepared in a manner similar to Example 109.

### Example 110

### 1-(2-Aminoethyl)-2-indoloylguanidine hydrochloride:

Yield: 54.0%, M.P.: 240°C
¹H NMR (DMSO-d₆) δ: 3.14-3.30 (2H, m), 4.77 (2H, t, J=6.3Hz), 7.22 (1H, dd, J=7.3, 7.6Hz), 7.45 (1H, dd, J=7.3, 7.6Hz), 7.77 (1H, d, J=7.6Hz), 7.83 (1H, d, J=7.6Hz), 8.03 (1H, br-s), 8.20 (3H, br-s), 8.58 (2H, br-s), 8.74 (2H, br-s), 12.14 (1H, br-s).

### Example 111

### Preparation of 4-aminomethyl-1-methyl-2-indoloylguanidine

The reaction was carried out in a manner similar to Example 1 except for using 1.40 g (4.21 mmol) of ethyl 1-methyl-4-tert-butyloxycarbonylaminomethyl-2-indolecarboxylate, 4.02 g (42.1 mmol) of guanidine hydrochloride and 60 ml of a methanol solution of 2.27 g (42.1 mmol) of sodium methoxide. 1-Methyl-4-tert-butyloxycarbonylaminomethyl-2-indoloylguanidine hydrochloride was obtained in an amount of 1.50 g. After the compound was dissolved in 35 ml of trifluoroacetic acid and 70 ml of methylene chloride, the solution was stirred at room temperature for 2 hours. The reaction mixture was concentrated under reduced pressure. Thereafter ice water was poured onto the resulting residue and the aqueous layer was rendered alkaline with 28% aqueous ammonia. The mixture was then extracted three times with ethyl acetate. The combined extracts were washed with saturated sodium chloride solution. After drying over anhydrous magnesium sulfate, the solvent was distilled off under reduced pressure. The resulting residue was converted with hydrogen chloride/ether into the hydrochloride. Thus 0.58 g (43.2%) of 4-aminomethyl-1-methyl-2-indoloyl-guanidine hydrochloride was obtained.
M.P.: 283-284°C
¹H NMR (DMSO-d₆) δ: 4.06 (3H, s), 4.28 (2H, d, J=6.6Hz), 7.32 (1H, d, J=6.9Hz), 7.43-7.49 (1H, m), 7.66 (1H, d, J=8.3Hz), 8.28 (1H, s), 8.5-8.7 (5H, m), 8.79 (2H, br-s), 12.28 (1H, br-s).

The following compounds of Examples 112 to 117 were prepared in a manner similar to Example 111.

### Example 112

### 7-(3-Aminopropoxy)-1-methyl-2-indoloylguanidine hydrochloride:

Yield: 51.8%, M.P.: 287-288°C
¹H NMR (DMSO-d₆) δ: 2.09-2.17 (2H, m), 3.32 (2H, br-s), 4.21 (2H, t, J=5.9Hz), 4.28 (3H, s), 6.86 (1H, d, J=6.9Hz), 7.05 (1H, t, J=7.9Hz), 7.28 (1H, d, J=7.9Hz), 7.76 (1H, s), 7.98 (3H, br-s), 8.47-8.67 (4H, m), 11.92 (1H, br-s).

### Example 113

### 7-(3-Aminopropoxy)-4-chloro-1-methyl-2-indoloylguanidine hydrochloride:

Yield: 41.7%, M.P.: 299-300°C
¹H NMR (DMSO-d₆) δ: 2.14-2.19 (2H, m), 3.00-3.02 (2H, m), 4.21-4.26 (2H, m), 4.28 (3H, s), 6.83 (1H, d, J=8.3Hz), 7.10 (1H, d, J=8.3Hz), 7.88 (1H, s), 8.18 (3H, br-s), 8.6-8.7 (4H, m), 12.12 (1H, br-s).

### Example 114

### 6-(3-Aminopropoxy)-1-methyl-2-indoloylguanidine hydrochloride:

Yield: 61.3%, M.P.: 280-281°C
¹H NMR (DMSO-d₆) δ: 2.09-2.16 (2H, m), 3.01 (2H, t, J=6.3Hz), 4.02 (3H, s), 4.19-4.24 (2H, m), 6.87 (1H, dd, J=2.0, 8.9Hz), 7.15 (1H, s), 7.75 (1H, d, J=8.9Hz), 7.95 (1H, s), 8.07 (3H, br-s), 8.51-8.80 (4H, m), 12.00 (1H, br-s).

### Example 115

### 1-(3-Aminopropyl)-4-chloro-2-indoloylguanidine hydrochloride:

Yield: 46.0%, M.P.: 280-282°C
¹H NMR (DMSO-d₆) δ: 1.95-2.16 (2H, m), 2.65-2.88 (2H, m), 4.66 (2H, t, J=6.6Hz), 7.29 (1H, d, J=7.6Hz), 7.42 (1H, dd, J=7.6, 8.3Hz), 7.79 (1H, d, J=3.0Hz), 8.02 (3H, br-s), 8.11 (1H, s), 8.68 (2H, br-s), 8.78 (2H, br-s), 12.2 (1H, br-s).

### Example 116

### 7-(2-Aminoethoxy)-4-chloro-1-methyl-2-indoloylguanidine hydrochloride:

Yield: 31.9%, M.P.: 285°C
¹H NMR (DMSO-d₆) δ: 3.2-3.4 (2H, m), 4.30 (3H, s), 4.33-4.37 (2H, m), 6.89 (1H, d, J=8.3Hz), 7.13 (1H, d, J=8.3Hz), 7.83 (1H, s), 8.33 (3H, br-s), 8.6-8.7 (4H, m), 12.06 (1H, br-s).

### Example 117

### 6-(3-Aminopropoxy)-4-chloro-1-methyl-2-indoloylguanidine hydrochloride:

Yield: 38.7%, M.P.: 230°C
¹H NMR (DMSO-d₆) δ: 2.06-2.11 (2H, m), 2.97-2.99 (2H, m), 4.00 (3H, s), 4.18-4.23 (2H, m), 6.96 (1H, d, J=1.7Hz), 7.14 (1H, s), 7.95 (1H, s), 8.09 (3H, br-s), 8.5-8.7 (4H, m), 12.03 (1H, br-s).

### Example 118

### Synthesis of 4-hydroxymethyl-1-methyl-2-indoloylguanidine

The reaction was carried out in a manner similar to Example 1 except for using 1.50 g (4.73 mmol) of ethyl 1-methyl-4-(2-tetrahydropyranyl)oxymethyl-2-indolecarboxylate, 6.02 g (63.0 mmol) of guanidine hydrochloride and a solution of 3.40 g (63.0 mmol) of sodium methoxide in 60 ml of methanol. 1-Methyl-4-(2-tetrahyaropyranyl)oxymethyl-2-indoloylguanidine was obtained. After the compound was dissolved in a mixture of 30 ml of 2N hydrochloric acid and 60 ml of tetrahydrofuran, the mixture was stirred at room temperature for an hour. The reaction mixture was poured onto ice water and the aqueous layer was rendered alkaline with 28% aqueous ammonia. The mixture was then extracted three times with ethyl acetate. The combined extracts were washed with saturated sodium chloride solution. After drying over anhydrous magnesium sulfate, the solvent was distilled off under reduced pressure. The resulting residue was purified by silica gel column chromatography to give 4-hydroxymethyl-1-methyl-2-indoloylguanidine. Next, the compound was treated with hydrogen chloride/methanol to convert into the hydrochloride. 4-Hydroxymethyl-1-methyl-2-indoloyl-guanidine hydrochloride was thus obtained in an amount of 0.58 g (44.2%).
M.P.: 226-229°C
¹H NMR (DMSO-d₆) δ: 4.03 (3H, s), 4.80 (2H, s), 5.26 (1H, br-s), 7.17 (1H, d, J=6.9Hz), 7.34-7.39 (1H, m), 7.48 (1H, d, J=8.3Hz), 7.93 (1H, s), 8.48-8.60 (4H, m), 11.81 (1H, br-s).

The following compounds of Examples 119 to 128 were prepared in a manner similar to Example 118.

### Example 119

### 7-(2-Hydroxyethoxy)-1-methyl-2-indoloylguanidine hydrochloride:

Yield: 62.5%, M.P.: 243-244°C
¹H NMR (DMSO-d₆) δ: 3.82 (2H, br-s), 4.12-4.15 (2H, m), 4.31 (3H, s), 4.94 (1H, br-s), 6.86 (1H, d, J=7.3Hz), 7.03 (1H, t, J=7.9Hz), 7.26 (1H, d, J=7.3Hz), 7.73 (1H, s), 8.45-8.63 (4H, m), 11.82 (1H, br-s).

### Example 120

### 4-Chloro-7-(2-hydroxyethoxy)-1-methyl-2-indoloylguanidine hydrochloride:

Yield: 17.7%, M.P.: 277-279°C
¹H NMR (DMSO-d₆) δ: 3.79-3.83 (2H, m), 4.12-4.15 (2H, m), 4.31 (3H, s), 4.9 (1H, br-s), 6.85 (1H, d, J=8.3Hz), 7.10 (1H, d, J=8.3Hz), 7.75 (1H, s), 8.58 (4H, br-s), 11.88 (1H, br-s).

### Example 121

### 6-(2-Hydroxyethoxy)-1-methyl-2-indoloylguanidine hydrochloride:

Yield: 59.8%, M.P.: 265-268°C
¹H NMR (DMSO-d₆) δ: 3.32-3.77 (2H, m), 3.98 (3H, s), 4.08-4.11 (2H, m), 4.91 (2H, m), 4.91 (1H, br-s), 6.81-6.85 (1H, m), 7.08 (1H, s), 7.61 (1H, d, J=8.9Hz), 7.81 (1H, s), 8.39-8.64 (4H, m), 11.77 (1H, br-s).

### Example 122

### 4-Chloro-7-(2,3-dihydroxypropoxy)-1-methyl-2-indoloylguanidine hydrochloride:

Yield: 40.2%, M.P.: 237-238°C
¹H NMR (DMSO-d₆) δ: 3.50 (2H, t, J=5.9Hz), 3.88-3.91 (1H, m), 4.03 (1H, dd, J=5.6, 9.9Hz), 4.15 (1H, dd, J=4.0, 9.9Hz), 4.30 (3H, s), 6.85 (1H, d, J=8.3Hz), 7.11 (1H, d, J=8.3Hz), 7.68 (1H, s), 8.50 (4H, br-s), 11.76 (1H, br-s).

### Example 123

### 4-Chloro-7-(3-hydroxypropoxy)-1-methyl-2-indoloylguanidine hydrochloride:

Yield: 53.2%, M.P.: 210-212°C
¹H NMR (DMSO-d₆) δ: 1.93-2.02 (2H, m), 3.60-3.64 (2H, m), 4.15-4.20 (2H, m), 4.28 (3H, s), 6.84 (1H, d, J=8.6Hz), 7.09 (1H, d, J=8.3Hz), 7.77 (1H, s), 8.5-8.6 (4H, m), 11.92 (1H, br-s).

### Example 124

### 4-Chloro-7-(4-hydroxybutoxy)-1-methyl-2-indoloylguanidine hydrochloride:

Yield: 69.5%, M.P.: 220-222°C
¹H NMR (DMSO-d₆) δ: 1.59-1.67 (2H, m), 1.84-1.89 (2H, m), 3.45-3.50 (2H, m), 4.10-4.15 (2H, m), 4.29 (3H, s), 6.84 (1H, d, J=8.3Hz), 7.10 (1H, d, J=8.3Hz), 7.10 (1H, d, J=8.3Hz), 7.71 (1H, s), 8.52 (4H, br-s), 11.80 (1H, br-s).

### Example 125

### 4-Chloro-1-(3-hydroxypropyl)-2-indoloylguanidine hydrochloride:

Yield: 60.0%, M.P.: 213-215°C
¹H NMR (DMSO-d₆) δ: 1.78-1.98 (2H, m), 3.30-3.45 (2H, m), 4.61 (2H, t, J=7.3Hz), 4.68 (1H, br-s), 7.27 (1H, d, J=7.6Hz), 7.39 (1H, dd, J=7.3, 8.6Hz), 7.66 (1H, d, J=8.6Hz), 7.93 (1H, s), 8.55 (2H, br-s), 8.64 (2H, br-s), 11.96 (1H, br-s).

### Example 126

### 4-Chloro-1-(4-hydroxybutyl)-2-indoloylguanidine hydrochloride:

Yield: 48.0%, M.P.: 226-227°C
¹H NMR (DMSO-d₆) δ: 1.28-1.51 (2H, m), 1.60-1.84 (2H, m), 3.37 (2H, t, J=6.6Hz), 4.44 (1H, br-s), 4.58 (1H, t, J=7.3Hz), 7.28 (1H, d, J=7.6Hz), 7.39 (1H, dd, J=7.6, 8.6Hz), 7.68 (1H, d, J=8.6Hz), 7.92 (1H, s), 8.53 (2H, br-s), 8.63 (2H, br-s), 11.92 (1H, br-s).

### Example 127

### 4-Chloro-6-(2-hydroxyethoxy)-1-methyl-2-indoloylguanidine hydrochloride:

Yield: 51.9%, M.P.: 250-252°C
¹H NMR (DMSO-d₆) δ: 3.74-3.77 (2H, m), 3.99 (3H, s), 4.11 (2H, t, J=5.0Hz), 6.94 (1H, d, J=2.0Hz), 7.13 (1H, s), 7.80 (1H, s), 8.3-8.7 (4H, m), 11.76 (1H, br-s).

### Example 128

### 1-(3,4-Dihydroxybutyl)-2-indoloylguanidine hydrochloride:

Yield: 73.0%, M.P.: 219-222°C
¹H NMR (DMSO-d₆) δ: 1.53-1.73 (1H, m), 1.85-2.04 (1H, m), 3.12-3.55 (3H, m), 4.37-4.88 (4H, m), 7.18 (1H, t, J=7.3Hz), 7.40 (1H, ddd, J=1.0, 7.3, 7.8Hz), 7.65 (1H, d, J=8.3Hz), 7.74 (1H, d, J=7.9Hz), 7.86 (1H, s), 8.21 (2H, br-s), 8.67 (2H, br-s), 11.87 (1H, br-s).

### Example 129

### Preparation of 1-(2-carboxyethyl)-2-indoloylguanidine

After 0.80 g (2.23 mmol) of 1-[2-[1-(4-methyl-2,6,7-trioxabicyclo[2.2.2]octyl)]ethyl]-2-indoloylguanidine was suspended in 80 ml of 1,2-dimethoxyethane, 8 ml of 1N hydrochloric acid was added to the suspension. The mixture was stirred at room temperature for 20 minutes. Subsequently 10 ml of 4N sodium hydroxide solution was added to the mixture followed by stirring at room temperature for 40 minutes. Then 10 ml of 4N hydrochloric acid was added to the mixture followed by stirring at room temperature for an hour. The reaction mixture was concentrated under reduced pressure. After the resulting residue was washed with water, water was filtered off. The filtered matter was recrystallized from 0.5 N hydrochloric acid to give 0.44 g (64.0%) of 1-(2-carboxyethyl)-2-indoloyl-guanidine hydrochloride.
M.P.: 254°C
¹H NMR (DMSO-d₆) δ: 2.72 (2H, t, J=7.3Hz), 4.76 (2H, t, J=7.4Hz), 7.17 (1H, t, J=7.9Hz), 7.40 (1H, ddd, J=1.0, 6.9, 7.4Hz), 7.68 (1H, d, J=8.6Hz), 7.73 (1H, d, J=7.9Hz), 7.91 (1H, s), 8.50 (2H, br-s), 8.72 (2H, br-s), 12.22 (1.5H, br-s).

### Example 130

### Preparation of 7-carboxymethoxy-4-chloro-1-methyl-2-indoloylguanidine

A suspension of 0.40 g (1.11 mmol) of 7-carbamoylmethoxy-4-chloro-1-methyl-2-indoloylguanidine obtained in Example 61 in 100 ml of 2N hydrochloric acid was refluxed for an hour. The reaction mixture was gradually cooled. The precipitated crystals were filtered and dried under reduced pressure to give 0.39 g (97.2%) of 7-carboxymethoxy-4-chloro-1-methyl-2-indoloylguanidine hydrochloride.
M.P.: 283-284°C
¹H NMR (DMSO-d₆) δ: 4.34 (3H, s), 4.84 (2H, s), 6.82 (1H, d, J=8.3Hz), 7.09 (1H, d, J=8.3Hz), 7.69 (1H, s), 8.48 (4H, br-s), 11.5-13.5 (1.3H, br-s).

The following compounds of Examples 131 and 132 were prepared in a manner similar to Example 130.

### Example 131

### 7-Carboxymethoxy-1-methyl-2-indoloylguanidine hydrochloride:

Yield: 41.5%, M.P.: 264°C
¹H NMR (DMSO-d₆) δ: 4.34 (3H, s), 4.84 (2H, s), 6.83 (1H, d, J=7.6Hz), 7.03 (1H, t, J=7.9Hz), 7.30 (1H, d, J=7.9Hz), 7.74 (1H, s), 8.47-8.63 (4H, m), 11.71-12.07 (1H, m), 12.6-13.3 (1H, m).

### Example 132

### 6-Carboxymethoxy-1-methyl-2-indoloylguanidine hydrochloride:

Yield: 53.0%, M.P.: 298°C
¹H NMR (DMSO-d₆) δ: 3.97 (3H, s), 4.79 (2H, s), 6.85 (1H, dd, J=2.0, 8.9Hz), 7.09 (1H, s), 7.63 (1H, t, J=8.9Hz), 7.72 (1H, s), 8.34-8.51 (4H, m), 10-13 (2H, m).

### Example 133

### Preparation of 1-methyl-7-(2-phenylethylamino)-2-indoloyl-guanidine

### a) Preparation of ethyl 1-methyl-7-(2-phenylethylamino)-2-indolecarboxylate

A mixture of 0.10 g (0.46 mmol) of ethyl 7-amino-1-methyl-2-indolecarboxylate, 0.12 g (0.50 mmol) of phenylacetaldehyde as 50% isopropanol solution, 0.043 g (0.69 mmol) of sodium cyanogen borohydride and 0.1 ml of acetic acid in 5 ml of acetonitrile was stirred at room temperature for 15 minutes. Thereafter 0.2 ml of acetic acid was added to the reaction mixture. The resulting mixture was allowed to stand at room temperature for 15 hours. After 1N sodium hydroxide solution was added to the reaction mixture, the mixture was extracted three times with diethyl ether. The combined extracts were then washed with 1N potassium hydroxide solution. After drying over anhydrous magnesium sulfate, the solvent was distilled off under reduced pressure. The residue was purified by silica gel column chromatography to give 0.045 g (30.5%) of ethyl 1-methyl-7-(2-phenylethylamino)-2-indole-carboxylate.

### b) Preparation of 1-methyl-7-(2-phenylethylamino)-2-indoloylguanidine

A mixture of 0.16 g (0.51 mmol) of ethyl 1-methyl-7-(2-phenylethylamino)-2-indolecarboxylate, 0.49 g (5.09 mmol) of guanidine hydrochloride and 0.28 g (5.09 mmol) of sodium methoxide in 10 ml of methanol was reacted in a manner similar to Example 1 to give 0.075 g (39.5%) of 1-methyl-7-(2-phenylethylamino)-2-indoloylguanidine hydrochloride.
M.P.: 220-223°C
¹H NMR (DMSO-d₆) δ: 2.96-3.02 (2H, m), 3.29-3.35 (2H, m), 4.18 (3H, s), 6.60-6.95 (1H, m), 6.99 (1H, d, J=7.3Hz), 7.06 (1H, d, J=7.3Hz), 7.20-7.25 (1H, m), 7.31-7.33 (4H, m), 7.64 (1H, s), 8.42-8.59 (4H, m), 11.73 (1H, br-s).

The reaction was carried out in a manner similar to Example 133 to prepare the compound of Example 134.

### Example 134

### 1-Methyl-6-(2-phenylethylamino)-2-indoloylguanidine hydrochloride:

Yield: 26.6%, M.P.: 243-246°C
¹H NMR (DMSO-d₆) δ: 2.91-2.97 (2H, m), 3.38-3.51 (2H, m), 3.92 (3H, s), 6.70 (1H, s), 6.79 (1H, d, J=7.9Hz), 7.20-7.29 (1H, m), 7.31 (4H, m), 7.49 (1H, d, J=8.6Hz), 7.76 (1H, s), 8.35-8.63 (4H, m), 11.64 (1H, br-s).

### Reference Example 18

### Preparation of 1-(3-dimethylaminopropyl)-4-trifluoromethyl-2-indoloylguanidine

### a) Preparation of ethyl 1-(3-dimethyaminopropyl)-4-trifluoromethyl-2-indolecarboxylate

Ethyl 1-(3-dimethylaminopropyl)-4-trifluoromethyl-2-indolecarboxylate was obtained in an amount of 1.77 g (74%) in a manner similar to Reference Example 5 except for using 2.07 g (4.84 mmol) of ethyl 4-trifluoromethyl-2-indolecarboxylate, 0.43 g (10.7 mmol) of 60% sodium hydride, 1.15 g (7.26 mmol) of 3-chloropropyldimethylamine hydrochloride and 80 ml of dimethylformamide.

### b) Preparation of 1-(3-dimethylaminopropyl)-4-trifluoromethyl-2-indoloylguanidine

1-(3-Dimethylaminopropyl-4-trifluoromethyl-2-indoloylguanidine hydrochloride was obtained in an amount of 0.42 g (28%) in a manner similar to Example 1 except for using 1.77 g (3.45 mmol) of ethyl 1-(3-dimethylaminopropyl)-4-trifluoromethyl-2-indole-carboxylate, 3.30 g (34.5 mmol) of guanidine hydrochloride and 100 ml of a methanol solution of 1.87 g (34.5 mmol) of sodium methoxide.
M.P.: 252-255°C
¹H NMR (DMSO-d₆) δ: 2.07-2.30 (2H, m), 2.58-2.60 (6H, m), 3.00-3.19 (2H, m), 4.59-4.81 (2H, m), 7.49-8.67 (2H, m), 8.04-8.26 (2H, m), 8.71 (2H, br-s), 8.79 (2H, br-s), 10.69 (1H, br-s), 12.29 (1H, br-s).

The following compounds of Examples 135 to 139 were prepared in a manner similar to Reference Example 18.

### Example 135

### 1-(3-Dimethylaminopropyl)-2-indoloylguanidine hydrochloride:

Yield: 12.3%, M.P.: 240°C
¹H NMR (DMSO-d₆) δ: 2.04-2.27 (2H, m), 2.60-2.78 (6H, m), 2.98-3.17 (2H, m), 4.51-4.72 (2H, m), 7.12-7.28 (1H, m), 7.37-7.49 (1H, m), 7.75 (1H, d, J=8.3Hz), 8.07 (1H, s), 8.60 (2H, br-s), 8.81 (2H, br-s), 10.50 (1H, br-s), 12.15 (1H, br-s).

### Example 136

### 4-Chloro-1-(3-dimethylaminopropyl)-2-indoloylguanidine hydrochloride:

Yield: 47.6%, M.P.: 237-240°C
¹H NMR (DMSO-d₆) δ: 2.07-2.27 (2H, m), 2.70 (6H, d, J=1.3Hz), 3.02-3.14 (2H, m), 4.55-4.72 (2H, m), 7.30 (1H, d, J=7.3Hz), 7.38-7.48 (1H, m), 7.77 (1H, d, J=8.6Hz), 8.06 (1H, s), 8.61 (2H, br-s), 8.68 (2H, br-s), 10.36 (1H, br-s), 12.11 (1H, br-s).

### Example 137

### 1-[2-[(N-Pyrrolidinyl)ethyl]-2-indoloylguanidine hydrochloride:

Yield: 23.8%, M.P.: 236-239°C
¹H NMR (DMSO-d₆) δ: 1.75-2.11 (4H, m), 2.88-3.13 (2H, m), 3.40-3.68 (4H, m), 4.85-5.04 (2H, m), 7.16-7.29 (1H, m), 7.40-7.54 (1H, m), 7.78 (1H, d, J=7.9Hz), 7.87 (1H, d, J=7.9Hz), 8.10 (1H, s), 8.62 (2H, br-s), 8.81 (2H, br-s), 11.17 (1H, br-s), 12.24 (1H, br-s).

### Example 138

### 4-Chloro-1-[2-(N-pyrrolidinyl)ethyl]-2-indoloylguanidine hydrochloride:

Yield: 6.1%, M.P.: 220°C
¹H NMR (DMSO-d₆) δ: 1.72-2.10 (4H, m), 2.83-3.13 (2H, m), 3.41-3.69 (4H, m), 4.86-5.05 (2H, m), 7.32 (1H, d, J=7.7Hz), 7.45 (1H, dd, J=8.3, 7.7Hz), 7.89 (1H, d, J=8.3Hz), 8.14 (1H, br-s), 8.67 (2H, br-s), 8.74 (2H, br-s), 11.35 (1H, br-s), 12.28 (1H, br-s).

### Example 139

### 1-[2-(N-Morpholinyl)ethyl]-2-indoloylguanidine hydrochloride:

Yield: 20.5%, M.P.: 180°C
¹H NMR (DMSO-d₆) δ: 3.00-3.27 (2H, m), 3.27-3.70 (4H, m), 3.70-4.10 (4H, m), 4.88-5.14 (2H, m), 7.15-7.30 (1H, m), 7.39-7.52 (1H, m), 7.78 (1H, d, J=7.9Hz), 7.90 (1H, d, J=8.9Hz), 8.10 (1H, s), 8.65 (2H, br-s), 8.81 (2H, br-s), 11.85 (1H, br-s), 12.26 (1H, br-s).

### Example 140

### Preparation of 6-(3-aminopropoxy)-1-methyl-4-trifluoromethyl-2-indoloylguanidine

### a) Preparation of ethyl 6-benzyloxy-1-methyl-4-trifluoromethyl-2-indolecarboxylate

Ethyl 6-benzyloxy-1-methyl-4-trifluoromethyl-2-indolecarboxylate was obtained in an amount of 2.25 g in a manner similar to Reference Example 5 except for using 2.20 g (6.06 mmol) of ethyl 6-benzyloxy-4-trifluoromethyl-2-indolecarboxylate, 0.24 g (6.06 mmol) of 60% sodium hydride, 1.72 g (12.1 mmol) of methyl iodide and 50 ml of dimethylformamide.

### b) Preparation of ethyl 6-hydroxy-1-methyl-4-trifluoromethyl-2-indolecarboxylate

The reaction was carried out in a manner similar to Reference Example 15 a) except for using 2.23 g (5.91 mmol) of ethyl 6-benzyloxy-1-methyl-4-trifluoromethyl-2-indolecarboxylate, 0.3 g of 10% palladium/carbon and 50 ml of tetrahydrofuran. Ethyl 6-hydroxy-1-methyl-4-trifluoromethyl-2-indolecarboxylate was thus obtained in an amount of 1.70 g.

### c) Preparation of ethyl 6-(3-tert-butoxycarbonylaminopropoxy)-1-methyl-4-trifluoromethyl-2-indolecarboxylate

The reaction was carried out in a manner similar to Reference Example 5 except for using 1.00 g (3.48 mmol) of ethyl 6-hydroxy-1-methyl-4-trifluoro-methyl-2-indolecarboxylate, 0.14 g (3.48 mmol) of 60% sodium hydride, 0.99 g (3.48 mmol) of tert-butyl N-(3-iodopropyl)carbamate and 40 ml of dimethylformamide. Ethyl 6-(3-tert-butoxycarbonylaminopropoxy)-1-methyl-4-trifluoromethyl-2-indolecarboxylate was thus obtained in an amount of 1.28 g.

### d) Preparation of 6-(3-aminopropoxy)-1-methyl-4-trifluoromethyl-2-indoloylguanidine

The reaction was carried out in a manner similar to Example 1 except for using 1.28 g (2.88 mmol) of ethyl 6-(3-tert-butoxycarbonylaminopropoxy)-1-methyl-4-trifluoro-methyl-2-indolecarboxylate, 5.50 g (57.6 mmol) of guanidine hydrochloride and 60 ml of methanol solution of 3.11 g (57.6 mmol) of sodium methoxide. 6-(3-tert-Butoxycarbonylaminopropoxy)-1-methyl-4-trifluoromethyl-2-indoloylguanidine was thus obtained in an amount of 0.41 g. This compound, 0.41 g, was treated in a manner similar to Example 114 to give 0.27 g (21.8%) of 6-(3-aminopropoxy)-1-methyl-4-trifluoro-methyl-2-indoloylguanidine hydrochloride.
M.P.: 272-274°C
¹H NMR (DMSO-d₆) δ: 2.08-2.13 (2H, m), 2.99-3.01 (2H, m), 4.05 (3H, s), 4.24-4.28 (2H, m), 7.21 (1H, s), 7.48 (1H, s), 7.97 (1H, s), 8.07 (3H, br-s), 8.56-8.70 (4H, m), 12.6 (1H, br-s).

The compound of Example 141 was obtained in a manner similar to Example 140.

### Example 141

### 6-(3-Aminopropoxy)-1,4-dimethyl-2-indoloylguanidine hydrochloride:

M.P.: 265-267°C
¹H NMR (DMSO-d₆) δ: 2.04-2.09 (2H, m), 2.46 (3H, s), 2.96-2.99 (2H, m), 3.98 (3H, s), 4.13-4.18 (2H, m), 6.65 (1H, s), 6.91 (1H, s), 8.00-8.04 (4H, m), 8.44 (2H, br-s), 8.73 (2H, br-s), 11.92 (1H, br-s).

### Example 142

### Preparation of 6-(3-dimethylaminopropoxy)-1-methyl-4-trifluoromethyl-2-indoloylguanidine

### a) Preparation of ethyl 6-(3-dimethylaminopropoxy)-1-methyl-4-trifluoromethyl-2-indolecarboxylate

Ethyl 6-(3-dimethylaminopropoxy)-1-methyl-4-trifluoromethyl-2-indolecarboxylate was obtained in an amount of 0.72 g in a manner similar to Reference Example 4 except for using 1.00 g (3.48 mmol) of ethyl 6-hydroxy-1-methyl-4-trifluoromethyl-2-indole-carboxylate, 0.35 g (8.70 mmol) of 60% sodium hydride, 0.82 g (5.22 mmol) of 3-chloropropyldimethylamine hydrochloride and 40 ml of dimethylformamide.

### b) Preparation of 6-(3-dimethylaminopropoxy)-1-methyl-4-trifluoromethyl-2-indoloylguanidine

The reaction was carried out in a manner similar to Example 1 except for using 0.72 g (1.93 mmol) of ethyl 6-(3-dimethylaminopropoxy)-1-methyl-4-trifluoro-methyl-2-indolecarboxylate, 3.69 g (38.7 mmol) of guanidine hydrochloride and 50 ml of a methanol solution of 2.09 g (38.7 mmol) of sodium methoxide. 6-(3-dimethylaminopropoxy)-1-methyl-4-trifluoromethyl-2-indoloylguanidine was thus obtained in an amount of 0.40 g. This compound, 0.40 g, was treated in a manner similar to Example 1 to give 0.31 g (35.0%) of 6-(3-dimethylaminopropoxy)-1-methyl-4-trifluoro-methyl-2-indoloylguanidine hydrochloride.
M.P.: 264-265°C
¹H NMR (DMSO-d₆) δ: 2.17-2.23 (2H, m), 2.80 (6H, s), 3.2-3.4 (2H, m), 4.06 (3H, s), 4.23-4.27 (2H, m), 7.20 (1H, s), 7.50 (1H, s), 7.88 (1H, s), 8.5-8.7 (4H, m), 10.27 (1H, br-s), 11.90 (1H, br-s).

The compound of Example 143 was obtained in a manner similar to Example 142.

### Example 143

### 1,4-Dimethyl-6-(3-dimethylaminopropoxy)-2-indoloylguanidine hydrochloride:

M.P.: 282-284°C
¹H NMR (DMSO-d₆) δ: 2.14-2.20 (2H, m), 2.46 (3H, s), 2.79-2.80 (6H, m), 3.1-3.3 (2H, m), 3.98 (3H, s), 4.13-4.17 (2H, m), 6.66 (1H, s), 6.93 (1H, s), 7.99 (1H, s), 8.42-8.74 (4H, m), 10.26 (1H, br-s), 11.85 (1H, br-s).

### Experiment 1

### Inhibition of Na⁺/H⁺ exchanger activity in vitro: Method:

The experiment was performed by modifying the method of Yamori et al. described in J. Hypertension, 8, 153 (1990). That is, inhibition of the Na⁺/H⁺ exchanger activity was evaluated by the change in intracellular pH during acid loading, using the vascular smooth muscle cells isolated from the rat thoracic aorta.

### Results:

The results of IC₅₀ for the inhibition of the Na⁺/H⁺ exchanger activity tested are shown in Table 1 below.

**Table 1**

| Compound | IC₅₀ (µM) |
|---|---|
| Compound of Example 1 | 0.058 |
| Compound of Example 8 | 0.05 |
| Compound of Example 22 | 2.1 |
| Compound of Example 52 | 0.02 |
| Compound of Example 113 | 0.01 |
| Dimethyl amiloride for comparison | 0.60 |
| 5-Hexamethylene amiloride for comparison | 0.14 |

### Experiment 2

### Inhibition of Na⁺/H⁺ exchanger activity in vitro

### Method:

The experiment was performed by modifying the method of Mungre et al. described in Exp. Cell Res., 193, 236 (1991). That is, inhibition of the Na⁺/H⁺ exchanger activity was evaluated by the change in cell viability during acid loading, using the vascular smooth muscle cells isolated from the rat thoracic aorta.

### Results:

The compounds of the present invention shown in Examples were evaluated by the minimum effective concentration (MEC) for the inhibition of the Na⁺/H⁺ exchanger activity. The results are shown in Table 2.

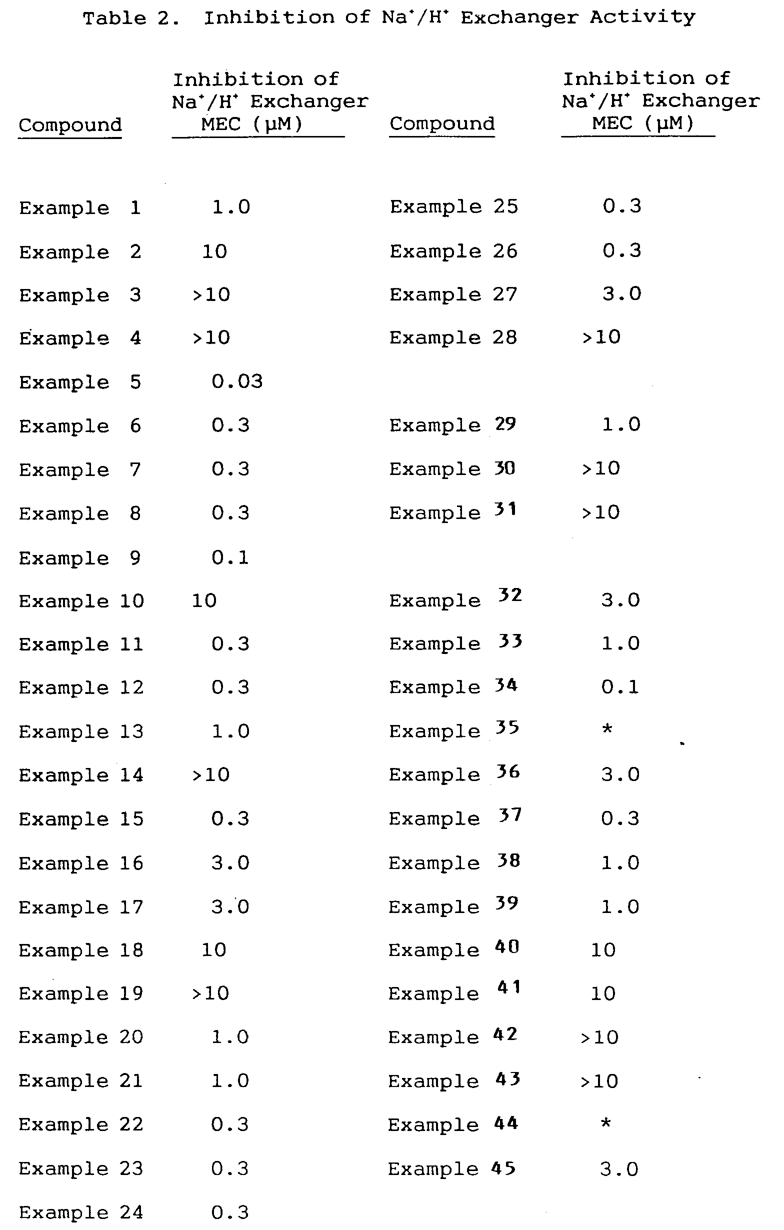

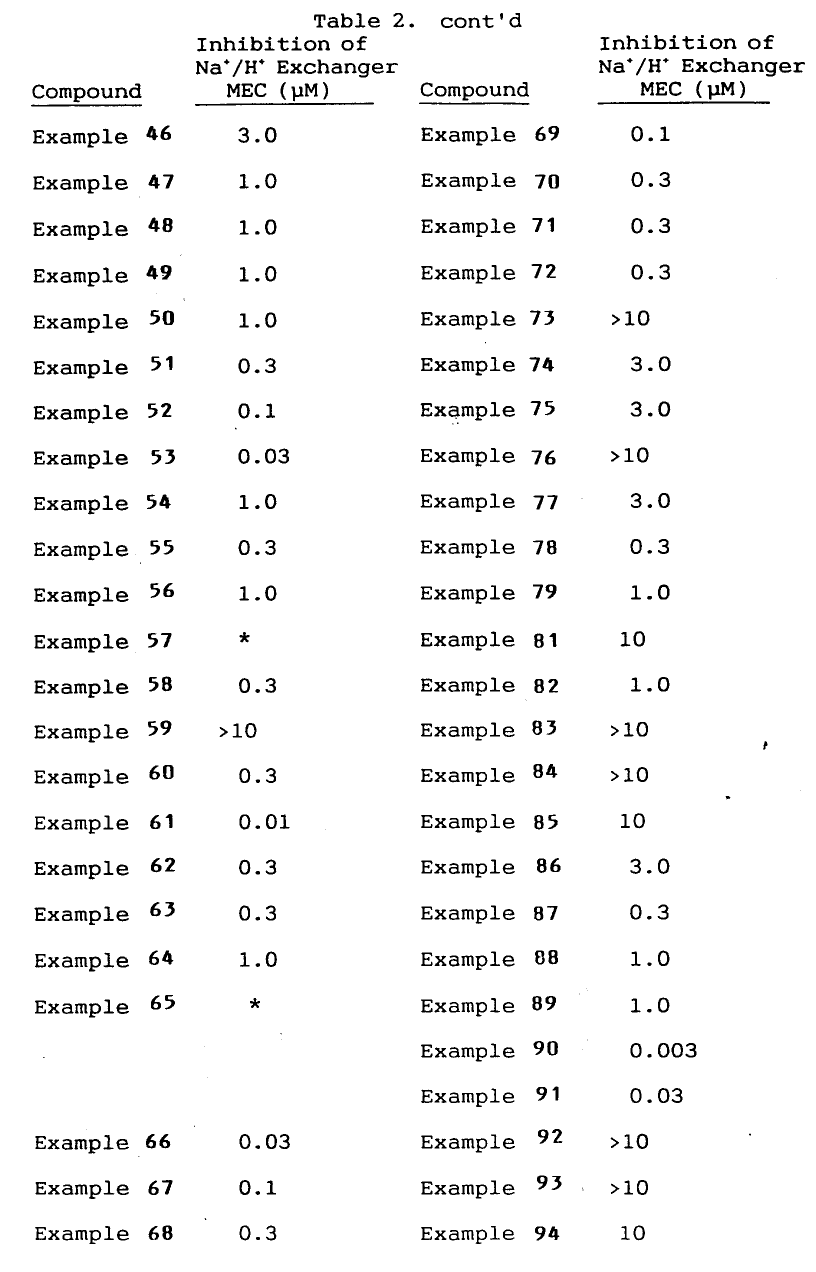

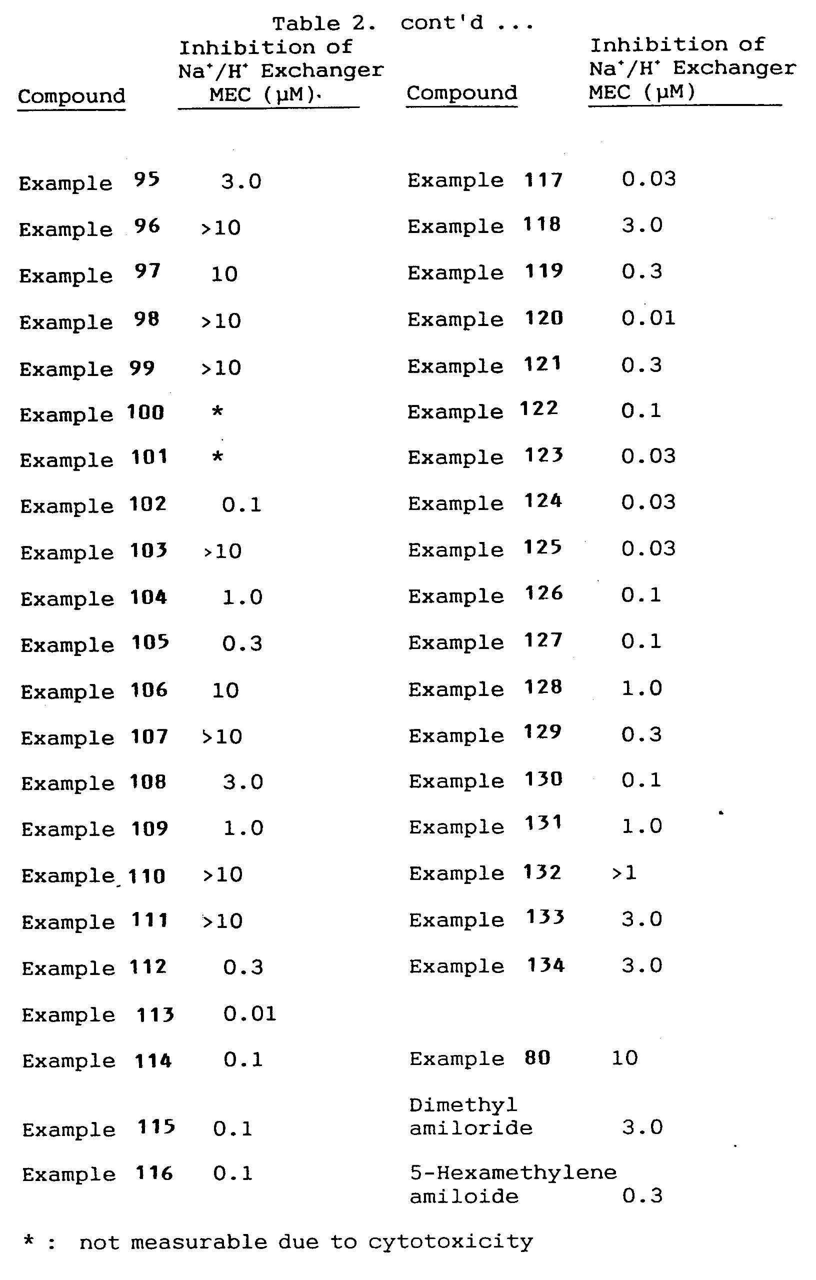

### Experiment 3

### Inhibition of Ischemia- and Reperfusion-induced Arrhythmia in vivo

### Method:

The experiment was performed by modifying the method of Crome et al. described in J. Cardiovasc. Pharmacol., 8, 1249 (1986). That is, the prevention of arrhythmia induced by reperfusion after rat coronary artery occlusion was evaluated by the incidence of ventricular tachycardia and ventricular fibrillation as well as the mortality.

### Results:

The compound of Example 1 in the present invention was evaluated by the method described above, with respect to the incidence of ventricular tachycardia and ventricular fibrillation, and mortality. The results are shown in Table 3 below.

**Table 3**

| Inhibition of Reperfusion-induced Arrhythmia | | | | |
|---|---|---|---|---|
| Compound | Dose (mg/kg) | Incidence of Ventricular Tachycardia (%) | Incidence of Ventricular Fibrillation (%) | Mortality (%) |
| Example 1 | 0.3 | 50 | 0 | 0 |
| | 0.1 | 70 | 10 | 10 |
| EIPA* | 1 | 43 | 0 | 0 |
| | 0.3 | 100 | 56 | 44 |
| Control** | - | 100 | 95 | 76 |

| | | | | |
|---|---|---|---|---|
| *EIPA : 5-N-ethyl-N-isopropyl amiloride | | | | |
| ** Control: untreated | | | | |

The indoloylguanidine derivatives of formula (1) inhibit the Na⁺/H⁺ exchanger activity and are useful for the prevention and treatment of diseases caused by the increased Na⁺/H⁺ exchanger activity, e.g., hypertension, cardiac ischemic reperfusion injury, arrhythmia, cerebral edema, cardiac hypertrophy, vascular lesions, atheroselerosis, etc.

## Claims

1. A compound which is An indoloylguanidine derivative of formula (1): 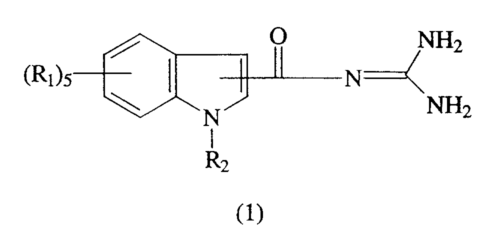 wherein
each R₁, which may be the same or different, is independently selected from hydrogen, an alkyl group which is unsubstituted or substituted by hydroxy, amino, cyano, carboxyl, alkoxycarbonyl, an aromatic group, an alkoxy group, a group -NR₆R₇ or a group -CONR₄R₅ wherein each of R₄ and R₅ independently represents hydrogen or a alkyl group, or R₄ and R₅ are combined together to form a saturated 5- to 7-membered cyclic amino group which may contain other heteroatoms in the ring, an alkenyl group, an alkynyl group, a cycloalkyl group, a halogen, a nitro group, a straight or branched alkanoyl group having up to 8 carbon atoms, an arylalkanoyl group having up to 10 carbon atoms, a carboxyl group, an alkoxycarbonyl group, an aromatic group selected from tolyl, naphthyl and heteroaryl, a group of formula: -OR₃, -NR₆R₇ or -SO₂NR₆R₇, or a group of formula -S(O)ₙR₄₀ selected from ethylsulphonyl, propylsulphonyl, isopropylsulphonyl and the corresponding alkylsulphinyl and alkylthio groups;
R₂ is a hydrogen atom, an alkyl group, a substituted alkyl group, a cycloalkyl group, a hydroxy group, an alkoxy group or a group of formula -CH₂R₂₀;
R₃ is hydrogen, an alkyl group, a substituted alkyl group, a cycloalkyl group or a group shown by formula: -CH₂R₃₀, wherein R₃₀ represents an alkenyl group or an alkynyl group;
each of R₆ and R₇, which may be the same or different, is independently selected from hydrogen, an alkyl group, a substituted alkyl group, a cycloalkyl group, an acyl group and a group of formula:
-CH₂R₆₀, wherein R₆₀ represents an alkenyl group or an alkynyl group; or R₆ and R₇, together with the nitrogen atom to which they are attached, form a saturated 5- to 7-membered cyclic amino group which optionally includes at least one other hetero atom in the ring;
R₄₀ is an alkyl group or a substituted alkyl group; n is 0, 1 or 2;
and
R₂₀ is an alkenyl group or an alkynyl group; and wherein the substituents R₁ and the guanidinocarbonyl group -C(=O)-N=C(NH₂)₂ are each, independently, attached to any one of the 5- and 6- membered rings of the indole nucleus; or 6-(3-aminopropoxy)-1-methyl-4-trifluoromethyl-2-indoloyl guanide, or 6-(3-dimethylaminopropoxy)-1-methyl-4-trifluoromethyl-2-indoloylguanidine; or a pharmaceutically acceptable acid addition salt thereof.

2. A compound according to claim 1, wherein each R₁, which may be the same or different, is independently selected from hydrogen, an alkyl group which is unsubstituted or substituted by hydroxy, amino, cyano, carboxyl, alkoxycarbonyl, an aromatic group, an alkoxy group, a group -NR₆R₇ or a group -CONR₄R₅ wherein each of R₄ and R₅ independently represents hydrogen or an alkyl group, or R₄ and R₅ are combined together to form a saturated 5- to 7-membered cyclic amino group which may contain other heteroatoms in the ring, an alkenyl group, a cycloalkyl group, a halogen, a nitro group, an alkanoyl group, carboxyl group, a tolyl, naphthyl or heteroaryl group, an ethylsulfonyl, propylsulphonyl or isopropylsulphonyl group, and a group of formula -OR₃ or -NR₆R₇;
R₃ is hydrogen, an alkyl group or a substituted alkyl group;
each of R₆ and R₇, which may be the same or different, is independently selected from hydrogen, an alkyl group, a substituted alkyl group, an alkanoyl group or an aroyl group; or R₆ and R₇, together with the nitrogen atom to which they are attached, form a saturated 5- to 7-membered cyclic amino group which optionally includes at least one other hereto atom in the ring.

3. A compound according to claim 1 wherein each R₁, which may be the same or different, is independently selected from hydrogen, an alkyl group, a substituted alkyl group wherein the substituent is a hydroxy group or a group -NR₆R₇ as defined in claim 1, an alkenyl group, a cycloalkyl group, a halogen, a nitro group, an alkanoyl group, a carboxyl group, an ethylsulfonyl, propylsulphonyl or isopropylsulphonyl group or a group of formula -OR₃₁ wherein R₃₁ is hydrogen, an alkyl group, or an alkyl group substituted with a hydroxy group, a carboxyl group, a phenyl group, a carbamoyl group, a mono- or di-alkylcarbamoyl group or a group of formula -NR₆R₇ wherein R₆ and R₇ are as defined in claim 1.

4. A compound according to claim 1 wherein each R₁, which may be the same or different, is independently selected from hydrogen, an alkyl group, an alkenyl group, an alkynyl group, a cycloalkyl group, a halogen, a nitro group, a carboxyl group, an alkoxycarbonyl group and a group of formula -OR₃, -NR₆R₇ or -SO₂NR₆R₇.

5. A compound according to claim 1, wherein each R₁ which may be the same or different, is independently selected from an alkyl group, an alkenyl group, a halogen, a nitro group or a group or formula -OR₃₂ wherein R₃₂ is hydrogen, an alkyl group or an alkyl group substituted by a hydroxy group, a carbamoyl group, a mono- or di-alkylcarbamoyl group or a group of formula: NR₆R₇ wherein R₆ and R₇ are as defined in claim 1.

6. A compound according to any one of the preceding claims wherein R₂ is hydrogen, an alkyl group, a substituted alkyl group, a hydroxy group or an alkoxy group.

7. A compound according to any one of the preceding claims, which is a 2-indoloylguanidine compound.

8. A compound according to claim 1, wherein each R₁, which may be the same or different, is independently selected from:
hydrogen,
an alkyl group having up to 8 carbon atoms,
a cycloalkyl group having 3 to 7 carbon atoms,
a halogen,
a nitro group, and
a group of formula: -OR₃ or -NR₆R₇;
R₂ represents
hydrogen,
an alkyl group having up to 8 carbon atoms, or
a substituted alkyl group having up to 8 carbon atoms in which the substituent is hydroxy, cyano, carboxyl, an alkoxycarbonyl group having up to 6 carbon atoms, an aryl group having up to 10 carbon atoms or carbamoyl;
R₃ represents
hydrogen,
an alkyl group having up to 8 carbon atoms,
a substituted alkyl group having up to 8 carbon atoms in which the substituent is a cycloalkyl group having 3 to 7 carbon atoms, an aryl group having up to 10 carbon atoms, a 5 or 6-membered heteroaryl group containing 1 to 4 nitrogen atoms or a 5 or 6-membered heteroaryl group containing 1 or 2 nitrogen atoms and one oxygen atom or one sulfur atom,
a cycloalkyl group having 3 to 7 carbon atoms, or
a group of formula: -CH₂R₃₀, wherein
R₃₀ represents an alkenyl group having up to 6 carbon atoms or an alkynyl group having 2 to 6 carbon atoms;
each of R₆ and R₇, which may be the same or different, is independently selected from:
hydrogen,
an alkyl group having up to 8 carbon atoms,
a substituted alkyl group having up to 8 carbon atoms in which the substituent is a cycloalkyl group having 3 to 7 carbon atoms, an aryl group having up to 10 carbon atoms, a 5 or 6-membered heteroaryl group containing 1 to 4 nitrogen atoms or a 5 or 6-membered heteroaryl group containing 1 or 2 nitrogen atoms and one oxygen atom or one sulfur atom,
a cycloalkyl group having 3 to 7 carbon atoms,
a group of formula: -CH₂R₆₀, wherein R₆₀ represents an alkenyl group having up to 6 carbon atoms or an alkynyl group having 2 to 6 carbon atoms, or
R₆ and R₇, together with the nitrogen atom to which they are attached, form a saturated 5- to 7-membered cyclic amino group containing 1 to 3 nitrogen atoms or a saturated 5- to 7-membered cyclic amino group containing one nitrogen atom and one oxygen atom;
and wherein the substituents R₁ and the guanidinocarbonyl group -C(=O)-N=C(NH₂)₂ are each, independently, attached to any one of the 5- and 6-membered rings of the indole nucleus.

9. A compound according to claim 1, wherein:
R₁ represents
hydrogen,
an alkyl group having up to 8 carbon atoms,
a halogen, or
a group of formula: -OR₃;
R₃ represents:
hydrogen, or
an alkyl group having up to 8 carbon atoms;
R₂ represents:
hydrogen,
an alkyl group having up to 8 carbon atoms, or
a substituted alkyl group having up to 8 carbon atoms in which the substituent is an aryl group having up to 10 carbon atoms, a 5 or 6-membered hetroaryl group containing 1 to 4 nitrogen atoms, or a 5 or 6-membered heteroaryl group containing 1 or 2 nitrogen atoms and one oxygen atom or one sulfur atom.

10. A compound according to claim 1, wherein R₁ represents hydrogen, methyl or methoxy, and R₂ represents methyl.

11. A compound according to claim 1 which is 1-methyl-2-indoloylguanidine

12. A compound according to claim 1 which is 1,4-dimethyl-2-indoloylguanidine

13. A compound according to claim 8, wherein:
each R₁, which may be identical or different, is independently selected from:
hydrogen,
an alkyl group having up to 8 carbon atoms,
a cycloalkyl group having 3 to 7 carbon atoms,
a halogen,
a nitro group, and
a group of formula: -OR₃, or -NR₆R₇,
R₂ represents:
hydrogen,
an alkyl group having up to 8 carbon atoms, or
a substituted alkyl group having up to 8 carbon atoms in which the substituent is hydroxy, cyano, carboxyl, an alkoxycarbonyl group having up to 6 carbon atoms, an aryl group having up to 10 carbon atoms or carbamoyl;
R₃ represents:
hydrogen,
an alkyl group having up to 8 carbon atoms, or
a cycloalkyl group having 3 to 7 carbon atoms,
each of R₆ and R₇, which may be the same of different, is independently selected from:
hydrogen,
an alkyl group having up to 8 carbon atoms,
a cycloalkyl group having 3 to 7 carbon atoms, and
R₆ and R₇, together with the nitrogen atom to which they are attached, form a saturated 5- to 7-membered cyclic amino group containing 1 to 3 nitrogen atoms or a saturated 5- to 7-membered cyclic amino group containing one nitrogen atom and one oxygen atom.

14. An indoloylguanidine derivative, according to claim 1, of formula (2): 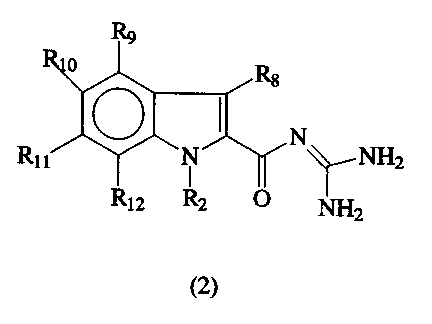 wherein
each of R₈, R₉, R₁₀, R₁₁, and R₁₂, which may be the same or different, is independently selected from hydrogen, an alkyl group which is unsubstituted or substituted by hydroxy, amino, cyano, carboxyl, alkoxycarbonyl, an aromatic group, an alkoxy group, a group -NR₆R₇ or a group -CONR₄R₅ wherein each of R₄ and R₅ independently represents hydrogen or an alkyl group, or R₄ and R₅ are combined together to form a saturated 5- to 7-membered cyclic amino group which may contain other heteroatoms in the ring, an alkenyl group, an alkynyl group, a cycloalkyl group, a halogen, a nitro group, a straight or branched alkanoyl group having up to 8 carbon atoms, an arylalkanoyl group having up to 10 carbon atoms, a carboxyl group, an alkoxycarbonyl group, an aromatic group selected from tolyl, naphthyl and heteroaryl, a group shown of formula: -OR₃, -NR₆R₇ or -SO₂NR₆R₇, or a group of formula -S(O)ₙR₄₀ selected from ethylsulphonyl, propysulphonyl, isopropylsulphony and the corresponding alkylsulphinyl and alkylthio groups;
R₂ is hydrogen, an alkyl group, a substituted alkyl group, a cycloalkyl group, a hydroxy group, an alkoxy group or a group of formula: -CH₂R₂₀
R₃ is hydrogen, an alkyl group, a substituted alkyl group, a cycloalkyl group or a group of formula: -CH₂R₃₀, wherein R₃₀ represents an alkenyl group or an alkynyl group;
each of R₆ and R₇, which may be the same or different, is independently selected from hydrogen, an alkyl group, a substituted alkyl group, a cycloalkyl group, an acyl group and a group of formula: -CH₂R₆₀, wherein R₆₀ is an alkenyl group or an alkynyl group; or R₆ and R₇, together with the nitrogen atom to which are they are attached, form a saturated 5- to 7-membered cyclic amino group which optionally includes at least one other heteroatom in the ring;
R₄₀ is an alkyl group or a substituted alkyl group;
n is 0, 1 or 2; and
R₂₀ is an alkenyl group or an alkynyl group;
or a pharmaceutically acceptable acid addition salt thereof.

15. A compound according to claim 14, wherein R₈ is hydrogen and R₁₀ is hydrogen or a halogen.

16. A compound according to claim 14 or 15, wherein R₉ is hydrogen, an alkyl group, a cycloalkyl, an alkenyl group, a halogen atom, a nitro group, an ethylsulfonyl, propylsulphonyl or isopropylsulphonyl group or a group of formula: -OR₃₃ wherein R₃₃ is hydrogen, an alkyl group or an aralkyl group.

17. A compound according to any one of claims 14 to 16, wherein each of R₁₁ and R₁₂ which may be the same or different, is independently selected from hydrogen, an alkyl group, a substituted alkyl group wherein the substituent is a hydroxy group or a group -NR₆R₇, an alkenyl group, a cycloalkyl group, a halogen, a nitro group, or a group of formula: -OR₃ or -NR₆R₇.

18. A compound according to any one of claims 14 to 17, wherein R₂ is hydrogen, an alkyl group, a substituted alkyl group, a hydroxy group or an alkoxy group.

19. A process for the preparation of an indoloylguanidine derivative, or a salt thereof, as defined in claim 1, the process comprising
(a) reacting a derivative of indolecarboxylic acid of the following formula (3): 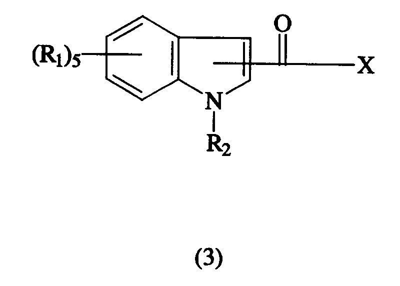 wherein R₁ and R₂ are as defined in claim 1 and X is a leaving group, with guanidine in an inert solvent; or
(b) reacting an indolecarboxylic acid of the following formula (4): 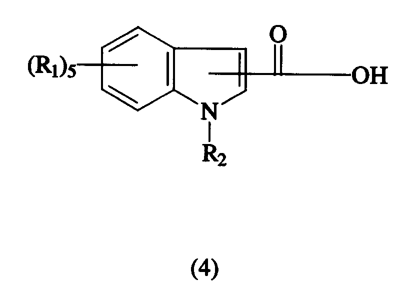 wherein R₁
and R₂ are as defined in claim 1, with guanidine in an inert solvent; or
(c) subjecting a compound of formula (5): 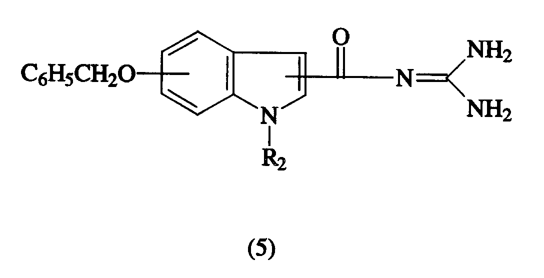 wherein R₂ is as defined in claim 1, to debenzylation, so as to obtain an indoloylguanidine derivative of formula (Ia): 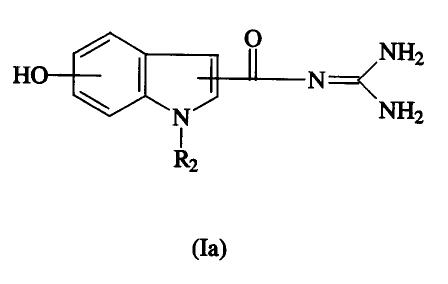 wherein R₂ is as defined in claim 1; or
(d) reducing a compound of formula (6): 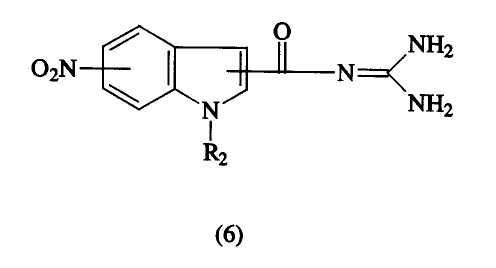 wherein R₂ is as defined in claim 1, so as to obtain an indoloylguanidine derivative of formula (Ib): 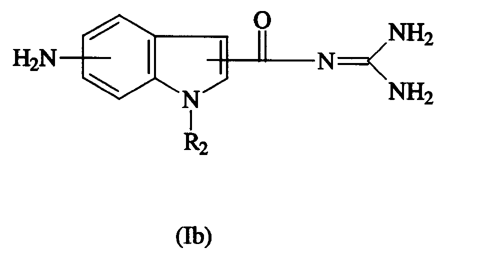 wherein R₂ is as defined in claim 1; and/or, if desired,
(e) converting a compound of formula (I), prepared by any of the above processes (a) to (d), into a pharmaceutically acceptable salt.

20. A pharmaceutical composition which comprises a pharmaceutically acceptable carrier or diluent and, as an active principal, a compound as claimed in any one of claims 1 to 18.

21. A compound as defined in any of claims 1 to 18 for use in a method of treatment of the human or animal body by therapy.

22. Use of a compound as defined in any one of claims 1 to 18 in the manufacture of a medicament for the treatment of a disease caused by increased Na⁺/H⁺ exchanger activity.

## Patentansprüche

1. Verbindung, die ein Indoloylguanidinderivat der Formel (1) ist: 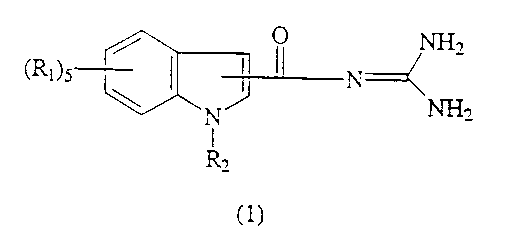 in der
jeder der Reste R₁, die gleich oder verschieden sein können, unabhängig voneinander ausgewählt ist aus einem Wasserstoffatom, einem Alkylrest, der unsubstituiert oder mit einer Hydroxy-, Amino-, Cyano-, Carboxyl- oder Alkoxycarbonylgruppe, einem aromatischen Rest, einem Alkoxyrest, einem Rest -NR₆R₇ oder einem Rest -CONR₄R₅ substituiert ist, bei dem jeder der Reste R₄ und R₅ unabhängig voneinander ein Wasserstoffatom oder einen Alkylrest darstellt oder R₄ und R₅ miteinander verbunden sind, wobei sie einen gesättigten 5- bis 7-gliedrigen cyclischen Aminorest bilden, der weitere Heteroatome im Ring enthalten kann, einem Alkenylrest, einem Alkinylrest, einem Cycloalkylrest, einem Halogenatom, einer Nitrogruppe, einem unverzweigten oder verzweigten Alkanoylrest mit bis zu 8 Kohlenstoffatomen, einem Arylalkanoylrest mit bis zu 10 Kohlenstoffatomen, einer Carboxylgruppe, einem Alkoxycarbonylrest, einem aus einer Tolylgruppe, einer Naphthylgruppe und einem Heteroarylrest ausgewählten aromatischen Rest, einem Rest der Formel: -OR₃, -NR₆R₇ oder -SO₂NR₆R₇, oder einem Rest der Formel -S(O)ₙR₄₀, der aus einer Ethylsulfonyl-, Propylsulfonyl- und Isopropylsulfonylgruppe und den entsprechenden Alkylsulfinyl- und Alkylthioresten ausgewählt ist;
R₂ ein Wasserstoffatom, einen Alkylrest, einen substituierten Alkylrest, einen Cycloalkylrest, eine Hydroxygruppe, einen Alkoxyrest oder einen Rest der Formel -CH₂R₂₀ bedeutet;
R₃ ein Wasserstoffatom, einen Alkylrest, einen substituierten Alkylrest, einen Cycloalkylrest oder einen durch die Formel: -CH₂R₃₀ gezeigten Rest darstellt, in der R₃₀ einen Alkenylrest oder einen Alkinylrest wiedergibt;
jeder der Reste R₆ und R₇, die gleich oder verschieden sein können, unabhängig voneinander aus einem Wasserstoffatom, einem Alkylrest, einem substituierten Alkylrest, einem Cycloalkylrest, einem Acylrest und einem Rest der Formel: -CH₂R₆₀ ausgewählt ist, in der R₆₀ einen Alkenylrest oder einen Alkinylrest wiedergibt, oder R₆ und R₇ zusammen mit dem Stickstoffatom, an das sie gebunden sind, einen gesättigten 5- bis 7-gliedrigen cyclischen Aminorest bilden, der gegebenenfalls mindestens ein weiteres Heteroatom im Ring einschließt;
R₄₀ einen Alkylrest oder einen substituierten Alkylrest darstellt;
n 0, 1 oder 2 ist und
R₂₀ einen Alkenylrest oder einen Alkinylrest bedeutet und in der die Substituenten R₁ und die Guanidinocarbonylgruppe -C(=O)-N=C(NH₂)₂ jeweils unabhängig voneinander an einen der 5- und 6-gliedrigen Ringe des Indolkerns gebunden sind, oder 6-(3-Aminopropoxy)-1-methyl-4-trifluoromethyl-2-indoloylguanidin oder 6-(3-Dimethylaminopropoxy)-1-methyl-4-trifluormethyl-2-indoloylguanidin oder ein pharmazeutisch verträgliches Säureadditionssalz davon.

2. Verbindung nach Anspruch 1, wobei jeder der Reste R₁, die gleich oder verschieden sein können, unabhängig voneinander aus einem Wasserstoffatom, einem Alkylrest, der unsubstituiert oder mit einer Hydroxy-, Amino-, Cyano-, Carboxyl- oder Alkoxycarbonylgruppe, einem aromatischen Rest, einem Alkoxyrest, einem Rest -NR₆R₇ oder einem Rest -CONR₄R₅ substituiert ist, bei dem jeder der Reste R₄ und R₅ unabhängig voneinander ein Wasserstoffatom oder einen Alkylrest wiedergibt oder R₄ und R₅ miteinander verbunden sind, wobei sie einen gesättigten 5- bis 7-gliedrigen cyclischen Aminorest bilden, der weitere Heteroatome im Ring enthalten kann, einem Alkenylrest, einem Cycloakylrest, einem Halogenatom, einer Nitrogruppe, einem Alkanoylrest, einer Carboxylgruppe, einer Tolylgruppe, einer Naphthylgruppe oder einem Heteroarylrest, einer Ethylsulfonyl-, Propylsulfonyl-, Isopropylsulfonylgruppe und einem Rest der Formel -OR₃ oder -NR₆R₇ ausgewählt ist;
R₃ ein Wasserstoffatom, einen Alkylrest oder einen substituierten Alkylrest bedeutet;
jeder der Reste R₆ und R₇, die gleich oder verschieden sein können, unabhängig voneinander aus einem Wasserstoffatom, einem Alkylrest, einem substituierten Alkylrest, einem Alkanoylrest oder einem Aroylrest ausgewählt ist oder R₆ und R₇, zusammen mit dem Stickstoffatom, an das sie gebunden sind, einen gesättigten 5- bis 7-gliedrigen cyclischen Aminorest bilden, der gegebenenfalls mindestens ein weiteres Heteroatom im Ring einschließt.

3. Verbindung nach Anspruch 1, wobei jeder der Reste R₁, die gleich oder verschieden sein können, unabhängig voneinander aus einem Wasserstoffatom, einem Alkylrest, einem substituierten Alkylrest, bei dem der Substituent eine Hydroxygruppe oder einen wie in Anspruch 1 definierten Rest -NR₆R₇ darstellt, einem Alkenylrest, einem Cycloalkylrest, einem Halogenatom, einer Nitrogruppe, einem Alkanoylrest, einer Carboxylgruppe, einer Ethylsulfonyl-, Propylsulfonyl- oder Isopropylsulfonylgruppe oder einem Rest der Formel -OR₃₁ ausgewählt ist, bei dem R₃₁ ein Wasserstoffatom, einen Alkylrest oder einen mit einer Hydroxygruppe, einer Carboxylgruppe, einer Phenylgruppe, einer Carbamoylgruppe, einem Mono- oder Dialkylcarbamoylrest oder einem Rest der Formel -NR₆R₇, bei der R₆ und R₇ wie in Anspruch 1 definiert sind, substituierten Alkylrest bedeutet.

4. Verbindung nach Anspruch 1, wobei jeder der Reste R₁, die gleich oder verschieden sein können, unabhängig voneinander aus einem Wasserstoffatom, einem Alkylrest, einem Alkenylrest, einem Alkinylrest, einem Cycloalkylrest, einem Halogenatom, einer Nitrogruppe, einer Carboxylgruppe, einem Alkoxycarbonylrest und einem Rest der Formel -OR₃, -NR₆R₇ oder -SO₂NR₆R₇ ausgewählt ist.

5. Verbindung nach Anspruch 1, wobei jeder der Reste R₁, die gleich oder verschieden sein können, unabhängig voneinander aus einem Alkylrest, einem Alkenylrest, einem Halogenatom, einer Nitrogruppe oder einem Rest der Formel -OR₃₂ ausgewählt ist, bei der R₃₂ ein Wasserstoffatom, einen Alkylrest oder einen durch eine Hydroxygruppe, eine Carbamoylgruppe, einen Mono- oder Dialkylcarbamoylrest oder einen Rest der Formel: NR₆R₇, bei der R₆ und R₇ wie in Anspruch 1 definiert sind, substituierten Alkylrest bedeutet.

6. Verbindung nach einem der vorstehenden Ansprüche, wobei R₂ ein Wasserstoffatom, einen Alkylrest, einen substituierten Alkylrest, eine Hydroxygruppe oder einen Alkoxyrest darstellt.

7. Verbindung nach einem der vorstehenden Ansprüche, nämlich eine 2-Indoloylguanidinverbindung.

8. Verbindung nach Anspruch 1, wobei jeder der Reste R₁, die gleich oder verschieden sein können, unabhängig voneinander aus:
einem Wasserstoffatom,
einem Alkylrest mit bis zu 8 Kohlenstoffatomen,
einem Cycloalkylrest mit 3 bis 7 Kohlenstoffatomen,
einem Halogenatom,
einer Nitrogruppe und
einem Rest der Formel: -OR₃ oder -NR₆R₇ ausgewählt ist;
R₂
ein Wasserstoffatom,
einen Alkylrest mit bis zu 8 Kohlenstoffatomen oder
einen substituierten Alkylrest mit bis zu 8 Kohlenstoffatomen darstellt, bei dem der Substituent eine Hydroxygruppe, eine Cyanogruppe, eine Carboxylgruppe, einen Alkoxycarbonylrest mit bis zu 6 Kohlenstoffatomen, einen Arylrest mit bis zu 10 Kohlenstoffatomen oder eine Carbamoylgruppe bedeutet;
R₃
ein Wasserstoffatom,
einen Alkylrest mit bis zu 8 Kohlenstoffatomen,
einen substituierten Alkylrest mit bis zu 8 Kohlenstoffatomen, bei dem der Substituent einen Cycloalkylrest mit 3 bis 7 Kohlenstoffatomen, einen Arylrest mit bis zu 10 Kohlenstoffatomen, einen 5- oder 6-gliedrigen Heteroarylrest, der 1 bis 4 Stickstoffatome enthält, oder einen 5- oder 6-gliedrigen Heteroarylrest, der 1 oder 2 Stickstoffatome und ein Sauerstoffatom oder ein Schwefelatom enthält, bedeutet,
einen Cycloalkylrest mit 3 bis 7 Kohlenstoffatomen oder
einen Rest der Formel: -CH₂R₃₀, bei der
R₃₀ einen Alkenylrest mit bis zu 6 Kohlenstoffatomen oder einen Alkinylrest mit 2 bis 6 Kohlenstoffatomen darstellt, wiedergibt;
jeder der Reste R₆ und R₇, die gleich oder verschieden sein können, unabhängig voneinander aus:
einem Wasserstoffatom,
einem Alkylrest mit bis zu 8 Kohlenstoffatomen,
einem substituierten Alkylrest mit bis zu 8 Kohlenstoffatomen, bei dem der Substituent einen Cycloalkylrest mit 3 bis 7 Kohlenstoffatomen, einen Arylrest mit bis zu 10 Kohlenstoffatomen, einen 5- oder 6-gliedrigen Heteroarylrest, der 1 bis 4 Stickstoffatome enthält, oder einen 5- oder 6-gliedrigen Heteroarylrest, der 1 oder 2 Stickstoffatome und ein Sauerstoffatom oder ein Schwefelatom enthält, bedeutet,
einem Cycloalkylrest mit 3 bis 7 Kohlenstoffatomen,
einem Rest der Formel: -CH₂R₆₀ ausgewählt ist, wobei R₆₀ einen Alkenylrest mit bis zu 6 Kohlenstoffatomen oder einen Alkinylrest mit 2 bis 6 Kohlenstoffatomen darstellt, oder
R₆ und R₇ zusammen mit dem Stickstoffatom, an das sie gebunden sind, einen gesättigten 5- bis 7-gliedrigen cyclischen Aminorest, der 1 bis 3 Stickstoffatome enthält, oder einen gesättigten 5- bis 7-gliedrigen cyclischen Aminorest, der ein Stickstoffatom und ein Sauerstoffatom enthält, bilden;
und wobei die Substituenten R₁ und die Guanidinocarbonylgruppe -C(=O)-N=C(NH₂)₂ jeweils unabhängig voneinander an einen der 5- und 6-gliedrigen Ringe des Indolkerns gebunden sind.

9. Verbindung nach Anspruch 1, wobei:
R₁
ein Wasserstoffatom,
einen Alkylrest mit bis zu 8 Kohlenstoffatomen,
ein Halogenatom oder
einen Rest der Formel: -OR₃ darstellt;
R₃
ein Wasserstoffatom oder
einen Alkylrest mit bis zu 8 Kohlenstoffatomen bedeutet;
R₂
ein Wasserstoffatom,
einen Alkylrest mit bis zu 8 Kohlenstoffatomen oder
einen substituierten Alkylrest mit bis zu 8 Kohlenstoffatomen darstellt, bei dem der Substituent einen Arylrest mit bis zu 10 Kohlenstoffatomen, einen 5- oder 6-gliedrigen Heteroarylrest, der 1 bis 4 Stickstoffatome enthält, oder einen 5- oder 6-gliedrigen Heteroarylrest, der 1 oder 2 Stickstoffatome und ein Sauerstoffatom oder ein Schwefelatom enthält, bedeutet.

10. Verbindung nach Anspruch 1, wobei R₁ ein Wasserstoffatom, eine Methyl- oder Methoxygruppe darstellt und R₂ eine Methylgruppe bedeutet.

11. Verbindung nach Anspruch 1, nämlich 1-Methyl-2-indoloylguanidin.

12. Verbindung nach Anspruch 1, nämlich 1,4-Dimethyl-2-indoloylguanidin.

13. Verbindung nach Anspruch 8, wobei:
jeder der Reste R₁, die identisch oder verschieden sein können, unabhängig voneinander aus:
einem Wasserstoffatom,
einem Alkylrest mit bis zu 8 Kohlenstoffatomen,
einem Cycloalkylrest mit 3 bis 7 Kohlenstoffatomen,
einem Halogenatom,
einer Nitrogruppe und
einem Rest der Formel: -OR₃ oder -NR₆R₇ ausgewählt ist;
R₂
ein Wasserstoffatom,
einen Alkylrest mit bis zu 8 Kohlenstoffatomen oder
einen substituierten Alkylrest mit bis zu 8 Kohlenstoffatomen darstellt, bei dem der Substituent eine Hydroxygruppe, eine Cyanogruppe, eine Carboxylgruppe, einen Alkoxycarbonylrest mit bis zu 6 Kohlenstoffatomen, einen Arylrest mit bis zu 10 Kohlenstoffatomen oder eine Carbamoylgruppe bedeutet;
R₃
ein Wasserstoffatom,
einen Alkylrest mit bis zu 8 Kohlenstoffatomen oder
einen Cycloalkylrest mit 3 bis 7 Kohlenstoffatomen darstellt,
jeder der Reste R₆ und R₇, die gleich oder verschieden sein können, unabhängig voneinander aus:
einem Wasserstoffatom,
einem Alkylrest mit bis zu 8 Kohlenstoffatomen,
einem Cycloalkylrest mit 3 bis 7 Kohlenstoffatomen ausgewählt ist und
R₆ und R₇, zusammen mit dem Stickstoffatom, an das sie gebunden sind, einen gesättigten 5- bis 7-gliedrigen cyclischen Aminorest, der 1 bis 3 Stickstoffatome enthält, oder einen gesättigten 5- bis 7-gliedrigen cyclischen Aminorest, der ein Stickstoffatom und ein Sauerstoffatom enthält, bilden.

14. Indoloylguanidinderivat nach Anspruch 1 der Formel (2): 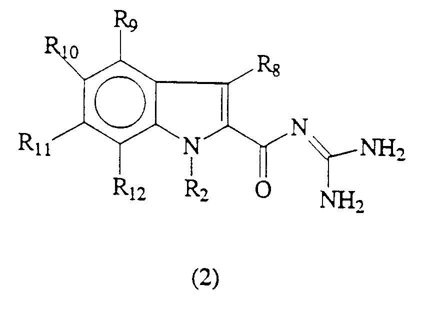 in der
jeder der Reste R₈, R₉, R₁₀, R₁₁, und R₁₂, die gleich oder verschieden sein können, unabhängig voneinander aus einem Wasserstoffatom, einem Alkylrest, der unsubstituiert oder mit einer Hydroxy-, Amino-, Cyano-, Carboxyl- oder Alkoxycarbonylgruppe, einem aromatischen Rest, einem Alkoxyrest, einem Rest -NR₆R₇ oder einem Rest -CONR₄R₅ substituiert ist, bei dem jeder der Reste R₄ und R₅ unabhängig voneinander ein Wasserstoffatom oder einen Alkylrest wiedergibt oder R₄ und R₅ miteinander verbunden sind, wobei sie einen gesättigten 5- bis 7-gliedrigen cyclischen Aminorest bilden, der weitere Heteroatome im Ring enthalten kann, einem Alkenylrest, einem Alkinylrest, einem Cycloalkylrest, einem Halogenatom, einer Nitrogruppe, einem unverzweigten oder verzweigten Alkanoylrest mit bis zu 8 Kohlenstoffatomen, einem Arylalkanoylrest mit bis zu 10 Kohlenstoffatomen, einer Carboxylgruppe, einem Alkoxycarbonylrest, einem aus einer Tolylgruppe, einer Naphthylgruppe und einem Heteroarylrest ausgewählten aromatischen Rest, einem durch die Formel: -OR₃, -NR₆R₇ oder -SO₂NR₆R₇ gezeigten Rest oder einem Rest der Formel -S(O)ₙR₄₀ ausgewählt ist, der aus einer Ethylsulfonyl-, Propylsufonyl-, Isopropylsulfonylgruppe und den entsprechenden Alkylsulfinyl- und Alkylthioresten ausgewählt ist;
R₂ ein Wasserstoffatom, einen Alkylrest, einen substituierten Alkylrest, einen Cycloalkylrest, eine Hydroxygruppe, einen Alkoxyrest oder einen Rest der Formel -CH₂R₂₀ darstellt;
R₃ ein Wasserstoffatom, einen Alkylrest, einen substituierten Alkylrest, einen Cycloalkylrest oder einen durch die Formel: -CH₂R₃₀ gezeigten Rest bedeutet, wobei R₃₀ einen Alkenylrest oder einen Alkinylrest wiedergibt;
jeder der Reste R₆ und R₇, die gleich oder verschieden sein können, unabhängig voneinander aus einem Wasserstoffatom, einem Alkylrest, einem substituierten Alkylrest, einem Cycloalkylrest, einem Acylrest und einem Rest der Formel: -CH₂R₆₀ ausgewählt ist, wobei R₆₀ einen Alkenylrest oder einen Alkinylrest bedeutet, oder R₆ und R₇ zusammen mit dem Stickstoffatom, an das sie gebunden sind, einen gesättigten 5- bis 7-gliedrigen cyclischen Aminorest bilden, der gegebenenfalls mindestens ein weiteres Heteroatom im Ring einschließt;
R₄₀ einen Alkylrest oder einen substituierten Alkylrest darstellt;
n 0, 1 oder 2 ist und
R₂₀ einen Alkenylrest oder einen Alkinylrest bedeutet,
oder ein pharmazeutisch verträgliches Säureadditionssalz davon.

15. Verbindung nach Anspruch 14, wobei R₈ ein Wasserstoffatom darstellt und R₁₀ ein Wasserstoff- oder Halogenatom bedeutet.

16. Verbindung nach Anspruch 14 oder 15, wobei R₉ ein Wasserstoffatom, einen Alkylrest, einen Cycloalkylrest, einen Alkenylrest, ein Halogenatom, eine Nitrogruppe, eine Ethylsulfonyl-, Propylsulfonyl- oder Isopropylsulfonylgruppe oder einen Rest der Formel: -OR₃₃ darstellt, wobei R₃₃ ein Wasserstoffatom, einen Alkylrest oder einen Aralkylrest bedeutet.

17. Verbindung nach einem der Ansprüche 14 bis 16, wobei jeder der Reste R₁₁ und R₁₂, die gleich oder verschieden sein können, unabhängig voneinander aus einem Wasserstoffatom, einem Alkylrest, einem substituierten Alkylrest, bei dem der Substituent eine Hydroxygruppe oder einen Rest -NR₆R₇ darstellt, einem Alkenylrest, einem Cycloalkylrest, einem Halogenatom, einer Nitrogruppe oder einem Rest der Formel: -OR₃ oder -NR₆R₇ ausgewählt ist.

18. Verbindung nach einem der Ansprüche 14 bis 17, wobei R₂ ein Wasserstoffatom, einen Alkylrest, einen substituierten Alkylrest, eine Hydroxygruppe oder einen Alkoxyrest bedeutet.

19. Verfahren zur Herstellung eines wie in Anspruch 1 definierten Indoloylguanidinderivats oder eines Salzes davon, wobei das Verfahren
(a) Umsetzen eines Derivats der Indolcarbonsäure der folgenden Formel (3): 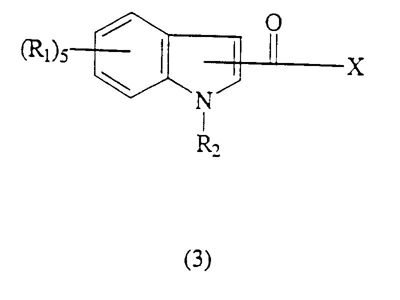 bei der R₁ und R₂ wie in Anspruch 1 definiert sind und X eine Abgangsgruppe bedeutet, mit Guanidin in einem inerten Lösungsmittel oder
(b) Umsetzen einer Indolcarbonsäure der folgenden Formel (4): 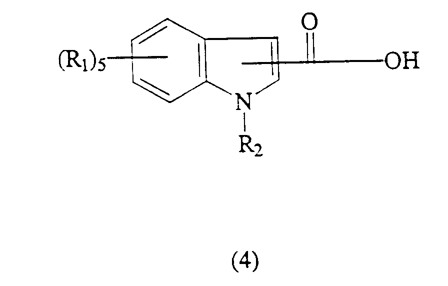 bei der R₁ und R₂ wie in Anspruch 1 definiert sind, mit Guanidin in einem inerten Lösungsmittel oder
(c) Debenzylieren einer Verbindung der Formel (5): 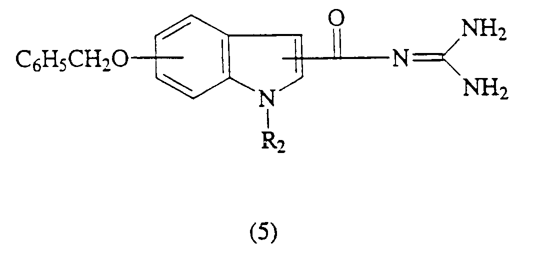 bei der R₂ wie in Anspruch 1 definiert ist, so daß das Indoloylguanidinderivat der Formel (Ia): 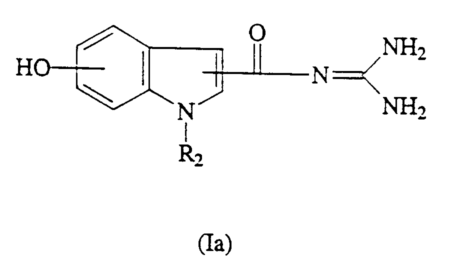 bei der R₂ wie in Anspruch 1 definiert ist, erhalten wird oder
(d) Reduzieren einer Verbindung der Formel (6): 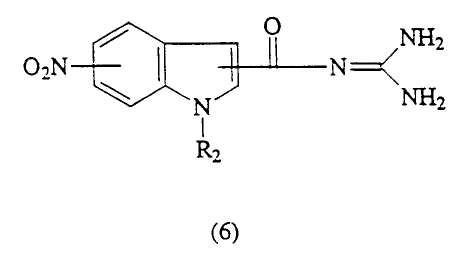 bei der R₂ wie in Anspruch 1 definiert ist, so daß das Indoloylguanidinderivat der Formel (Ib): 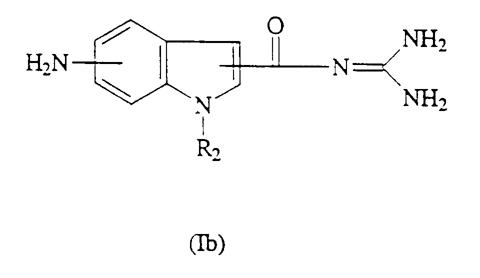 bei der R₂ wie in Anspruch 1 definiert ist, erhalten wird und/oder, falls gewünscht,
(e) Umwandeln der Verbindung der Formel (I), die durch eines der vorstehenden Verfahren (a) bis (d) hergestellt wird, in ein pharmazeutisch verträgliches Salz umfaßt.

20. Arzneimittel, das ein(en) pharmazeutisch verträglichen/s Träger oder Verdünnungsmittel und als wirksamen Grundbestandteil eine Verbindung nach einem der Ansprüche 1 bis 18 umfaßt.

21. Verbindung, wie in einem der Ansprüche 1 bis 18 definiert, zur Verwendung bei einem Verfahren zur Behandlung des menschlichen oder tierischen Körpers durch Therapie.

22. Verwendung einer Verbindung, wie in einem der Ansprüche 1 bis 18 definiert, bei der Herstellung eines Arzneimittels zur Behandlung einer Erkrankung, die durch eine erhöhte Na⁺/H⁺-Austauscheraktivität verursacht wird.

## Revendications

1. Composé qui est un dérivé d'indoloylguanidine de formule I : 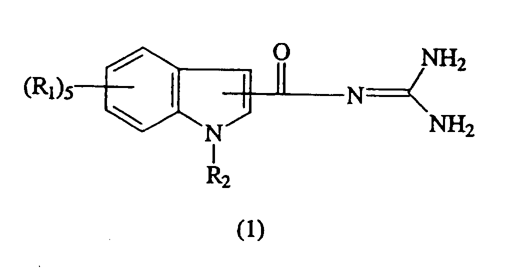 dans laquelle
chacun des groupements R₁, qui peuvent être identiques ou différents, est indépendamment choisi entre un atome d'hydrogène, un groupement alkyle qui est non substitué ou substitué par des radicaux hydroxy, amino, cyano, carboxyle, alcoxycarbonyle, un groupement aromatique, un groupement alcoxy, un groupement -NR₆R₇ ou un groupement
-CONR₄R₅ dans lequel chacun de R₄ et R₅ représente indépendamment un atome d'hydrogène ou un groupement alkyle, ou bien R₄ et R₅ sont combinés ensemble pour former un groupement amino cyclique à 5-7 chaînons saturé dont le noyau peut contenir d'autres hétéroatomes, un groupement alcényle, un groupement alcynyle, un groupement cycloalkyle, un atome d'halogène, un groupement nitro, un groupement alcanoyle linéaire ou ramifié ayant jusqu'à 8 atomes de carbone, un groupement arylalcanoyle ayant jusqu'à 10 atomes de carbone, un groupement carboxy, un groupement alcoxycarbonyle, un groupement aromatique choisi entre les groupements tolyle, naphtyle et hétéroaryle, un groupement de formule : -OR₃, -NR₆R₇ ou -SO₂NR₆R₇, ou un groupement de formule -S(O)ₙR₄₀ choisi parmi les groupements éthylsulfonyle, propylsulfonyle, isopropylsulfonyle et les groupements alkylsulfinyle et alkylthio correspondants ;
R₂ est un atome d'hydrogène, un groupement alkyle, un groupement alkyle substitué, un groupement cycloalkyle, un groupement hydroxy, un groupement alcoxy ou un groupement de formule -CH₂R₂₀ ;
R₃ est un atome d'hydrogène, un groupement alkyle, un groupement alkyle substitué, un groupement cycloalkyle ou un groupement représenté par la formule : -CH₂R₃₀, où R₃₀ représente un groupement alcényle ou un groupement alcynyle ;
chacun de R₆ et R₇, qui peuvent être identiques ou différents, est choisi indépendamment entre un atome d'hydrogène, un groupement alkyle substitué, un groupement cycloalkyle, un groupement acyle et un groupement de formule :
-CH₂R₆₀, où R₆₀ représente un groupement alcényle ou un groupement alcynyle ; ou bien R₆ et R₇, avec l'atome d'azote auquel ils sont fixés, forment un groupement amino cyclique à 5-7 chaînons saturé dont le noyau comprend éventuellement au moins un autre hétéroatome ;
R₄₀ est un groupement alkyle ou un groupement alkyle substitué ; n est 0, 1 ou 2 ;
et
R₂₀ est un groupement alcényle ou un groupement alcynyle ; et dans laquelle les substituants R₁ et le groupement guanidinocarbonyle -C(=O)-N=C(NH₂)₂ sont chacun, indépendamment, fixé à l'un quelconque des cycles à 5 et 6 chaînons du noyau indole ;
ou le 6-(3-aminopropoxy)-1-méthyl-4-trifluorométhyl-2-indoloyl-guanide, ou la 6-(3-diméthylaminopropoxy)-1-méthyl-4-trifluorométhyl-2-indoloylguanidine ;
ou un de leurs sels d'addition d'acide pharmaceutiquement acceptables.

2. Composé selon la revendication 1, dans lequel chacun des groupements R₁, qui peuvent être identiques ou différents, est indépendamment choisi entre un atome d'hydrogène, un groupement alkyle qui est non substitué ou substitué par des radicaux hydroxy, amino, cyano, carboxy, alcoxycarbonyle, un groupement aromatique, un groupement alcoxy, un groupement -NR₆R₇ ou un groupement -CONR₄R₅ où chacun de R₄ ou R₅ représente indépendamment un atome d'hydrogène ou un groupement alkyle, ou bien R₄ et R₅ sont combinés ensemble pour former un groupement amino cyclique à 5-7 chaînons saturé dont le noyau peut contenir d'autres hétéroatomes, un groupement alcényle, un groupement cycloalkyle, un atome d'halogène, un groupement nitro, un groupement alcanoyle, un groupement carboxyle, un groupement tolyle, naphtyle ou hétéroaryle, un groupement éthylsulfonyle, propylsulfonyle ou isopropylsulfonyle, et un groupement de formule -OR₃ ou -NR₆R₇ ;
R₃ est un atome d'hydrogène, un groupement alkyle ou un groupement alkyle substitué ;
chacun de R₆ et R₇, qui peuvent être identiques ou différents, est choisi indépendamment entre un atome d'hydrogène, un groupement alkyle, un groupement alkyle substitué, un groupement alcanoyle ou un groupement aroyle ; ou bien R₆ et R₇, avec l'atome d'azote auquel ils sont attachés, forment un groupement amino cyclique à 5-7 chaînons saturé dont le noyau comprend éventuellement au moins un autre hétéroatome.

3. Composé selon la revendication 1, dans lequel chacun des groupements R₁, qui peuvent être identiques ou différents, est choisi indépendamment entre un atome d'hydrogène, un groupement alkyle, un groupement alkyle substitué dont le substituant est un groupement hydroxy ou un groupement -NR₆R₇ tel que défini dans la revendication 1, un groupement alcényle, un groupement cycloalkyle, un atome d'halogène, un groupement nitro, un groupement alcanoyle, un groupement carboxy, un groupement éthylsulfonyle, propylsulfonyle ou isopropylsulfonyle ou un groupement de formule -OR₃₁ dans laquelle R₃₁ est un atome d'hydrogène, un groupement alkyle, ou un groupement alkyle substitué par un groupement hydroxy, un groupement carboxy, un groupement phényle, un groupement carbamoyle, un groupement mono- ou dialkylcarbamoyle ou un groupement de formule -NR₆R₇ dans laquelle R₆ et R₇ sont tels que définis dans la revendication 1.

4. Composé selon la revendication 1, dans lequel chacun des groupements R₁, qui peuvent être identiques ou différents, est indépendamment choisi entre un atome d'hydrogène, un groupement alkyle, un groupement alcényle, un groupement alcynyle, un groupement cycloalkyle, un atome d'halogène, un groupement nitro, un groupement carboxy, un groupement alcoxycarbonyle et un groupement de formule -OR₃, -NR₆R₇ ou -SO₂NR₆R₇.

5. Composé selon la revendication 1, dans lequel chacun des groupements R₁, qui peuvent être identiques ou différents, est indépendamment choisi entre un groupement alkyle, un groupement alcényle, un atome d'halogène, un groupement nitro ou un groupement de formule -OR₃₂ dans laquelle R₃₂ est un atome d'hydrogène, un groupement alkyle ou un groupement alkyle substitué par un groupement hydroxy, un groupement carbamoyle, un groupement mono- ou dialkylcarbamoyle ou un groupement de formule : -NR₆R₇ dans laquelle R₆ et R₇ sont tels que définis dans la revendication 1.

6. Composé selon l'une quelconque des revendications précédentes, dans lequel R₂ est un atome d'hydrogène, un groupement alkyle, un groupement alkyle substitué, un groupement hydroxy ou un groupement alcoxy.

7. Composé selon l'une quelconque des revendications précédentes, qui est un dérivé de 2-indoloylguanidine.

8. Composé selon la revendication 1, dans lequel chacun des groupements R₁, qui peuvent être identiques ou différents, est indépendamment choisi entre :
un atome d'hydrogène,
un groupement alkyle ayant jusqu'à 8 atomes de carbone,
un groupement cycloalkyle ayant de 3 à 7 atomes de carbone,
un atome d'halogène,
un groupement nitro,
un groupement de formule : -OR₃ ou -NR₆R₇ ;
R₂ représente
un atome d'hydrogène,
un groupement alkyle ayant jusqu'à 8 atomes de carbone, ou
un groupement alkyle substitué ayant jusqu'à 8 atomes de carbone dans lequel le substituant est un groupement hydroxy, cyano, carboxy, un groupement alcoxycarbonyle ayant jusqu'à 6 atomes de carbone, un groupement aryle ayant jusqu'à 10 atomes de carbone ou carbamoyle ;
R₃ représente
un atome d'hydrogène,
un groupement alkyle ayant jusqu'à 8 atomes de carbones dans lequel le substituant est un groupement cycloalkyle ayant 3 à 7 atomes de carbone, un groupement aryle ayant jusqu'à 10 atomes de carbone, un groupement hétéroaryle à 5 ou 6 chaînons contenant de 1 à 4 atomes d'azote ou un groupement hétéroaryle à 5 ou 6 chaînons contenant 1 ou 2 atomes d'azote et un atome d'oxygène ou un atome de soufre,
un groupement cycloalkyle ayant de 3 à 7 atomes de carbone, ou
un groupement de formule : -CH₂R₃₀, dans laquelle
R₃₀ représente un groupement alcényle ayant jusqu'à 6 atomes de carbone ou un groupement alcynyle ayant de 2 à 6 atomes de carbone ;
chacun de R₆ et R₇, qui peuvent être identiques ou différents, est indépendamment choisi entre :
un atome d'hydrogène,
un groupement alkyle ayant jusqu'à 8 atomes de carbone,
un groupement alkyle substitué ayant jusqu'à 8 atomes de carbone dans lequel le substituant est un groupement cycloalkyle ayant de 3 à 7 atomes de carbone, un groupement aryle ayant jusqu'à 10 atomes de carbone, un groupement hétéroaryle à 5 ou 6 chaînons contenant de 1 à 4 atomes d'azote ou un groupement hétéroaryle à 5 ou 6 chaînons contenant 1 ou 2 atomes d'azote et un atome d'oxygène ou un atome de soufre,
un groupement cycloalkyle ayant de 3 à 7 atomes de carbone,
un groupement de formule : -CH₂R₆₀, dans laquelle R₆₀ représente un groupement alcényle ayant jusqu'à 6 atomes de carbone ou un groupement alcynyle ayant de 2 à 6 atomes de carbone, ou
R₆ et R₇, pris avec l'atome d'azote auquel ils sont liés, forment un groupement amino cyclique à 5-7 chaînons saturé contenant 1 à 3 atomes d'azote ou un groupement amino cyclique à 5-7 chaînons contenant un atome d'azote et un atome d'oxygène ;
et dans lequel les substituants R₁ et le groupement guanidinocarbonyle -C(=O)-N=C(NH₂)₂ sont chacun, indépendamment, fixés à l'un quelconque des cycles à 5 et 6 chaînons du noyau indole.

9. Composé selon la revendication 1, dans lequel :
R₁ représente
un atome d'hydrogène,
un groupement alkyle ayant jusqu'à 8 atomes de carbone,
un atome d'halogène, ou
un groupement de formule : -OR₃ ;
R₃ représente :
un atome d'hydrogène, ou
un groupement alkyle ayant jusqu'à 8 atomes de carbone ;
R₂ représente :
un atome d'hydrogène,
un groupement alkyle ayant jusqu'à 8 atomes de carbone, ou
un groupement alkyle substitué ayant jusqu'à 8 atomes de carbone dans lequel le substituant est un groupement aryle ayant jusqu'à 10 atomes de carbone, un groupement hétéroaryle à 5 ou 6 chaînons contenant 1 à 4 atomes d'azote, ou un groupement hétéroaryle à 5 ou 6 chaînons contenant 1 ou 2 atomes d'azote et un atome d'oxygène ou un atome de soufre.

10. Composé selon la revendication 1, dans lequel R₁ représente un atome d'hydrogène, un groupement méthyle ou méthoxy, et R₂ représente un groupement méthyle.

11. Composé selon la revendication 1, qui est la 1-méthyl-2-indoloylguanidine.

12. Composé selon la revendication 1, qui est la 1,4-diméthyl-2-indoloylguanidine.

13. Composé selon la revendication 8, dans lequel :
chacun des groupements R₁ qui peuvent être identiques ou différents, est indépendamment choisi parmi :
un atome d'hydrogène,
un groupement alkyle ayant jusqu'à 8 atomes de carbone,
un groupement cycloalkyle ayant de 3 à 7 atomes de carbone,
un atome d'halogène,
un groupement nitro, et
un groupement de formule : - OR₃, ou NR₆R₇,
R₂ représente :
un atome d'hydrogène,
un groupement alkyle ayant jusqu'à 8 atomes de carbone , ou
un groupement alkyle substitué ayant jusqu'à 8 atomes de carbone dans lequel le substituant est un groupement hydroxy, cyano, carboxy, un groupement alcoxycarbonyle ayant jusqu'à 6 atomes de carbone, un groupement aryle ayant jusqu'à 10 atomes de carbone ou carbamoyle ;
R₃ représente :
un atome d'hydrogène,
un groupement alkyle ayant jusqu'à 8 atomes de carbone , ou
un groupement cycloalkyle ayant 3 à 7 atomes de carbone,
chacun de R₆ et R₇, qui peuvent être identiques ou différents, est indépendamment choisi entre :
un atome d'hydrogène,
un groupement alkyle ayant jusqu'à 8 atomes de carbone,
un groupement cycloalkyle ayant de 3 à 7 atomes de carbone, et
R₆ et R₇, pris avec l'atome d'azote auquel ils sont fixés, forment un groupement amino cyclique, à 5-7 chaînons saturé contenant 1 à 3 atomes d'azote ou un groupement amino cyclique à 5-7 chaînons saturé contenant un atome d'azote et un atome d'oxygène.

14. Dérivé d'indoloylguanidine selon la revendication 1, de formule (2) : 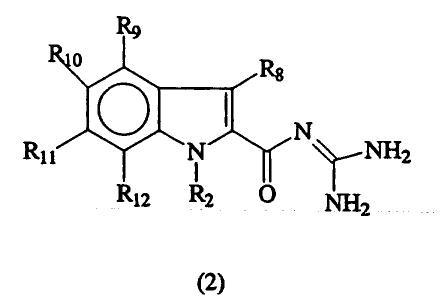 dans laquelle
chacun de R₈, R₉, R₁₀, R₁₁ et R₁₂, qui peuvent être identiques ou différents, est indépendamment choisi entre un atome d'hydrogène, un groupement alkyle qui est non substitué ou substitué par un groupement hydroxy, amino, cyano, carboxy, alcoxycarbonyle, un groupement aromatique, un groupement alcoxy, un groupement -NR₆R₇ ou un groupement -CONR₄R₅ où chacun de R₄ et R₅ représente indépendamment un atome d'hydrogène ou un groupement alkyle, ou bien R₄ et R₅ sont combinés ensemble pour former un groupement amino cyclique à 5-7 chaînons saturé dont le noyau peut contenir d'autres hétéroatomes, un groupement alcényle, un groupement alcynyle, un groupement cycloalkyle, un atome d'halogène, un groupement nitro, un groupement alcanoyle linéaire ou ramifié ayant jusqu'à 8 atomes de carbone, un groupement arylalcanoyle ayant jusqu'à 10 atomes de carbone, un groupement carboxy, un groupement alcoxycarbonyle, un groupement aromatique choisi entre les groupements tolyle, naphtyle et hétéroaryle, un groupement de formule : -OR₃, -NR₆R₇ ou -SO₂NR₆R7, ou un groupement de formule -S(O)ₙR₄₀ choisi entre les groupements éthylsulfonyle, propylsulfonyle, isopropylsulfonyle et les groupements alkylsulfinyle et alkylthio correspondants ;
R₂ est un atome d'hydrogène, un groupement alkyle, un groupement alkyle substitué, un groupement cycloalkyle, un groupement hydroxy, un groupement alcoxy ou un groupement de formule : -CH₂R₂₀
R₃ est un atome d'hydrogène, un groupement alkyle, un groupement alkyle substitué, un groupement cycloalkyle ou un groupement de formule : -CH₂R₃₀, où R₃₀ représente un groupement alcényle ou un groupement alcynyle ;
chacun de R₆ et R₇, qui peuvent être identiques ou différents, est indépendamment choisi entre un atome d'hydrogène, un groupement alkyle, un groupement alkyle substitué, un groupement cycloalkyle, un groupement acyle et un groupement de formule : -CH₂R₆₀, où R₆₀ est un groupement alcényle ou un groupement alcynyle ; ou bien R₆ et R₇, pris avec l'atome d'azote auquel ils sont fixés, forment un groupement amino cyclique à 5-7 chaînons saturé dont le noyau comprend éventuellement au moins un autre hétéroatome ;
R₄₀ est un groupement alkyle ou un groupement alkyle substitué ;
n est 0, 1 ou 2 ; et
R₂₀ est un groupement alcényle ou un groupement alcynyle ; ou un de ses sels d'addition d'acide pharmaceutiquement acceptable.

15. Composé selon la revendication 14, dans lequel R₈ est un atome d'hydrogène et R₁₀ est un atome d'hydrogène ou d'halogène.

16. Composé selon la revendication 14 ou 15, dans lequel R₉ est un atome d'hydrogène, un groupement alkyle, un groupement cycloalkyle, un groupement alcényle, un atome d'halogène, un groupement nitro, un groupement éthylsulfonyle, propylsulfonyle ou isopropylsulfonyle ou un groupement de formule : -OR₃₃ dans laquelle R₃₃ est un atome d'hydrogène ou un groupement alkyle ou aralkyle.

17. Composé selon l'une quelconque des revendications 14 à 16, dans lequel chacun de R₁₁ et R₁₂, qui peuvent être identiques ou différents, est indépendamment choisi entre un atome d'hydrogène, un groupement alkyle, un groupement alkyle substitué dans lequel le substituant est un groupement hydroxy ou un groupement -NR₆R₇, un groupement alcényle, un groupement cycloalkyle, un atome d'halogène, un groupement nitro ou un groupement de formule : -OR₃ ou NR₆R₇.

18. Composé selon l'une quelconque des revendications 14 à 17, dans lequel R₂ est un atome d'hydrogène, un groupement alkyle, un groupement alkyle substitué, un groupement hydroxy ou un groupement alcoxy.

19. Procédé pour la préparation d'un dérivé d'indoloylguanidine, ou d'un de ses sels, tel que défini dans la revendication 1, le procédé comprenant :
a) la réaction d'un dérivé d'acide indolecarboxylique de formule suivante (3) : 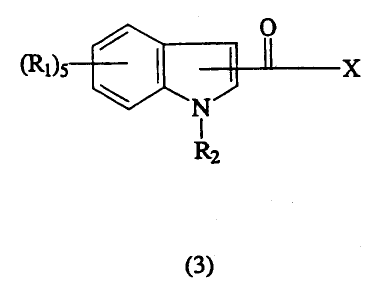 dans laquelle R₁ et R₂ sont tels que définis dans la revendication 1 et X est un groupement labile, avec de la guanidine dans un solvant inerte ; ou
b) la réaction d'un acide indolecarboxylique de formule suivante (4) : 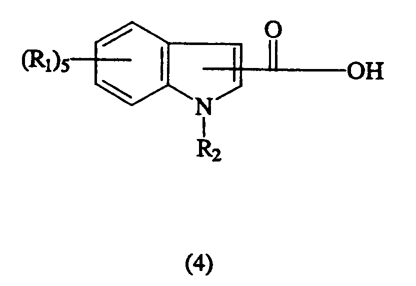 dans laquelle R₁ et R₂ sont tels que définis dans la revendication 1, avec de la guanidine dans un solvant inerte ; ou
c) la soumission d'un composé de formule (5) : 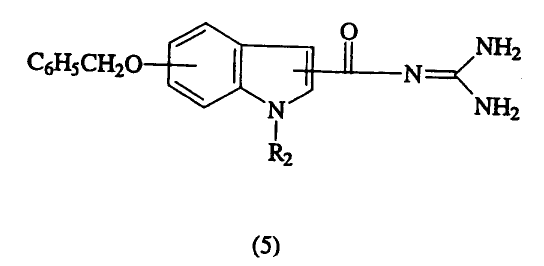 dans laquelle R₂ est tel que défini dans la revendication 1, à une débenzylation, de façon à obtenir un dérivé d'indoloylguanidine de formule (Ia) : 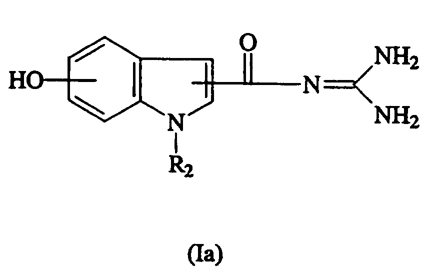 dans laquelle R₂ est tel que défini dans la revendication 1 ; ou
d) la réduction d'un composé de formule (6) : 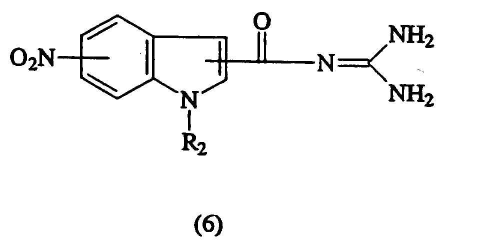 dans laquelle R₂ est tel que défini dans la revendication 1, de façon à obtenir un dérivé d'indoloylguanidine de formule (Ib) : 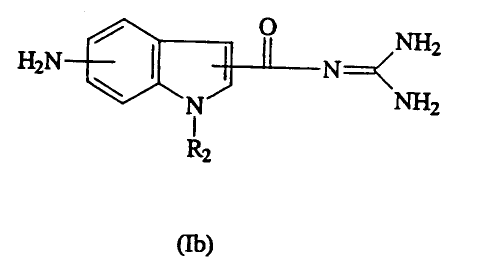 dans laquelle R₂ est tel que défini dans la revendication 1 ; et/ou si on le désire,
e) la conversion d'un composé de formule (I), préparé par l'un quelconque des procédés précédents (a) à (d), en un sel pharmaceutiquement acceptable.

20. Composition pharmaceutique qui comprend un support ou diluant pharmaceutiquement acceptable et, comme principe actif, un composé tel que revendiqué dans l'une quelconque des revendications 1 à 18.

21. Composé tel que défini dans l'une quelconque des revendications 1 à 18, à utiliser dans un procédé de traitement du corps humain ou animal par thérapie.

22. Utilisation d'un composé tel que défini dans l'une quelconque des revendications 1 à 18 dans la fabrication d'un médicament pour le traitement d'une maladie provoquée par une activité accrue des échangeurs Na+/H+.
